# EUROPÄISCHE PATENTSCHRIFT

(11) **EP 1 310 474 B1**
(45) Veröffentlichungstag und Bekanntmachung des Hinweises auf die Patenterteilung: **03.01.2007**
(21) Anmeldenummer: 02024495.0
(22) Anmeldetag: 30.10.2002
(51) Int. Cl.: C07C 25/18, C07C 43/225, C07C 255/50, C07C 331/28, C07C 381/00, C09K 19/12, G02F 1/13

(54) **Fluorierte Biphenylverbindungen und ihre Verwendung in flüssigkristallinen Medien**
Fluorinated biphenyl compounds and their use in liquid crystal compositions
Biphényles fluorés et leur utilisation dans des compositions de cristaux liquides

(30) Priorität: 09.11.2001 DE 10155079
(43) Veröffentlichungstag der Anmeldung: 14.05.2003
(60) Teilanmeldung: 06016221.1
(73) Patentinhaber: Merck Patent GmbH, 64293 Darmstadt (DE)
(72) Erfinder: Reiffenrath, Volker, 64380 Rossdorf (DE); Heckmeier, Michael, Dr., 69502 Hemsbach (DE); Engel, Martin, 64291 Darmstadt (DE); Schuler, Brigitte, 63762 Gross Ostheim (DE)

(56) Entgegenhaltungen:
- DE-A- 4 325 985
- DE-A- 19 748 109
- DE-A- 19 958 154
- GOUDA K-I ET AL: "CROSS-COUPLING REACTIONS OF ARYL CHLORIDES WITH ORGANOCHLOROSILANES: HIGHLY EFFECTIVE METHODS FOR ARYLATION OR ALKENYLATION OF ARYL CHLORIDES" JOURNAL OF ORGANIC CHEMISTRY, Bd. 61, Nr. 21, 1996, Seiten 7232-7233, XP000627290 ISSN: 0022-3263
- GRAY, G.W. ET AL.: "THE SYNTHESIS AND TRANSITION TEMPERATURES OF SOME 4'-ALKYL- AND 4'-ALKOXY-4-CYANO-3-FLUOROBIPHENYLS" LIQUID CRYSTALS, Bd. 19, Nr. 1, 1995, Seiten 77-83, XP000536259 ISSN: 0267-8292

## Beschreibung

Die vorliegende Erfindung betrifft ein flüssigkristallines Medium, dessen Verwendung für elektrooptische Zwecke und dieses Medium enthaltende Anzeigen.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("supertwisted nematic"), SBE-Zellen ("superbirefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannungen und einen hohen Kontrast ergeben.

Weiterhin sollten sie bei üblichen Betriebstemperaturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielsweise sollten Materialien für Zellen mit verdrillt nematischer Struktur eine positive dielektrische Anisotropie und eine geringe elektrische Leitfähigkeit aufweisen.

Beispielsweise sind für Matrix-Flüssigkristallanzeigen mit integrierten nicht-linearen Elementen zur Schaltung einzelner Bildpunkte (MFK-Anzeigen) Medien mit großer positiver dielektrischer Anisotropie, breiten nematischen Phasen, relativ niedriger Doppelbrechung, sehr hohem spezifischen Widerstand, guter UV- und Temperaturstabilität und geringem Dampfdruck erwünscht.

Derartige Matrix-Flüssigkristallanzeigen sind bekannt. Als nichtlineare Elemente zur individuellen Schaltung der einzelnen Bildpunkte können beispielsweise aktive Elemente (d.h. Transistoren) verwendet werden. Man spricht dann von einer "aktiven Matrix", wobei man zwei Typen unterscheiden kann:
1. MOS (Metal Oxide Semiconductor) oder andere Dioden auf Silizium-Wafer als Substrat.
2. Dünnfilm-Transistoren (TFT) auf einer Glasplatte als Substrat.

Die Verwendung von einkristallinem Silizium als Substratmaterial beschränkt die Displaygröße, da auch die modulartige Zusammensetzung verschiedener Teildisplays an den Stößen zu Problemen führt.

Bei dem aussichtsreicheren Typ 2, welcher bevorzugt ist, wird als elektrooptischer Effekt üblicherweise der TN-Effekt verwendet. Man unterscheidet zwei Technologien: TFT's aus Verbindungshalbleitern wie z.B. CdSe oder TFT's auf der Basis von polykristallinem oder amorphem Silizium. An letzterer Technologie wird weltweit mit großer Intensität gearbeitet.

Die TFT-Matrix ist auf der Innenseite der einen Glasplatte der Anzeige aufgebracht, während die andere Glasplatte auf der Innenseite die transparente Gegenelektrode trägt. Im Vergleich zu der Größe der Bildpunkt-Elektrode ist der TFT sehr klein und stört das Bild praktisch nicht. Diese Technologie kann auch für voll farbtaugliche Bilddarstellungen erweitert werden, wobei ein Mosaik von roten, grünen und blauen Filtern derart angeordnet ist, dass je ein Filterelement einem schaltbaren Bildelement gegenüber liegt.

Die TFT-Anzeigen arbeiten üblicherweise als TN-Zellen mit gekreuzten Polarisatoren in Transmission und sind von hinten beleuchtet.

Der Begriff MFK-Anzeigen umfasst hier jedes Matrix-Display mit integrierten nichtlinearen Elementen, d.h. neben der aktiven Matrix auch Anzeigen mit passiven Elementen wie Varistoren oder Dioden (MIM = Metall-Isolator-Metall).

Derartige MFK-Anzeigen eignen sich insbesondere für TV-Anwendungen (z.B. Taschenfernseher) oder für hochinformative Displays für Rechneranwendungen (Laptop) und im Automobil- oder Flugzeugbau. Neben Problemen hinsichtlich der Winkelabhängigkeit des Kontrastes und der Schaltzeiten resultieren bei MFK-Anzeigen Schwierigkeiten bedingt durch nicht ausreichend hohen spezifischen Widerstand der Flüssigkristallmischungen [TOGASHI, S., SEKIGUCHI, K., TANABE, H., YAMAMOTO, E., SORIMACHI, K., TAJIMA, E., WATANABE, H., SHIMIZU, H., Proc. Eurodisplay 84, Sept. 1984: A 210-288 Matrix LCD Controlled by Double Stage Diode Rings, p. 141 ff, Paris; STROMER, M., Proc. Eurodisplay 84, Sept. 1984: Design of Thin Film Transistors for Matrix Adressing of Television Liquid Crystal Displays, p. 145 ff, Paris]. Mit abnehmendem Widerstand verschlechtert sich der Kontrast einer MFK-Anzeige und es kann das Problem der "after image elimination" auftreten. Da der spezifische Widerstand der Flüssigkristallmischung durch Wechselwirkung mit den inneren Oberflächen der Anzeige im allgemeinen über die Lebenszeit einer MFK-Anzeige abnimmt, ist ein hoher (Anfangs)-Widerstand sehr wichtig, um akzeptable Standzeiten zu erhalten. Insbesondere bei low-volt-Mischungen war es bisher nicht möglich, sehr hohe spezifische Widerstände zu realisieren. Weiterhin ist es wichtig, dass der spezifische Widerstand eine möglichst geringe Zunahme bei steigender Temperatur sowie nach Temperatur- und/oder UV-Belastung zeigt. Besonders nachteilig sind auch die Tieftemperatureigenschaften der Mischungen aus dem Stand der Technik. Gefordert wird, dass auch bei tiefen Temperaturen keine Kristallisation und/oder smektische Phasen auftreten und die Temperaturabhängigkeit der Viskosität möglichst gering ist. Die MFK-Anzeigen aus dem Stand der Technik genügen somit nicht den heutigen Anforderungen.

Neben Flüssigkristallanzeigen, die eine Hintergrundbeleuchtung verwenden, also transmissiv und gegebenenfalls transflektiv betrieben werden, sind besonders auch reflektive Flüssigkristallanzeigen interessant. Diese reflektiven Flüssigkristallanzeigen benutzen das Umgebungslicht zur Informationsdarstellung. Somit verbrauchen sie wesentlich weniger Energie als hintergrundbeleuchtete Flüssigkristallanzeigen mit entsprechender Größe und Auflösung. Da der TN-Effekt durch einen sehr guten Kontrast gekennzeichnet ist, sind derartige reflektive Anzeigen auch bei hellen Umgebungsverhältnissen noch gut abzulesen. Dies ist bereits von einfachen reflektiven TN-Anzeigen, wie sie in z. B. Armbanduhren und Taschenrechnern verwendet werden, bekannt. Jedoch ist das Prinzip auch auf hochwertige, höher auflösende Aktiv-Matrix angesteuerte Anzeigen wie z. B. TFT-Displays anwendbar. Hier ist wie bereits bei den allgemeinen üblichen transmissiven TFT-TN-Anzeigen die Verwendung von Flüssigkristallen mit niedriger Doppelbrechung (Δn) nötig, um eine geringe optische Verzögerung (d · Δn) zu erreichen. Diese geringe optische Verzögerung führt zu einer meist akzeptablen geringen Blickwinkelabhängigkeit des Kontrastes (vgl. DE 30 22 818). Bei reflektiven Anzeigen ist die Verwendung von Flüssigkristallen mit kleiner Doppelbrechung noch wichtiger als bei transmissiven Anzeigen, da bei reflektiven Anzeigen die effektive Schichtdicke, die das Licht durchquert, ungefähr doppelt so groß ist wie bei transmissiven Anzeigen mit derselben Schichtdicke.

DE 43 25 985 A, DE 197 48 109 A, DE 199 58 154 A, Gray et al., Liq. Cryst. 19(1), 77-83 (1995) und Gouda et al., J. Org. chem. 61(21), 7232-7233 (1996) offenbaren Biphenyle mit polaren Resten, aber keine Flüssigkristallmedien gemäß der vorliegenden Erfindung.

Es besteht somit immer noch ein großer Bedarf nach MFK-Anzeigen mit sehr hohem spezifischen Widerstand bei gleichzeitig großem Arbeitstemperaturbereich, kurzen Schaltzeiten auch bei tiefen Temperaturen und niedriger Schwellenspannung, die diese Nachteile nicht oder nur in geringerem Maße zeigen.

Bei TN-(Schadt-Helfrich)-Zellen sind Medien erwünscht, die folgende Vorteile in den Zellen ermöglichen:
- erweiterter nematischer Phasenbereich (insbesondere zu tiefen Temperaturen)
- Schaltbarkeit bei extrem tiefen Temperaturen (out-door-use, Automobil, Avionik)
- erhöhte Beständigkeit gegenüber UV-Strahlung (längere Lebensdauer)
- kleiner optischer Doppelbrechung für geringe Schichtdicken
- kleine Schwellenspannung

Mit den aus dem Stand der Technik zur Verfügung stehenden Medien ist es nicht möglich, diese Vorteile unter gleichzeitigem Erhalt der übrigen Parameter zu realisieren.

Bei höher verdrillten Zellen (STN) sind Medien erwünscht, die eine höhere Multiplexierbarkeit und/oder kleinere Schwellenspannungen und/oder breitere nematische Phasenbereiche (insbesondere bei tiefen Temperaturen) ermöglichen. Hierzu ist eine weitere Ausdehnung des zur Verfügung stehenden Parameterraumes (Klärpunkt, Übergang smektisch-nematisch bzw. Schmelzpunkt, Viskosität, dielektrische Größen, elastische Größen) dringend erwünscht.

Der Erfindung liegt die Aufgabe zugrunde, Medien insbesondere für derartige MFK-, TN- oder STN-Anzeigen bereitzustellen, die die oben angegebenen Nachteile nicht oder nur in geringerem Maße, und vorzugsweise gleichzeitig sehr hohe spezifische Widerstände und niedrige Schwellenspannungen aufweisen. Für diese Aufgabe werden flüssigkristalline Verbindungen benötigt, die einen hohen Klärpunkt und eine niedrige Rotationsviskosität besitzen.

Es wurde nun gefunden, dass diese Aufgabe gelöst werden kann, wenn man die flüssigkristalline Verbindung verwendet, die einen terminalen polaren Rest und eine terminale CH₃-Gruppe aufweisen. Die Verbindungen der Formel I reduzieren positiv die elastischen Konstanten, insbesondere K₁ und führen zu Mischungen mit besonders niedrigen Schwellenspannungen.

Gegenstand der Erfindung sind somit ein flüssigkristallines Medium auf der Basis eines Gemisches von polaren Verbindungen mit positiver oder negativer dielektrischer Anisotropie enthaltend eine oder mehrere Verbindungen der Formel I, 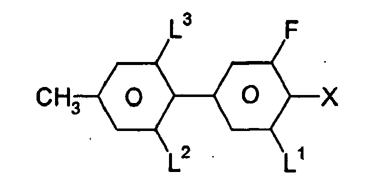 worin
- X: F, Cl, CN, SF₅, NCS, einen halogenierten Alkylrest mit bis zu 8 C-Atomen, wobei eine oder mehrere CH₂-Gruppen durch -O- oder -CH=CH- so ersetzt sein können, dass O-Atome nicht direkt miteinander verknüpft sind und
- L¹, L², L³: jeweils unabhängig voneinander H oder F
bedeutet.

Die Verbindungen der Formel I sind in reinem Zustand farblos und bilden in der Regel flüssigkristalline Mesophasen in einem für die elektrooptische Verwendung günstig gelegenen Temperaturbereich. Insbesondere zeichnen sich die erfindungsgemäßen Biphenyle durch ihre hohen dielektrischen Anisotropien und ihre niedrigen Werte für die Rotationsviskosität aus. Chemisch, thermisch und gegen Licht sind sie stabil.

X bedeutet in den Verbindungen der Formel I vorzugsweise F, Cl, CN, NCS, CF₃, SF₅, CF₂H, CF₂CF₃, CF₂CF₂CF₃, CH₂CH₂CF₃, CF₂CH₂CF_{3,} OCF₃, OCF₂H, OCFHCF₃, OCFHCFH₂, OCH₂C₃F₇, OCH₂CF₂CHFCF_{3,} O(CH₂)₃CF₃, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCFHCFHCF₃, OCH₂CF₂CF₃, OCF₂CF₂CF₃, OCF₂CFHCFH_{2,} OCF₂CH₂CF₂H, OCFHCF₂CFH₂, OCFHCFHCF₂H, OCFHCH₂CF_{3,} OCH₂CFHCF₃, OCH₂CF₂CF₂H, OCF₂CFHCH₃, OCF₂CH₂CFH₂, OCFHCF₂CH₃, OCFHCFHCFH₂, OCFHCH₂CF₃, OCH₂CF₂CFH_{2,} OCH₂CFHCF₂H, OCF₂CH₂CH₃, OCFHCFHCH₃, OCFHCH₂CFH₂, OCH₂CF₂CH₃, OCH₂CFHCFH₂, OCH₂CH₂CF₂H, OCHCH₂CH₃, OCH₂CFHCH₃, OCH₂CH₂CF₂H, OCClFCF₃, OCClFCClF₂, OCClFCFH_{2,} OCFHCCl₂F, OCClFCF₂H, OCClFCClF₂, OCF₂CClH₂, OCF₂CCl₂H, OCF₂CCl₂F, OCF₂CClFH, OCF₂CClF₂, OCF₂CF₂CClF₂, OCF₂CF₂CCl₂F, OCClFCF₂CF₃, OCClFCF₂CF₂H, OCClFCF₂CCIF₂, OCClFCFHCF₃, OCClFCClFCF₃, OCCl₂CF₂CF₃, OCClHCF₂CF₃, OCClFCF₂CF₃, OCClFCClFCF₃, OCF₂CClFCFH₂, OCF₂CF₂CCl₂F, OCF₂CCl₂CF₂H, OCF₂CH₂CClF₂, OCClFCF₂CFH₂, OCFHCF₂CCl₂F, OCClFCFHCF₂H, OCClFCClFCF₂H, OCFHCFHCClF₂, OCClFCH₂CF₃, OCFHCCl₂CF₃, OCCl₂CFHCF₃, OCH₂CClFCF₃, OCCl₂CF₂CF₂H, OCH₂CF₂CClF₂, OCF₂CClFCH₃, OCF₂CFHCCl₂H, OCF₂CCl₂CFH₂, OCF₂CH₂CCl₂F, OCClFCF₂CH₃, OCFHCF₂CCl₂H, OCClFCClFCFH₂, OCFHCFHCCl₂F, OCClFCH₂CF₃, OCFHCCl₂CF₃, OCCl₂CF₂CFH₂, OCH₂CF₂CCl₂F, OCCl₂CFHCF₂H, OCClHCClFCF₂H, OCF₂CC)HCCtH₂, OCF₂CH₂CCl₂H, OCClFCFHCH₃, OCF₂CClFCCl₂H, OCClFCH₂CFH₂, OCFHCCl₂CFH₂, OCCl₂CF₂CH₃, OCH₂CF₂CClH₂, OCCl₂CFHCFH₂, OCH₂CClFCFCl₂, OCH₂CH₂CF₂H, OCClHCClHCF₂H, OCH₂CCl₂CF₂H, OCClFCH₂CH₃, OCFHCH₂CCl₂H, OCClHCFHCClH₂, OCH₂CFHCCl₂H, OCCl₂CH₂CF₂H, OCH₂CCl₂CF₂H, CH=CF₂, CF=CF₂, OCH=CF₂, OCF=CF₂, CH=CHF, OCH=CHF, CF=CHF, OCF=CHF, insbesondere F, Cl, CN, NCS, CF₃, SF₅, CF₂H, CF₂CF₃, CF₂CF₂CF₃, CH₂CH₂CF₃, CF₂CH₂CF₃, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCH₂C₃F₇, OCF2CF2CF3, OCF₂CHFCF₃ und OCClFCF₂CF₃.

Bevorzugte kleinere Gruppen von Verbindungen der Formel I sind diejenigen der Teilformeln I1 bis I5: 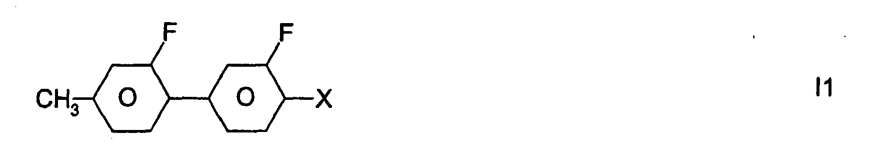 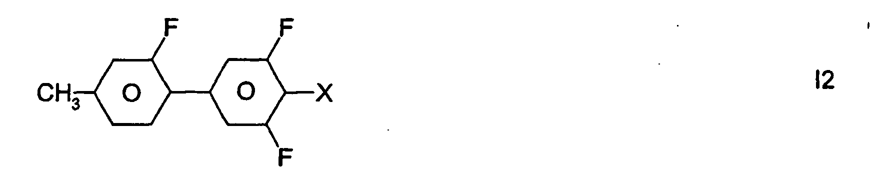 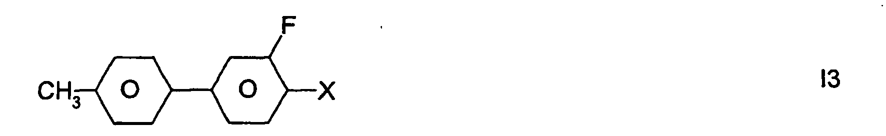 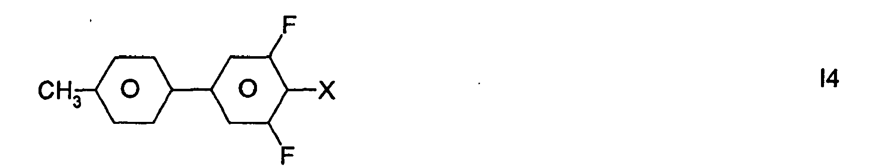 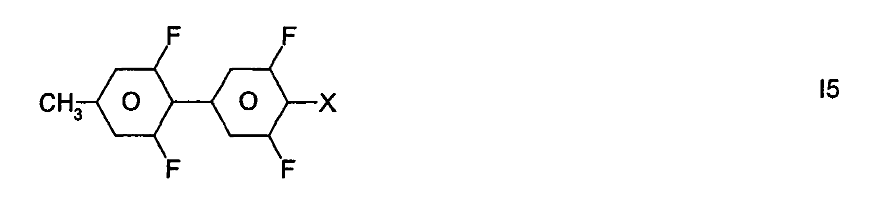 worin

X die in Anspruch 1 angegebene Bedeutung hat. X bedeutet vorzugsweise in den Teilformeln I1 bis I5 F oder OCF₃. Insbesondere bevorzugt sind Verbindungen der Formel 12, worin X F oder OCF₃ bedeutet.

Besonders bevorzugte Medien enthalten eine oder mehrere Verbindungen ausgewählt aus der Gruppe der Verbindungen der Formeln

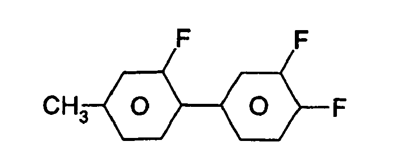

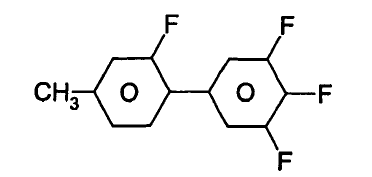

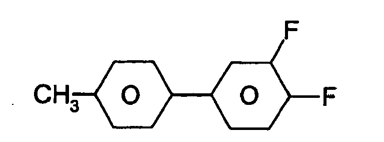

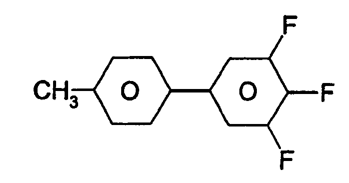

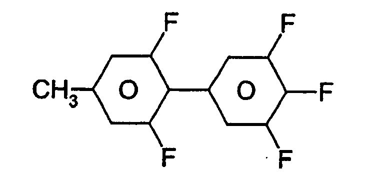

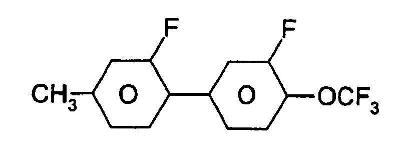

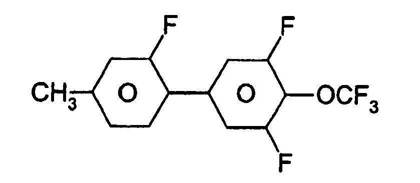

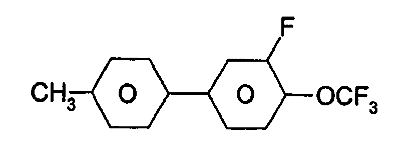

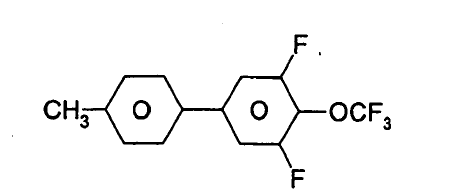

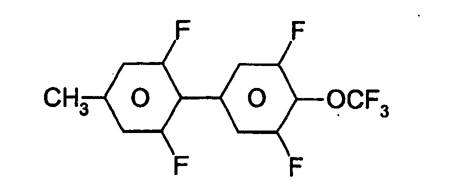

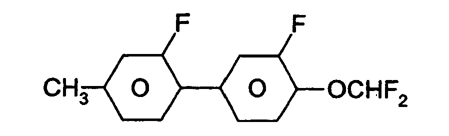

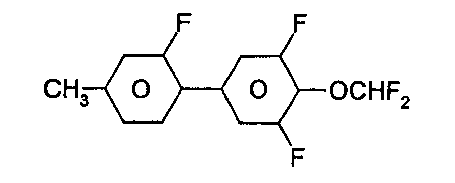

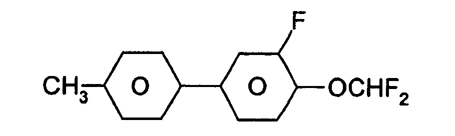

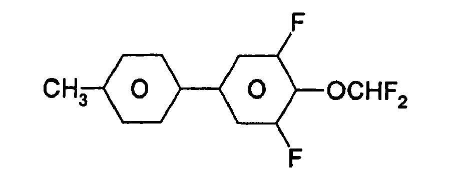

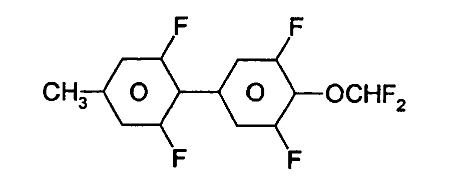

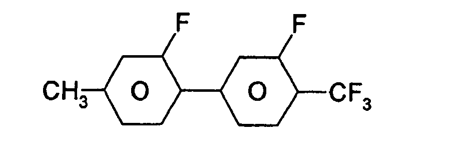

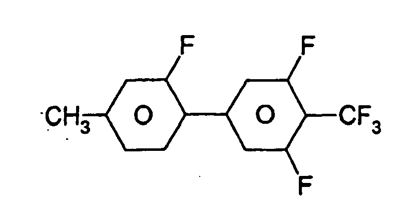

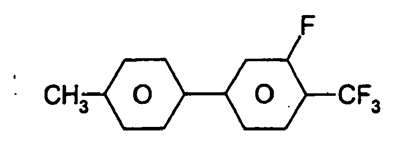

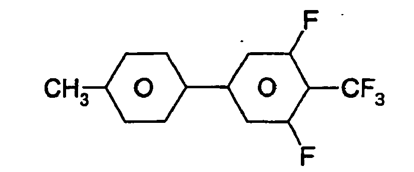

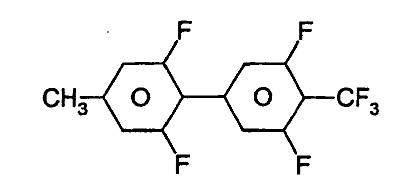

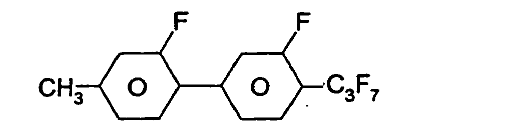

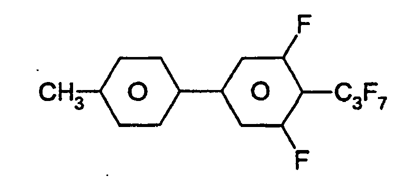

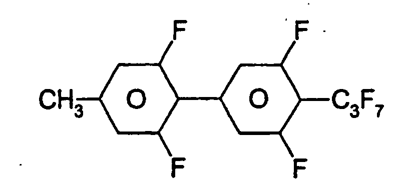

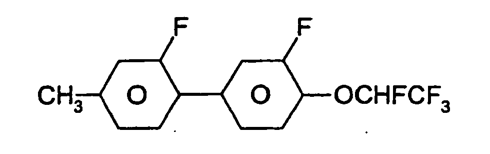

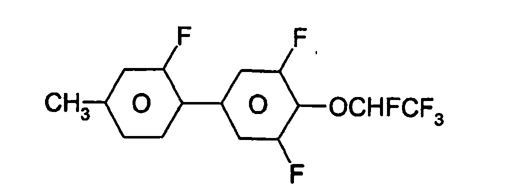

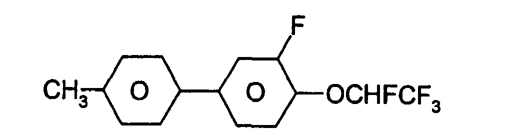

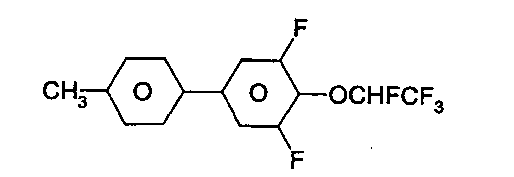

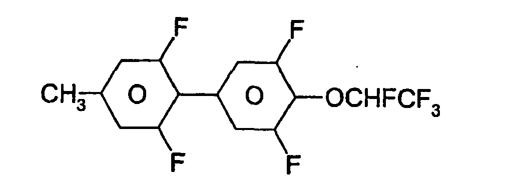

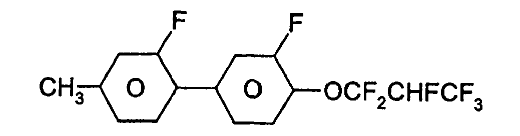

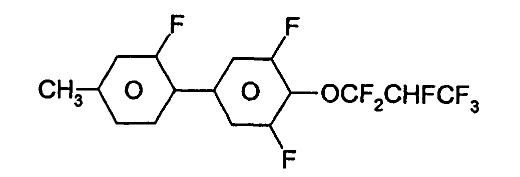

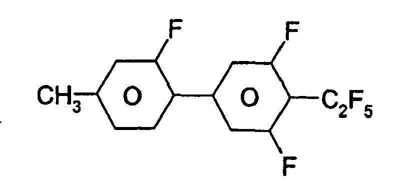

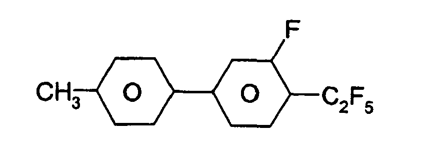

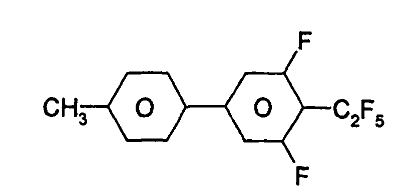

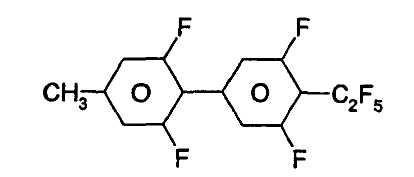

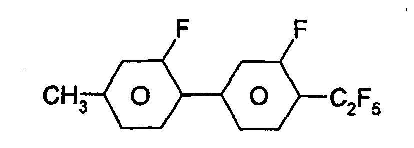

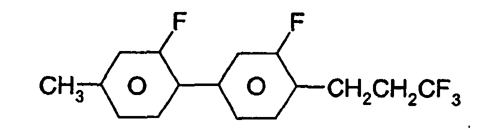

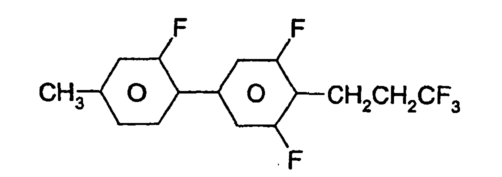

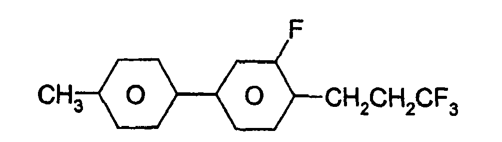

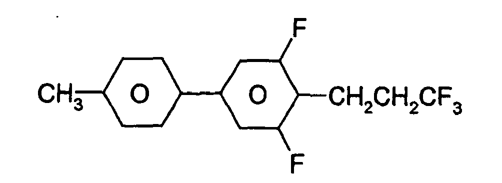

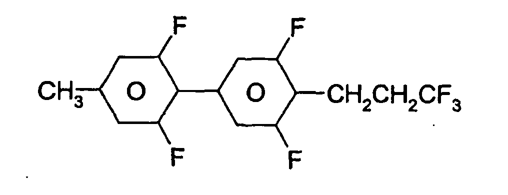

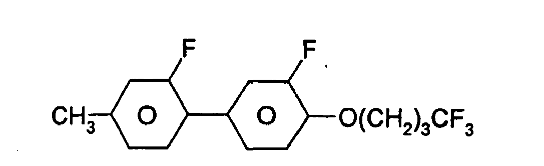

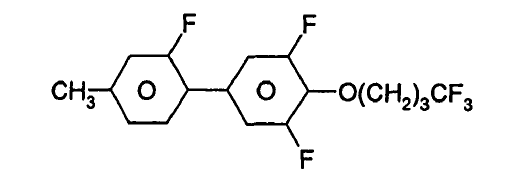

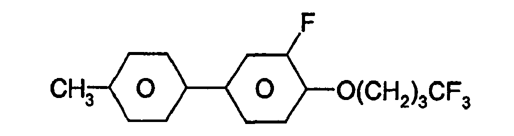

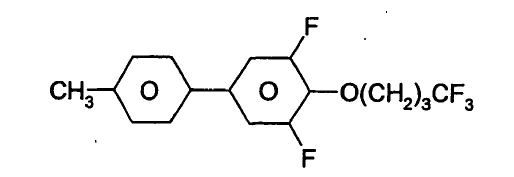

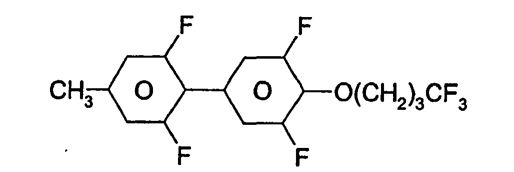

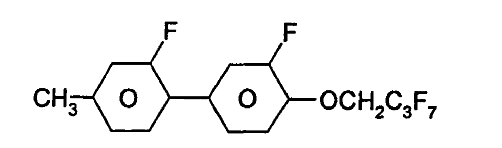

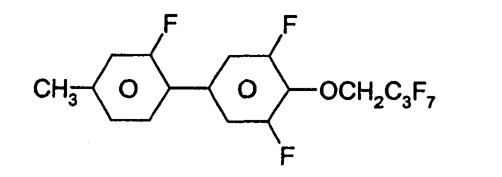

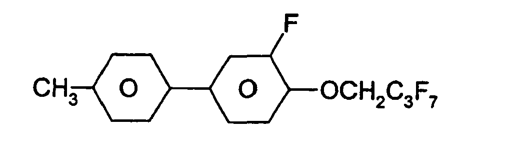

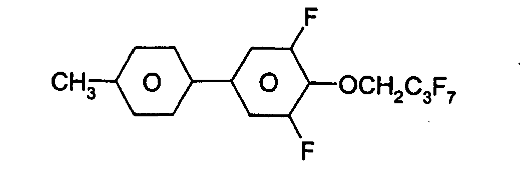

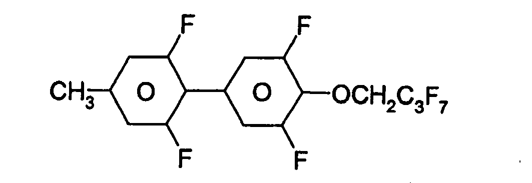

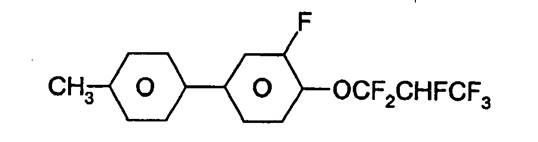

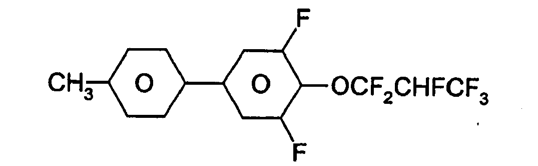

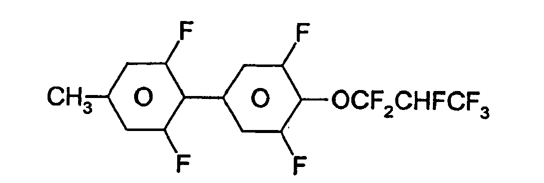

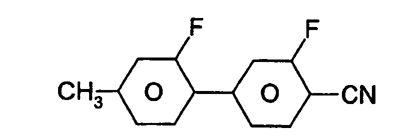

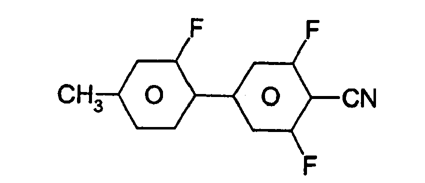

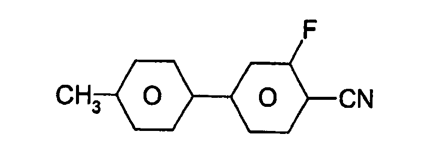

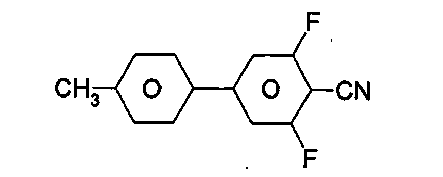

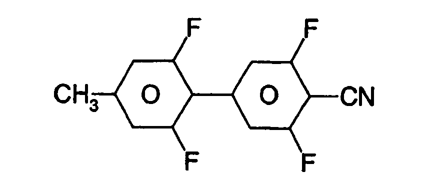

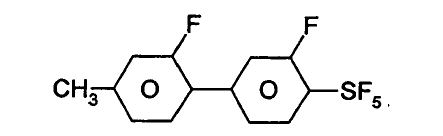

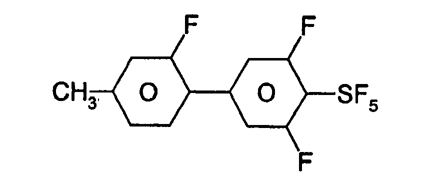

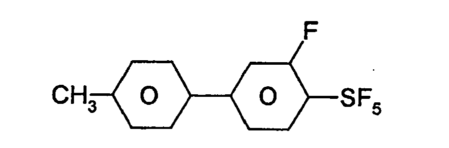

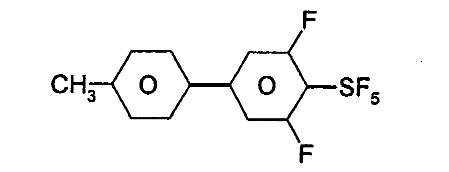

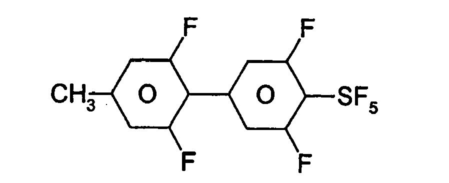

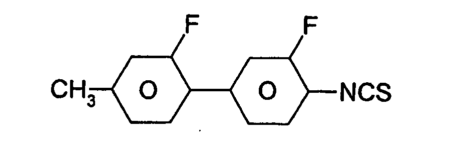

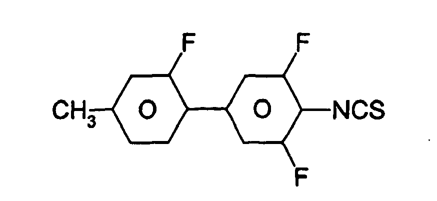

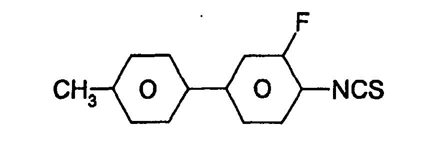

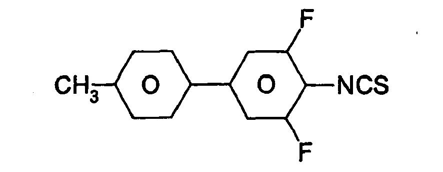

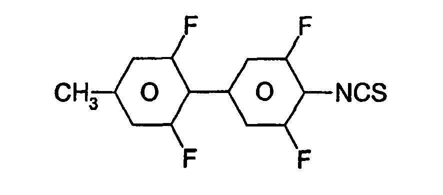

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man auch von an sich bekannten, hier nicht näher erwähnten Varianten Gebrauch machen.

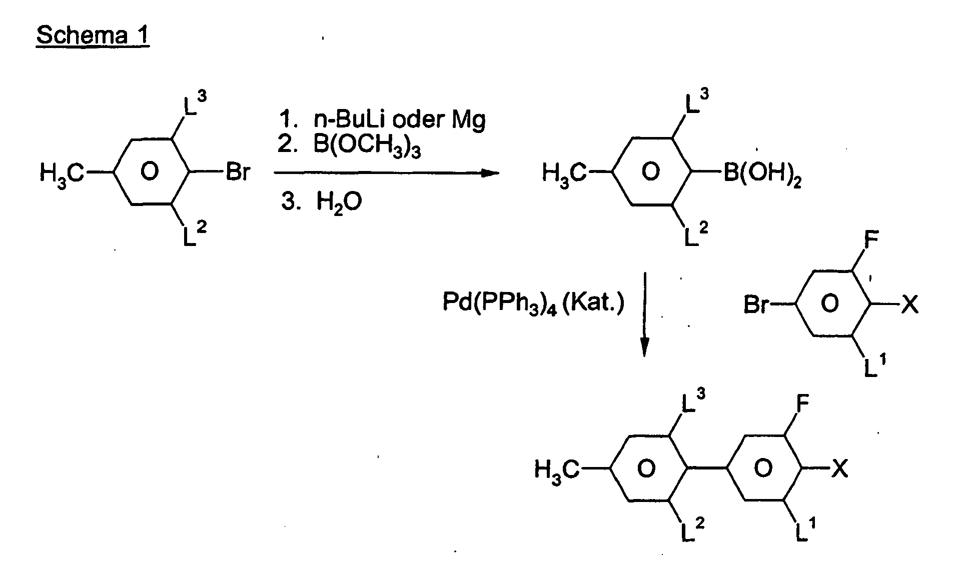

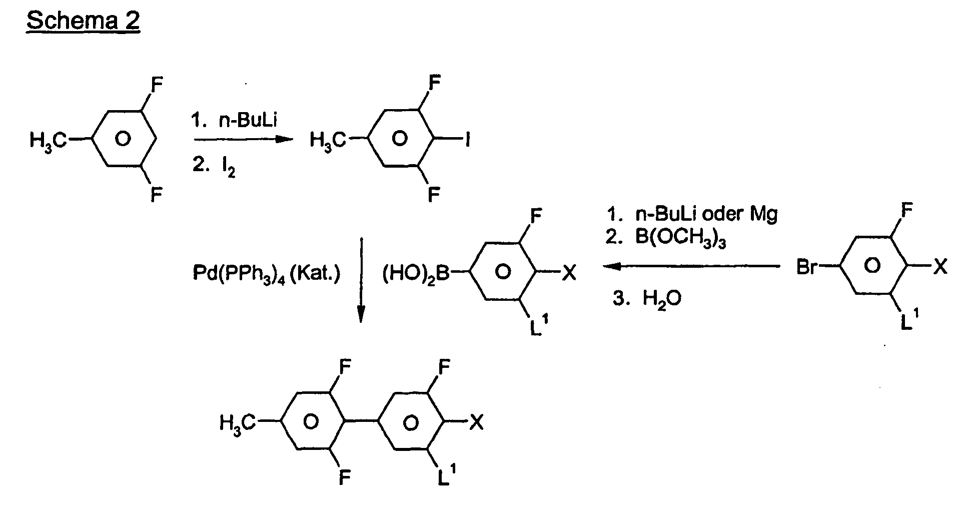

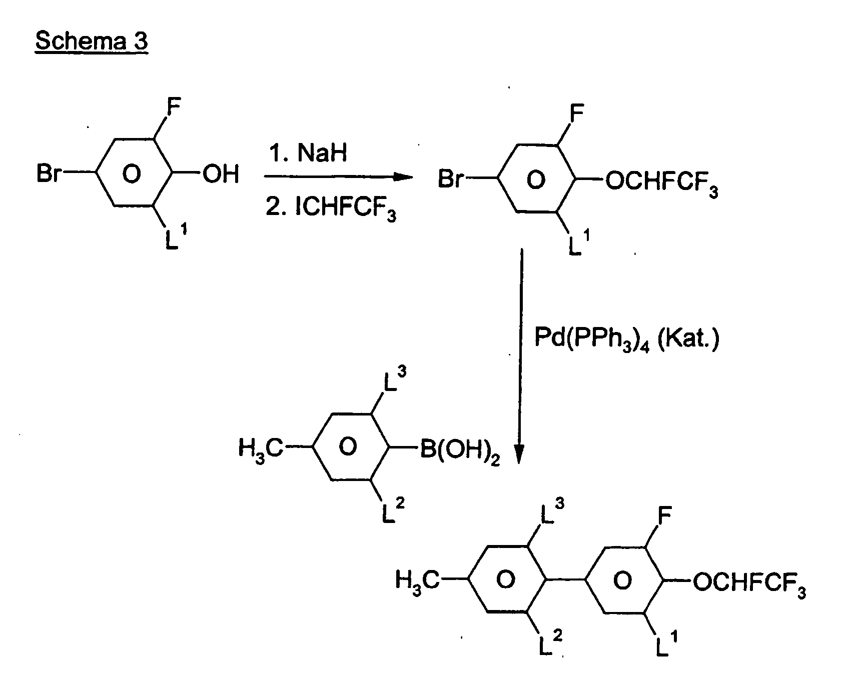

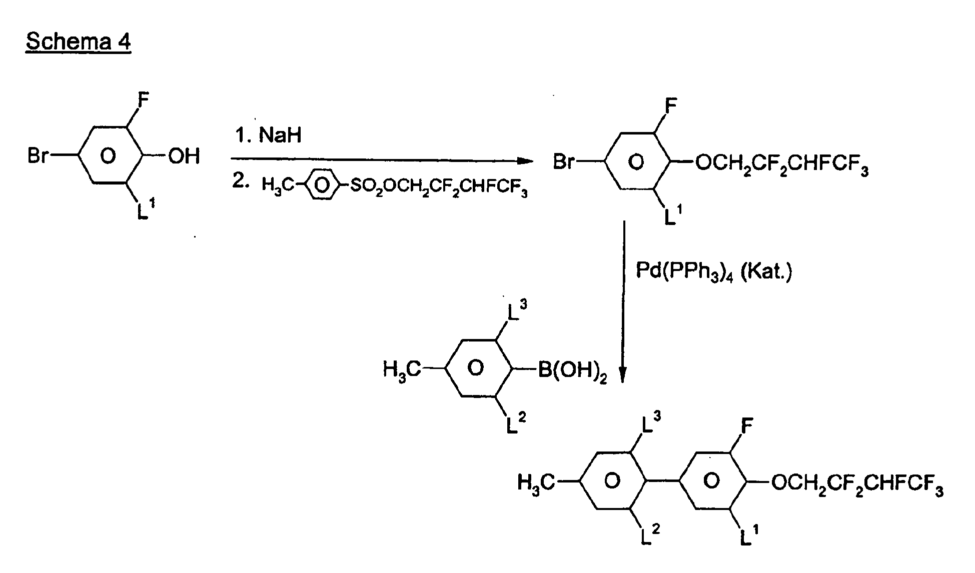

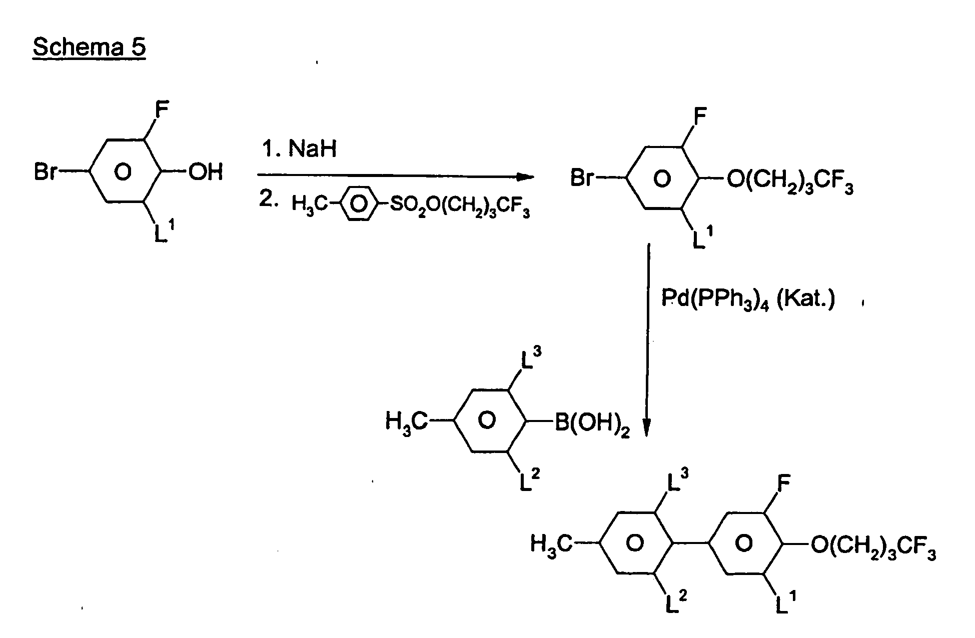

Gegenstand der Erfindung sind auch elektrooptische Anzeigen (insbesondere STN- oder MFK-Anzeigen mit zwei planparallelen Trägerplatten, die mit einer Umrandung eine Zelle bilden, integrierten nicht-linearen Elementen zur Schaltung einzelner Bildpunkte auf den Trägerplatten und einer in der Zelle befindlichen nematischen Flüssigkristallmischung mit positiver dielektrischer Anisotropie und hohem spezifischem Widerstand), die derartige Medien enthalten sowie die Verwendung dieser Medien für elektrooptische Zwecke.

Die erfindungsgemäßen Flüssigkristallmischungen ermöglichen eine bedeutende Erweiterung des zur Verfügung stehenden Parameterraumes.

Die erzielbaren Kombinationen aus Klärpunkt, optischer Anisotropie, Viskosität bei tiefer Temperatur, thermischer und UV-Stabilität und dielektrischer Anisotropie übertreffen bei weitem bisherige Materialien aus dem Stand der Technik.

Die Forderung nach hohem Klärpunkt, nematischer Phase bei tiefer Temperatur sowie einem hohen Δε konnte bislang nur unzureichend erfüllt werden. Flüssigkristallmischungen, wie z. B. MLC-6476 und MLC-6625 (Merck KGaA, Darmstadt, Deutschland) weisen zwar vergleichbare Klärpunkte und Tieftemperaturstabilitäten auf, sie haben jedoch relativ hohe Δn-Werte als auch höhere Schwellenspannungen von ca. ≥ 1,7 V.

Andere Mischungssysteme besitzen vergleichbare Viskositäten und Werte von Δε, weisen jedoch nur Klärpunkte in der Gegend von 60 °C auf.

Die erfindungsgemäßen Flüssigkristallmischungen ermöglichen es bei Beibehaltung der nematischen Phase bis -20 °C und bevorzugt bis -30 °C, besonders bevorzugt bis -40 °C, Klärpunkte oberhalb 80 °C, vorzugsweise oberhalb 90 °C, besonders bevorzugt oberhalb 100 °C, gleichzeitig dielektrische Anisotropiewerte Δε ≥ 4, vorzugsweise ≥ 6 und einen hohen Wert für den spezifischen Widerstand zu erreichen, wodurch hervorragende STN- und MKF-Anzeigen erzielt werden können. Insbesondere sind die Mischungen durch kleine Operationsspannungen gekennzeichnet. Die TN-Schwellen liegen unterhalb 1,5 V, vorzugsweise unterhalb 1,3 V. Es versteht sich, dass durch geeignete Wahl der Komponenten der erfindungsgemäßen Mischungen auch höhere Klärpunkte (z.B. oberhalb 110 °C) bei höheren Schwellenspannung oder niedrigere Klärpunkte bei niedrigeren Schwellenspannungen unter Erhalt der anderen vorteilhaften Eigenschaften realisiert werden können. Ebenso können bei entsprechend wenig erhöhten Viskositäten Mischungen mit größerem Δε und somit geringeren Schwellen erhalten werden. Die erfindungsgemäßen MFK-Anzeigen arbeiten vorzugsweise im ersten Transmissionsminimum nach Gooch und Tarry [C.H. Gooch und H.A. Tarry, Electron. Lett. 10, 2-4, 1974; C.H. Gooch und H.A. Tarry, Appl. Phys., Vol. 8, 1575-1584, 1975], wobei hier neben besonders günstigen elektrooptischen Eigenschaften wie geringe Winkelabhängigkeit des Kontrastes (DE-PS 30 22 818) bei gleicher Schwellenspannung wie in einer analogen Anzeige im zweiten Minimum eine kleinerere dielektrische Anisotropie ausreichend ist. Hierdurch lassen sich unter Verwendung der erfindungsgemäßen Mischungen im ersten Minimum deutlich höhere spezifische Widerstände verwirklichen als bei Mischungen mit Cyanverbindungen. Der Fachmann kann durch geeignete Wahl der einzelnen Komponenten und deren Gewichtsanteilen mit einfachen Routinemethoden die für eine vorgegebene Schichtdicke der MFK-Anzeige erforderliche Doppelbrechung einstellen.

Die Fließviskosität ν₂₀ bei 20 °C ist vorzugsweise < 60 mm² · s⁻¹, besonders bevorzugt < 50 mm² · s⁻¹. Der nematische Phasenbereich ist vorzugsweise mindestens 90°, insbesondere mindestens 100°. Vorzugsweise erstreckt sich dieser Bereich mindestens von -30° bis +80°.

Messungen des "Capacity Holding-ratio" (HR) [S. Matsumoto et al., Liquid Crystals 5, 1320 (1989); K. Niwa et al., Proc. SID Conference, San Francisco, June 1984, p. 304 (1984); G. Weber et al., Liquid Crystals 5, 1381 (1989)] haben ergeben, dass erfindungsgemäße Mischungen enthaltend Verbindungen der Formel I eine deutlich kleinere Abnahme des HR mit steigender Temperatur aufweisen als analoge Mischungen enthaltend anstelle den Verbindungen der Formel I Cyanophenylcyclohexane der Formel 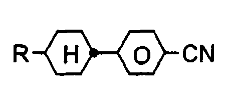 oder Ester der Formel

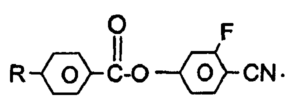

Auch die UV-Stabilität der erfindungsgemäßen Mischungen ist erheblich besser, d. h. sie zeigen eine deutlich kleinere Abnahme des HR unter UV-Belastung.

Vorzugsweise basieren die erfindungsgemäßen Medien auf ein oder mehreren (vorzugsweise ein, zwei, drei oder mehr) Verbindungen der Formel 1, d.h. der Anteil dieser Verbindungen ist 5-95 %, vorzugsweise 10-60 % und besonders bevorzugt im Bereich von 15-40 %.

Die einzelnen Verbindungen der Formeln 1 bis IX und deren Unterformeln, die in den erfindungsgemäßen Medien verwendet werden können, sind entweder bekannt, oder sie können analog zu den bekannten Verbindungen hergestellt werden.

Bevorzugte Ausführungsformen sind im folgenden angegeben:
- Medium enthält zusätzlich eine oder mehrere Verbindungen ausgewählt aus der Gruppe bestehend aus den allgemeinen Formeln II bis X: 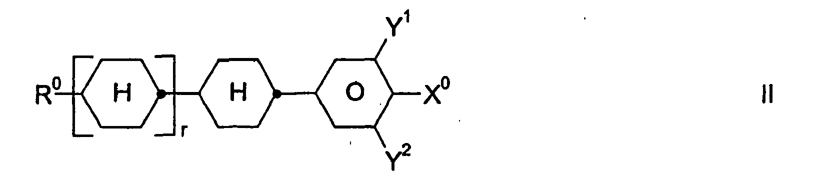 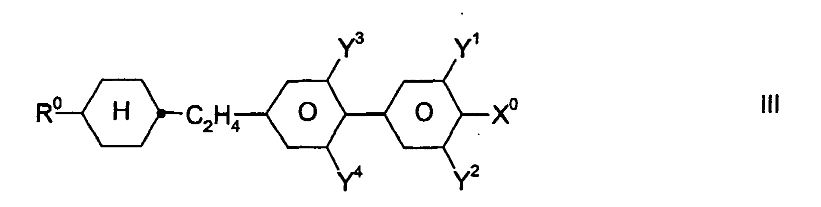 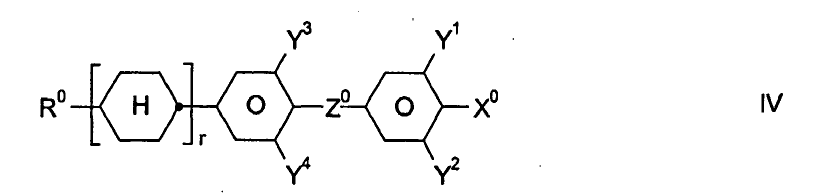 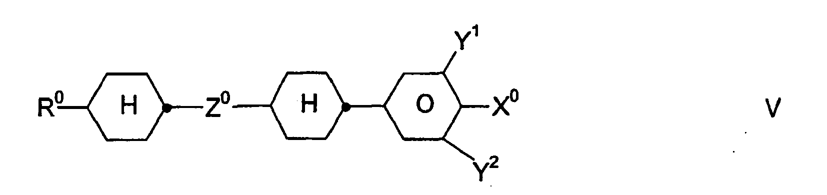 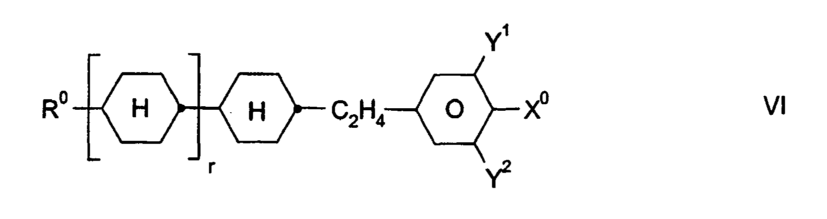 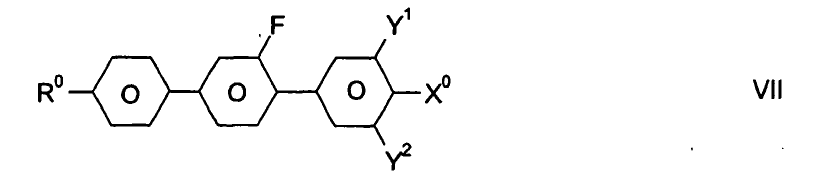 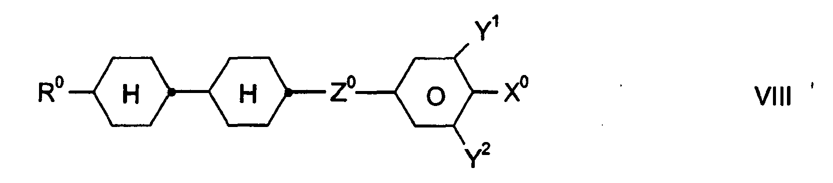 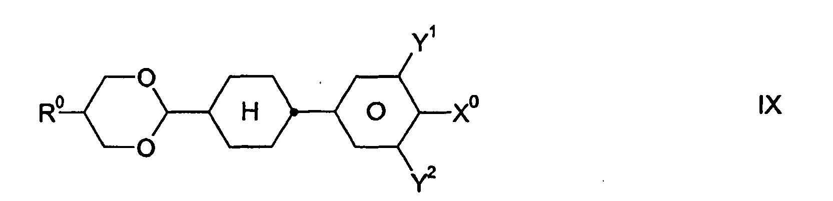 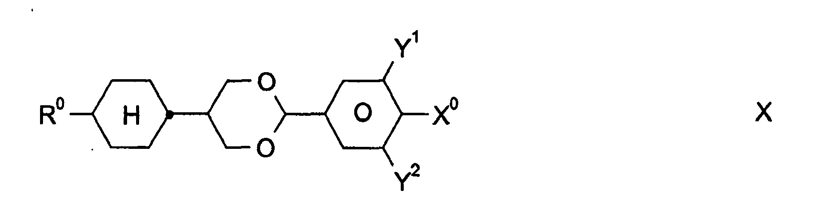
worin die einzelnen Reste die folgenden Bedeutungen haben:
- R⁰: n-Alkyl, Oxaalkyl, Fluoralkyl, Alkenyloxy oder Alkenyl mit 2 bis 12 C-Atomen
- X⁰: F, Cl, halogeniertes Alkyl, halogeniertes Alkenyl, halogeniertes Alkenyloxy oder halogeniertes Alkoxy mit jeweils bis zu 8 C-Atomen,
- Z⁰: -CH=CH-, -C₂H₄-, -CH₂O-, -OCH₂-, -(CH₂)₄-, -C₂F₄-, -CF=CF-, -CF₂O-, -OCF₂- oder -COO-,
- Y¹, Y², Y³ und Y⁴: jeweils unabhängig voneinander H oder F, und
- r: 0 oder 1.

Die Verbindung der Formel IV ist vorzugsweise 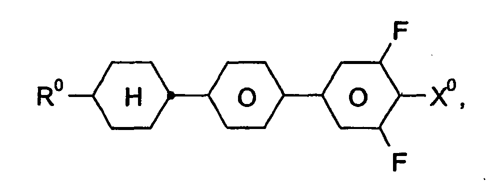 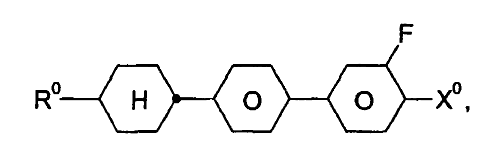 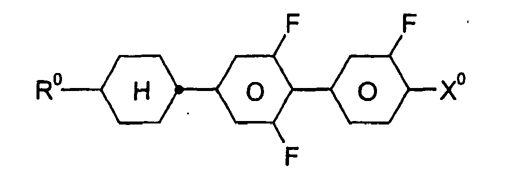 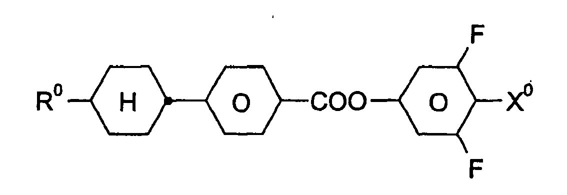 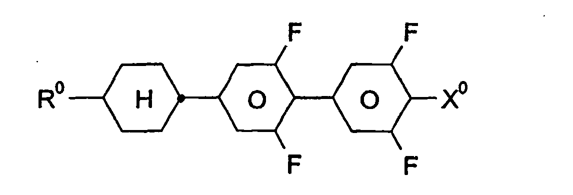 oder 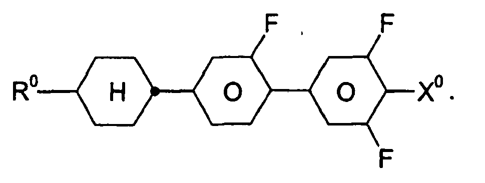
- Das Medium enthält insbesondere zusätzlich eine oder mehrere Verbindungen der Formeln 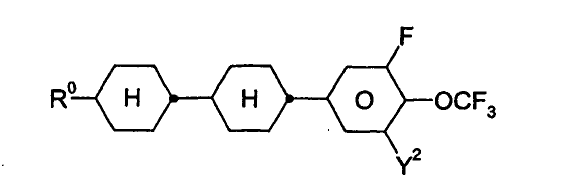 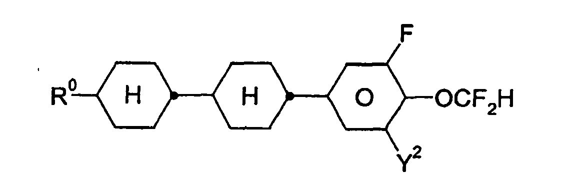 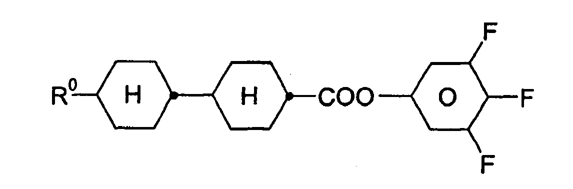 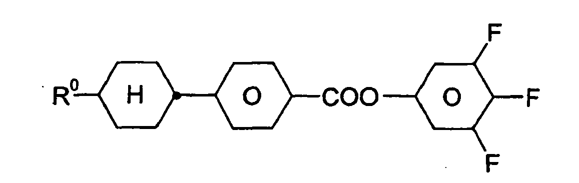 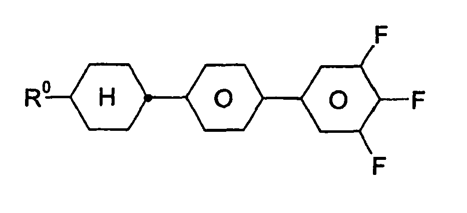 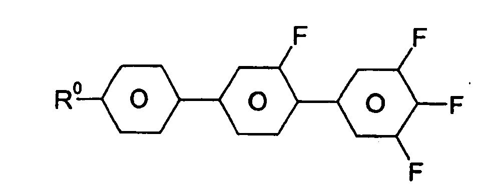 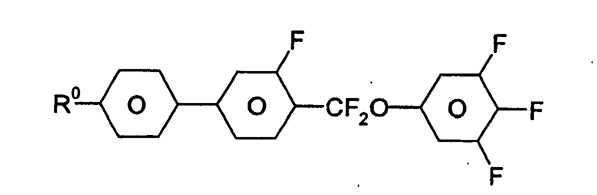 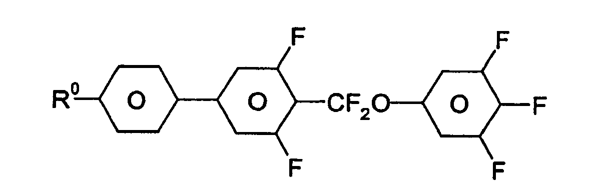 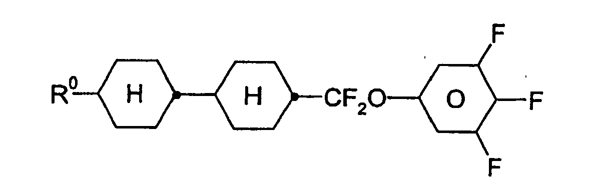 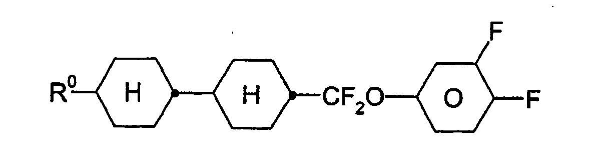 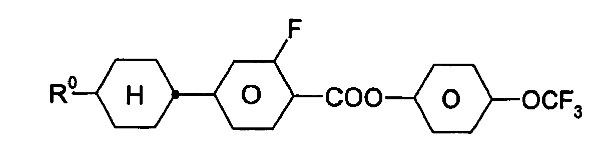 und/oder 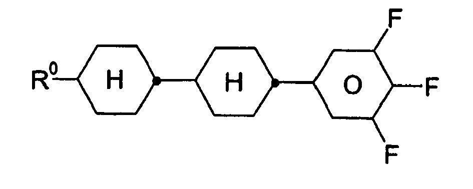 worin R⁰ und Y² die oben angegebene Bedeutung haben.
- Das Medium enthält vorzugsweise ein, zwei oder drei, ferner vier, Homologe der Verbindungen ausgewählt aus der Gruppe H1 bis H18 (n = 2-12): 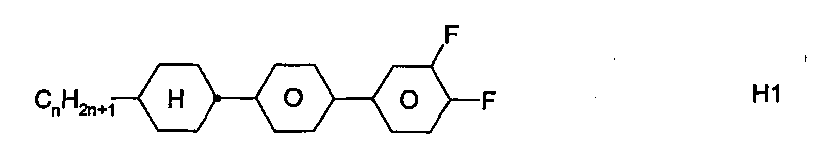 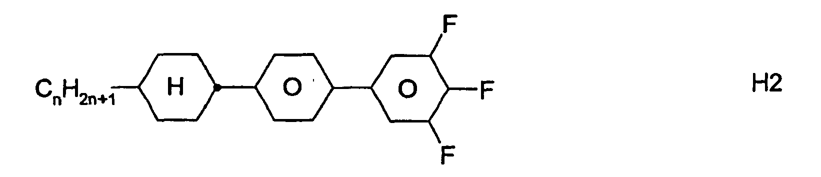 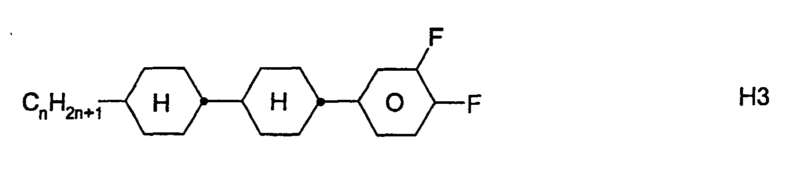 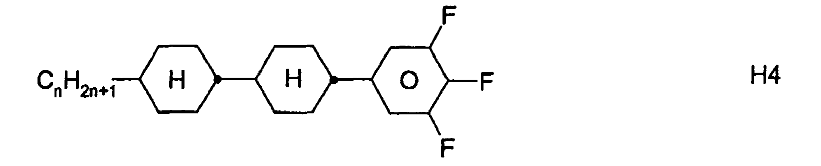 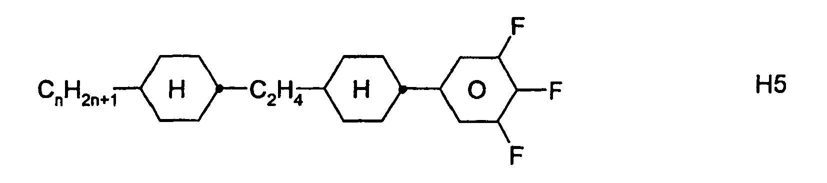 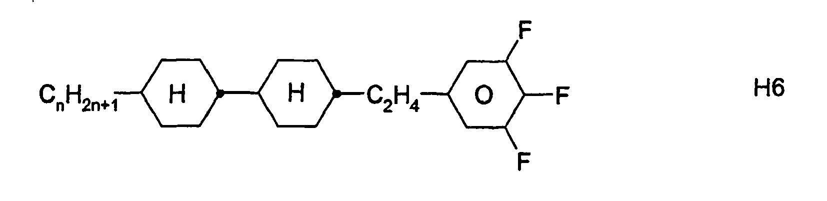 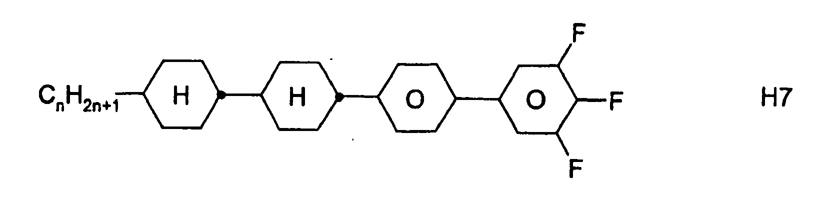 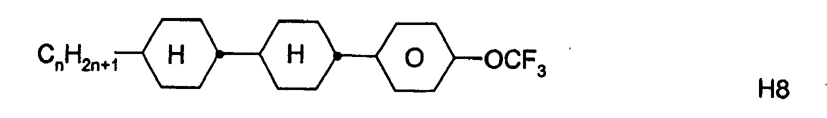 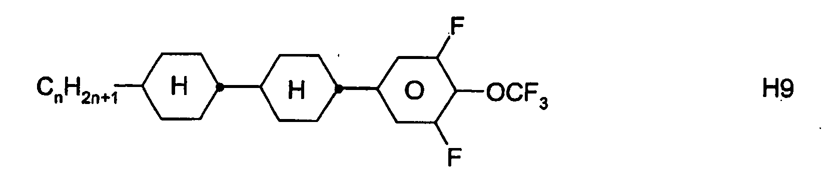 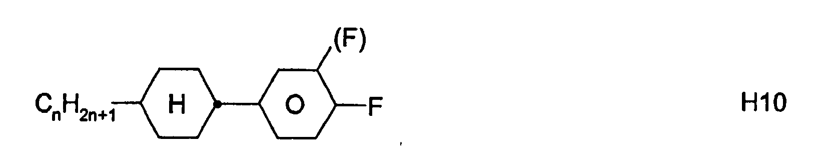 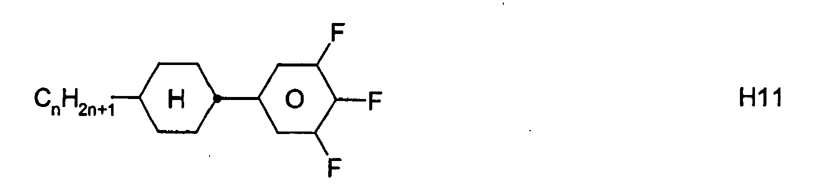 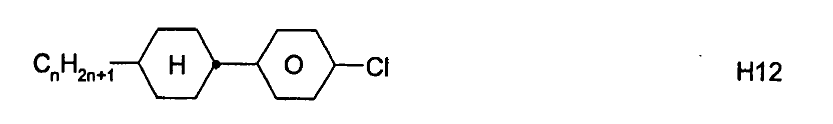 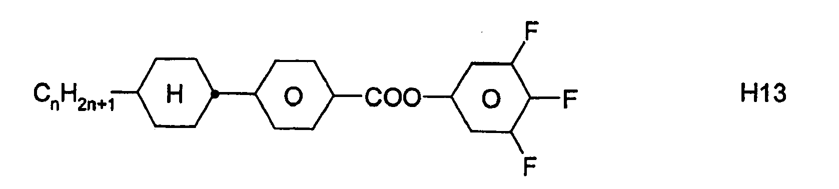 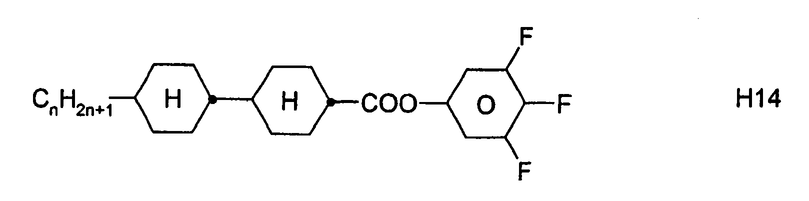 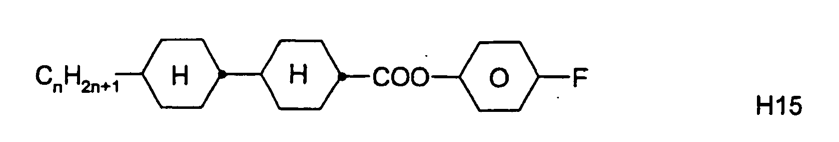 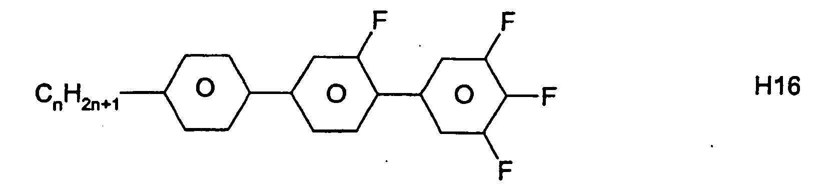 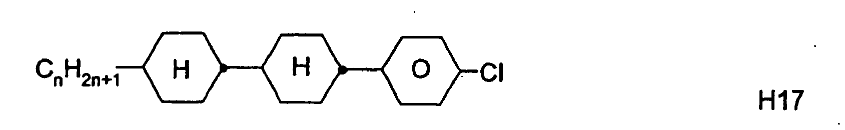 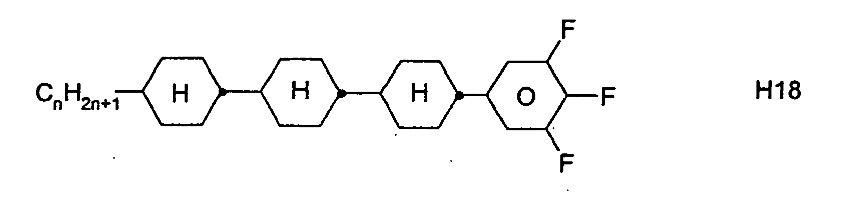
- Das Medium enthält zusätzlich ein oder mehrere Dioxane der Formel DI und/oder DII, 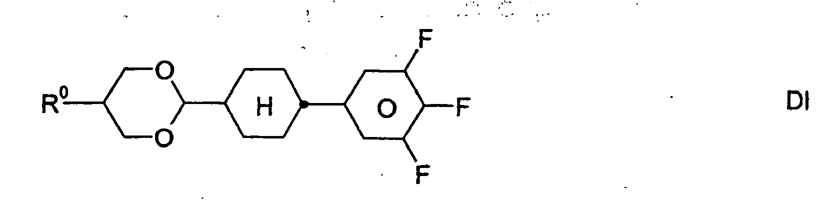 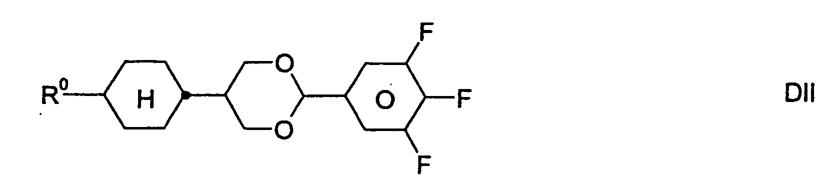 worin R⁰ die in Formel II angegebenen Bedeutungen hat. Vorzugsweise bedeutet R⁰ in den Verbindungen der Formel Dl und/oder DII geradkettiges Alkyl oder Alkenyl mit bis zu 8 C-Atomen.
- Das Medium enthält zusätzlich eine oder mehrere Verbindungen ausgewählt aus der Gruppe bestehend aus den allgemeinen Formeln XI bis XVI: 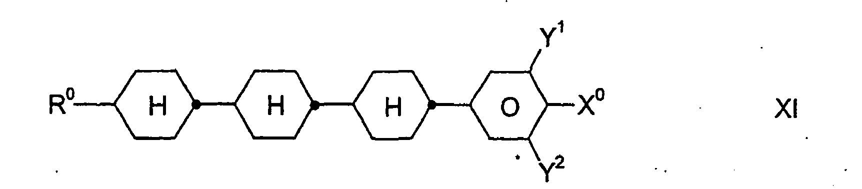 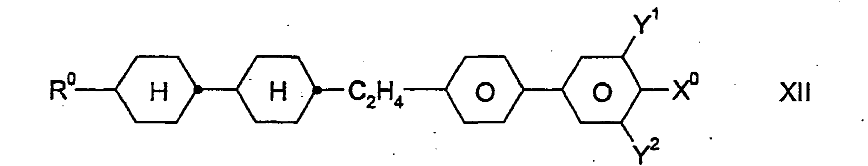 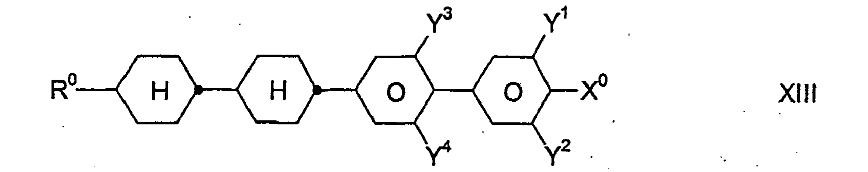 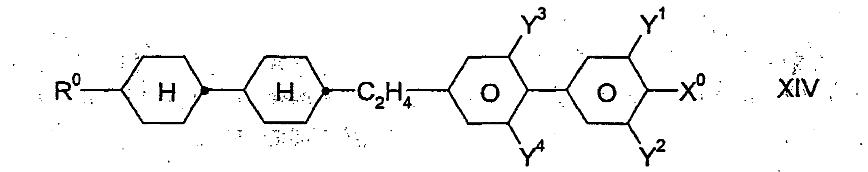 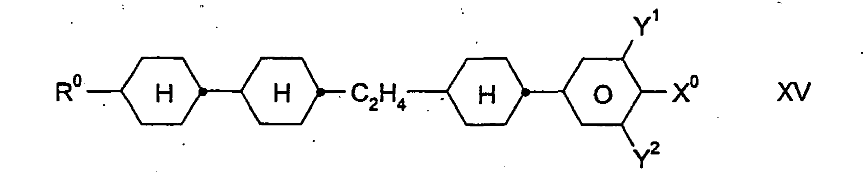 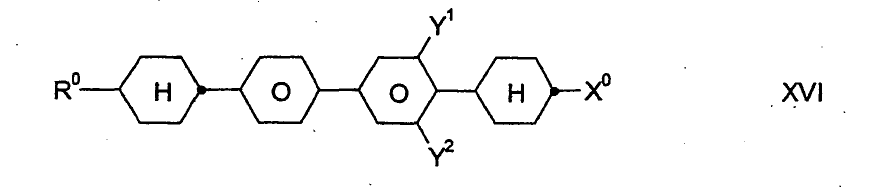 worin R⁰, X⁰, Y¹,Y², Y³ und Y⁴ jeweils unabhängig voneinander eine der in Formel II-X angegebenen Bedeutungen haben. Vorzugsweise bedeutet X⁰ F, Cl, CF₃, OCF₃ oder OCHF₂. R⁰ bedeutet vorzugsweise Alkyl, Oxaalkyl, Fluoralkyl, Alkenyl oder Alkenyloxy.
- Der Anteil an Verbindungen der Formeln I bis X zusammen beträgt im Gesamtgemisch mindestens 50 Gew.-%.
- Der Anteil an Verbindungen der Formel I beträgt im Gesamtgemisch 5 bis 50 Gew.-%.
- Der Anteil an Verbindungen der Formeln II bis X im Gesamtgemisch beträgt 30 bis 70 Gew.-%.
- 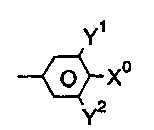 ist vorzugsweise 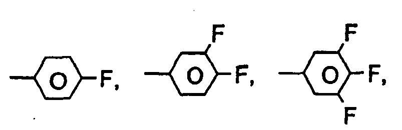 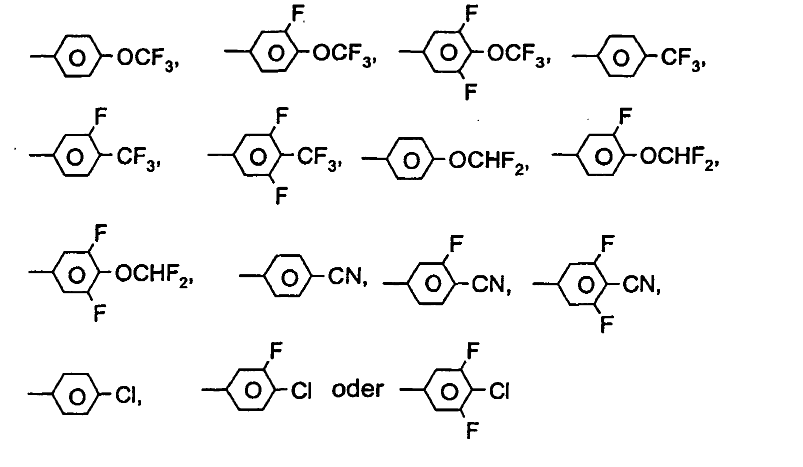
- Das Medium enthält Verbindungen der Formeln II, III, IV, V, VI, VII, VIII, IX und/oder X.
- R⁰ ist geradkettiges Alkyl oder Alkenyl mit 2 bis 8 C-Atomen.
- Das Medium besteht im wesentlichen aus Verbindungen der Formeln I bis XVI.
- Das Medium enthält weitere Verbindungen, vorzugsweise ausgewählt aus der folgenden Gruppe bestehend aus den allgemeinen Formeln XVII bis XX: 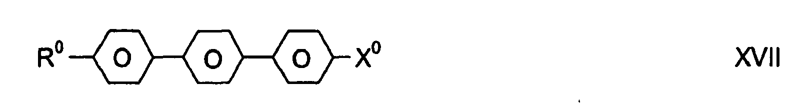 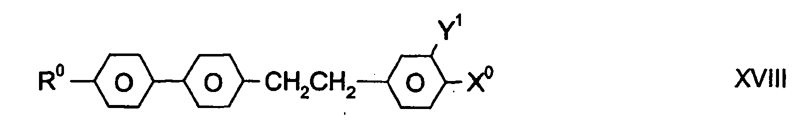 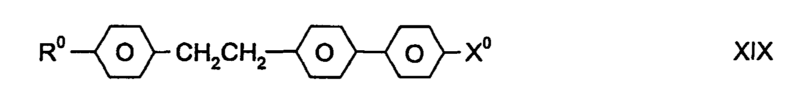 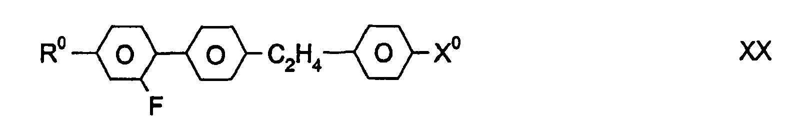 worin R⁰, Y¹ und X⁰ die oben angegebene Bedeutung haben und die 1,4-Phenylenringe durch CN, Chlor oder Fluor substituiert sein können. Vorzugsweise sind die 1,4-Phenylenringe ein- oder mehrfach durch Fluoratome substituiert.
- Das Medium enthält weitere Verbindungen, vorzugsweise ausgewählt aus der folgenden Gruppe bestehend aus den Formeln RI bis RXV, 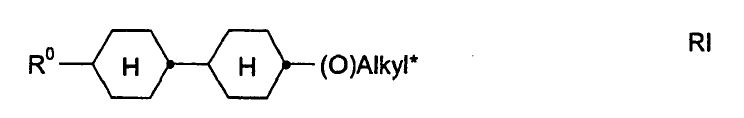 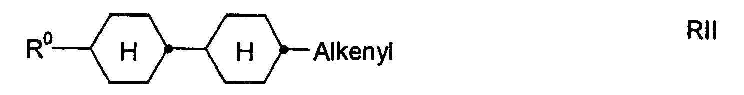 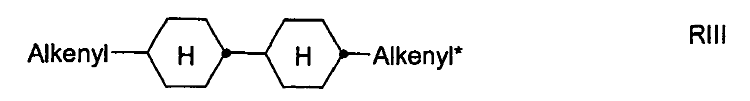 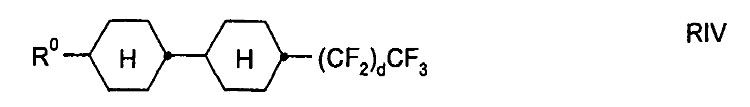 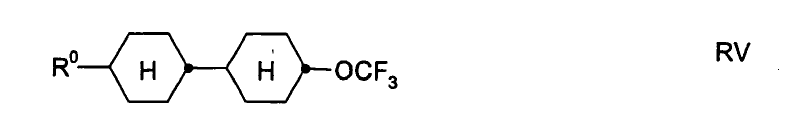 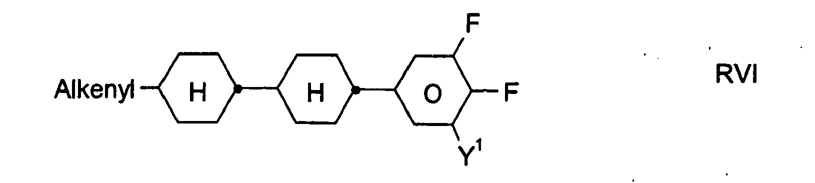 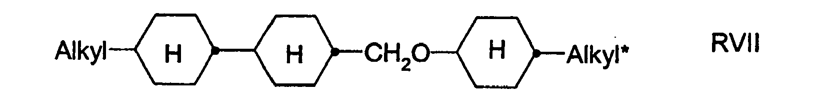 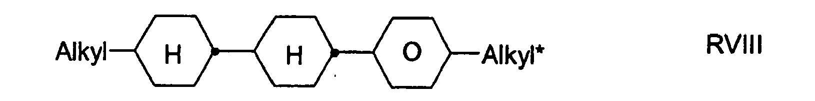 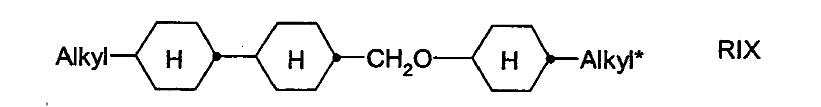 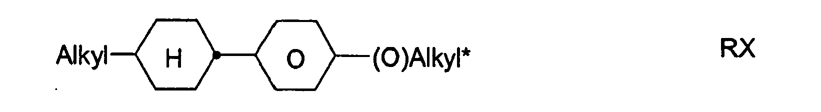 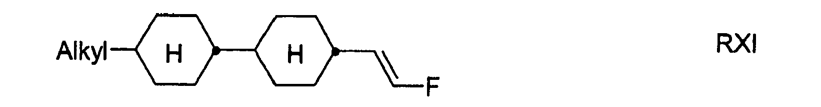 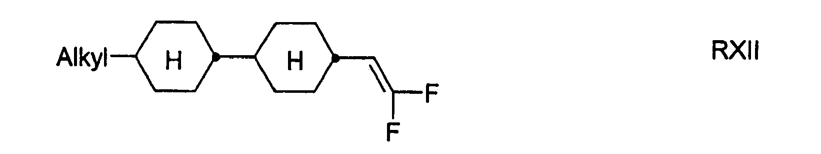 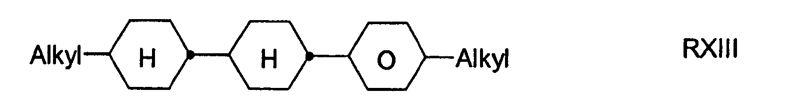 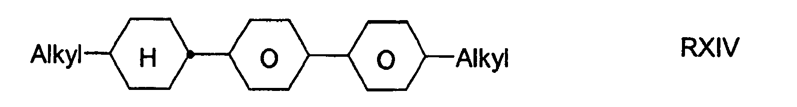 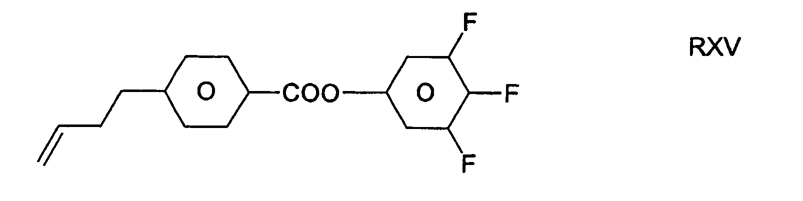 worin
   - R⁰: n-Alkyl, Oxaalkyl, Fluoralkyl, Alkenyloxy oder Alkenyl mit jeweils 2 bis 12 C-Atomen,
   - d: 0, 1 oder 2,
   - Y¹: H oder F,
   - Alkyl oder Alkyl*: jeweils unabhängig voneinander ein geradkettiger oder verzweigter Alkylrest mit 2 bis 8 C-Atomen,
   - Alkenyl oder Alkenyl*: jeweils unabhängig voneinander einen geradkettigen oder verzweigten Alkenylrest mit 2 bis 8 C-Atomen
   bedeuten.
- Das Medium enthält vorzugsweise ein oder mehrere Verbindungen der Formeln 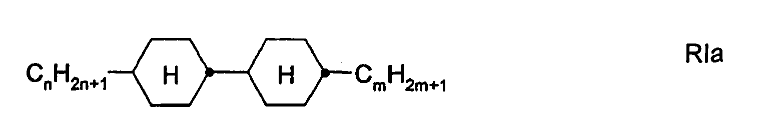 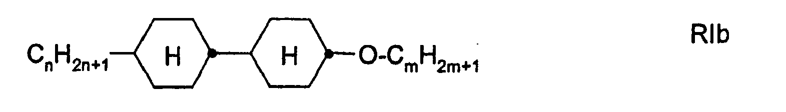 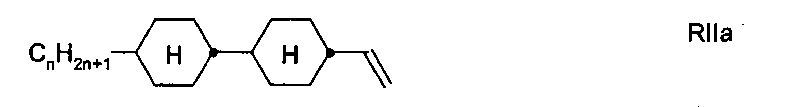 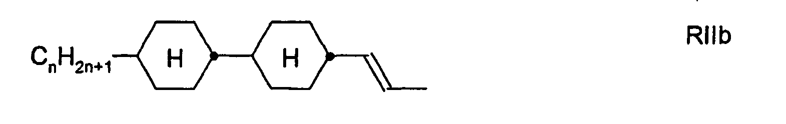 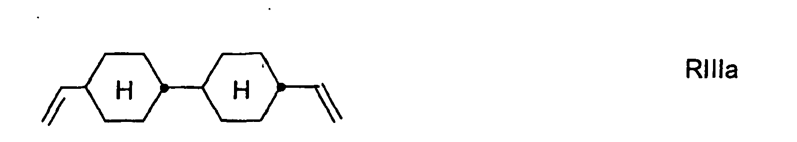 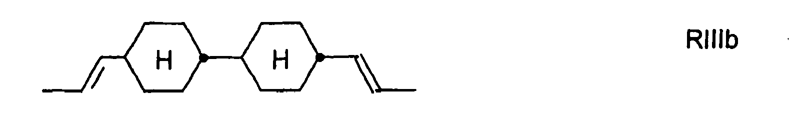 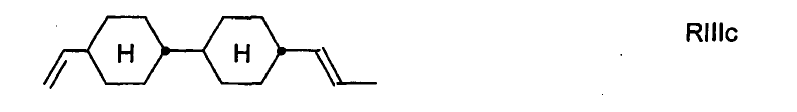 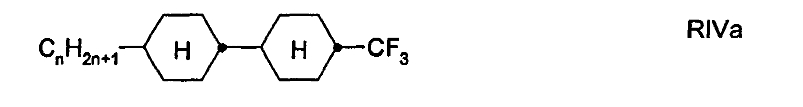 worin n und m jeweils eine ganze Zahl von 2 bis 8 bedeuten.
- Das Medium enthält eine oder mehrere Verbindungen der Formel 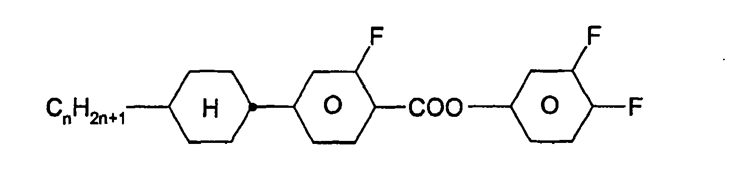 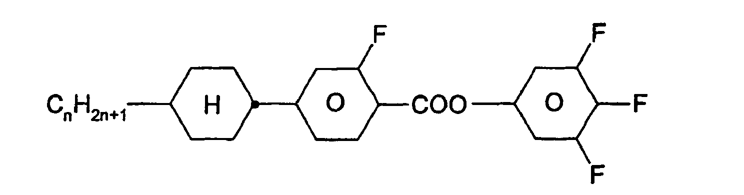 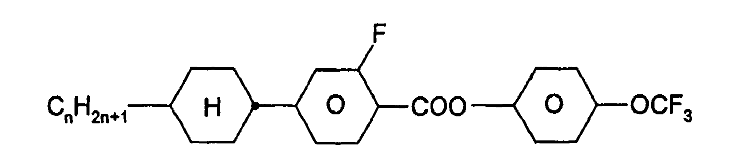 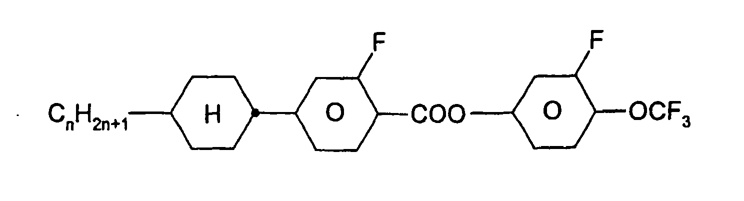 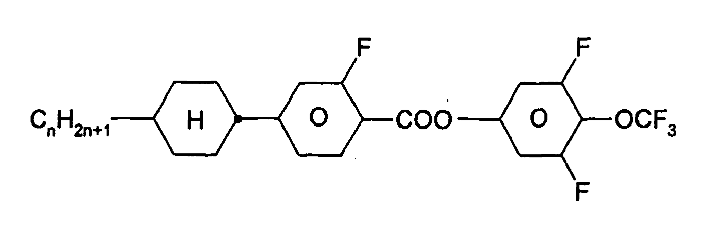
- Das Medium enthält vorzugsweise eine oder mehrere Verbindungen mit einer CF₂O-Brücke, insbesondere Verbindungen der Formel 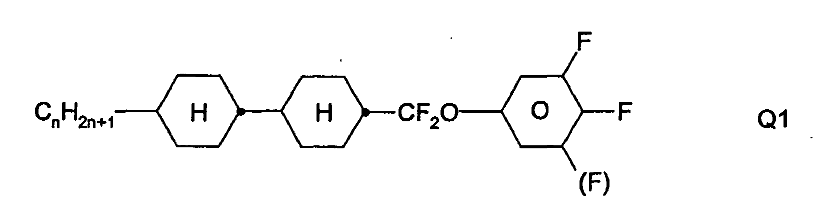 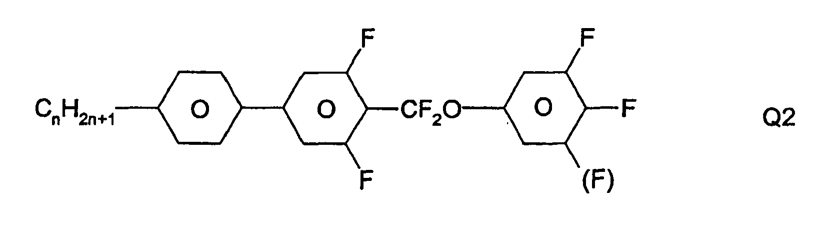 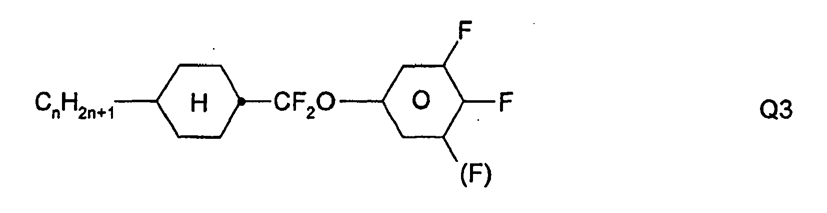
- Das Gewichtsverhältnis I: (II + III + IV + V + VI + VII + VIII + IX + X) ist vorzugsweise 1 : 10 bis 10 : 1.

Es wurde gefunden, dass bereits ein relativ geringer Anteil an Verbindungen der Formel I im Gemisch mit üblichen Flüssigkristallmaterialien, insbesondere jedoch mit einer oder mehreren Verbindungen der Formel II, III, IV, V, VI, VII, VIII, IX und/oder X zu einer beträchtlichen Erniedrigung der Schwellenspannung und zu niedrigen Werten für die Doppelbrechung führt, wobei gleichzeitig breite nematische Phasen mit tiefen Übergangstemperaturen smektisch-nematisch beobachtet werden, wodurch die Lagerstabilität verbessert wird. Die Verbindungen der Formeln I bis X sind farblos, stabil und untereinander und mit anderen Flüssigkristallmaterialien gut mischbar.

Der Ausdruck "Alkyl" oder "Alkyl*"umfasst geradkettige und verzweigte Alkylgruppen mit 2 bis 8 Kohlenstoffatomen, insbesondere die geradkettigen Gruppen Ethyl, Propyl, Butyl, Pentyl, Hexyl und Heptyl. Gruppen mit 2-5 Kohlenstoffatomen sind im allgemeinen bevorzugt.

Der Ausdruck "Alkenyl" oder "Alkenyl*" umfasst geradkettige und verzweigte Alkenylgruppen mit bis zu 8 Kohlenstoffatomen, insbesondere die geradkettigen Gruppen. Besonders bevorzugte Alkenylgruppen sind C₂-C₇-1 E-Alkenyl, C₄-C₇-3E-Alkenyl, C₅-C₇-4-Alkenyl, C₆-C₇-5-Alkenyl und C₇-6-Alkenyl, insbesondere C₂-C₇-1E-Alkenyl, C₄-C₇-3E-Alkenyl und C₅-C₇-4-Alkenyl. Beispiele bevorzugter Alkenylgruppen sind Vinyl, 1 E-Propenyl, 1 E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfasst vorzugsweise geradkettige Gruppen mit endständigen Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl" umfasst vorzugsweise geradkettige Reste der Formel CₙH₂ₙ₊₁-O-(CH₂)ₘ, worin n und m jeweils unabhängig voneinander 1 bis 6 bedeuten. Vorzugsweise ist n = 1 und m 1 bis 6.

Durch geeignete Wahl der Bedeutungen von R⁰ und X⁰ können die Ansprechzeiten, die Schwellenspannung, die Steilheit der Transmissionskennlinien etc. in gewünschter Weise modifiziert werden. Beispielsweise führen 1 E-Alkenylreste, 3E-Alkenylreste, 2E-Alkenyloxyreste und dergleichen in der Regel zu kürzeren Ansprechzeiten, verbesserten nematischen Tendenzen und einem höheren Verhältnis der elastischen Konstanten k₃₃ (bend) und k₁₁ (splay) im Vergleich zu Alkyl- bzw. Alkoxyresten. 4-Alkenylreste, 3-Alkenylreste und dergleichen ergeben im allgemeinen tiefere Schwellenspannungen und größere Werte von k₃₃/k₁₁ im Vergleich zu Alkyl- und Alkoxyresten.

Eine Gruppe -CH₂CH₂- in Z¹ führt im allgemeinen zu höheren Werten von k₃₃/k₁₁ im Vergleich zu einer einfachen Kovalenzbindung. Höhere Werte von k₃₃/k₁₁ ermöglichen z.B. flachere Transmissionskennlinien in TN-Zellen mit 90° Verdrillung (zur Erzielung von Grautönen) und steilere Transmissionskennlinien in STN-, SBE- und OMI-Zellen (höhere Multiplexierbarkeit) und umgekehrt.

Das optimale Mengenverhältnis der Verbindungen der Formeln I und II + III + IV + V + VI + VII + VIII + IX + X hängt weitgehend von den gewünschten Eigenschaften, von der Wahl der Komponenten der Formeln I, II, III, IV, V, VI, VII, VIII, IX und/oder X und von der Wahl weiterer gegebenenfalls vorhandener Komponenten ab. Geeignete Mengenverhältnisse innerhalb des oben angegebenen Bereichs können von Fall zu Fall leicht ermittelt werden.

Die Gesamtmenge an Verbindungen der Formeln I bis XVI in den erfindungsgemäßen Gemischen ist nicht kritisch. Die Gemische können daher eine oder mehrere weitere Komponenten enthalten zwecks Optimierung verschiedener Eigenschaften. Der beobachtete Effekt auf die Ansprechzeiten und die Schwellenspannung ist jedoch in der Regel umso größer je höher die Gesamtkonzentration an Verbindungen der Formeln I bis XVI ist.

In einer besonders bevorzugten Ausführungsform enthalten die erfindungsgemäßen Medien Verbindungen der Formel II bis X (vorzugsweise II und/oder III), worin X⁰ OCF₃, OCHF₂, F, OCH=CF₂, OCF=CF₂, OCF₂CHFCF₃, oder OCF₂-CF₂H bedeutet. Eine günstige synergistische Wirkung mit den Verbindungen der Formel I führt zu besonders vorteilhaften Eigenschaften.

Die erfindungsgemäßen Mischungen mit niedriger optischer Anisotropie (Δn < 0,07) sind insbesondere für reflektive Displays geeignet. Low Vₜₕ-Mischungen, sind insbesondere für 2,5 V-Treiber, 3,3 V-Treiber und 4V- oder 5V-Treiber geeignet. Für letztere Anwendungen sind Ester-freie Mischungen bevorzugt. Weiterhin sind die erfindungsgemäßen Mischungen für high Δn-IPS-Anwendungen geeignet.

Der Aufbau der erfindungsgemäßen MFK-Anzeige aus Polarisatoren, Elektrodengrundplatten und Elektroden mit Oberflächenbehandlung entspricht der für derartige Anzeigen üblichen Bauweise. Dabei ist der Begriff der üblichen Bauweise hier weit gefasst und umfasst auch alle Abwandlungen und Modifikationen der MFK-Anzeige, insbesondere auch Matrix-Anzeigeelemente auf Basis poly-Si TFT oder MIM.

Ein wesentlicher Unterschied der erfindungsgemäßen Anzeigen zu den bisher üblichen auf der Basis der verdrillten nematischen Zelle besteht jedoch in der Wahl der Flüssigkristallparameter der Flüssigkristallschicht.

Die Herstellung der erfindungsgemäß verwendbaren Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, zweckmäßig bei erhöhter Temperatur. Es ist auch möglich, Lösungen der Komponenten in einem organischen Lösungsmittel, z.B. in Aceton, Chloroform oder Methanol, zu mischen und das Lösungsmittel nach Durchmischung wieder zu entfernen, beispielsweise durch Destillation.

Die Dielektrika können auch weitere, dem Fachmann bekannte und in der Literatur beschriebene Zusätze enthalten. Beispielsweise können 0-15 % pleochroitische Farbstoffe oder chirale Dotierstoffe zugesetzt werden.

C bedeutet eine kristalline, S eine smektische, S_{C} eine smektische C, N eine nematische und I die isotrope Phase.

V₁₀ bezeichnet die Spannung für 10 % Transmission (Blickrichtung senkrecht zur Plattenoberfläche). tₒₙ bezeichnet die Einschaltzeit und t_{off} die Ausschaltzeit bei einer Betriebsspannung entsprechend dem 2-fachen Wert von V₁₀. Δn bezeichnet die optische Anisotropie und nₒ den Brechungsindex. Δε bezeichnet die dielektrische Anisotropie (Δε = ε_{∥} - ε_{┴}, wobei ε_{∥} die Dielektrizitätskonstante parallel zu den Moleküllängsachsen und ε_{⊥} die Dielektrizitätskonstante senkrecht dazu bedeutet). Die elektrooptischen Daten wurden in einer TN-Zelle im 1. Minimum (d.h. bei einem d An-Wert von 0,5) bei 20 °C gemessen, sofern nicht ausdrücklich etwas anderes angegeben wird. Die optischen Daten wurden bei 20 °C gemessen, sofern nicht ausdrücklich etwas anderes angegeben wird.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. n und m bedeuten jeweils unabhängig voneinander 1, 2, 3, 4, 5, 6, 7, 8, 9, 10, 11, 12, 13, 14 oder 15. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R₂ | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nF.F.F | CₙH₂ₙ₊₁ | F | F | F |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-C₂H₂ₛ- | CN | H | H |
| nAm | CₙH₂ₙ₊₁ | COOCₘH₂ₘ₊₁ | H | H |
| nOCCF₂.F.F | CₙH₂ₙ₊₁ | OCH₂CF₂H | F | F |

Bevorzugte Mischungskomponenten finden sich in den Tabellen A und B.

**Tabelle A:**

| | |
|---|---|
| 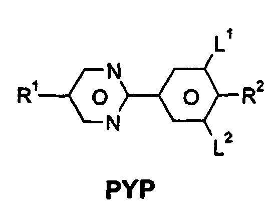 | 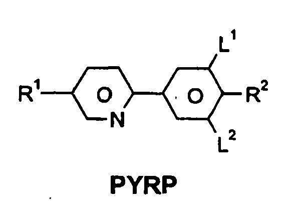 |
| 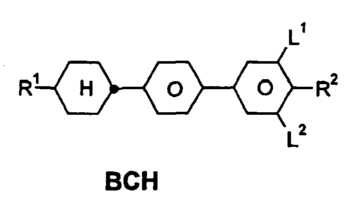 | 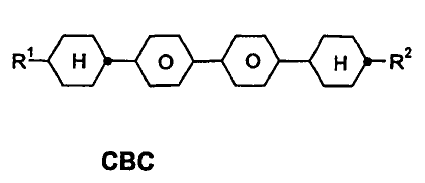 |
| 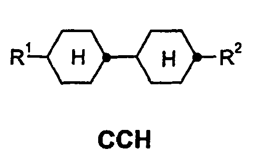 | 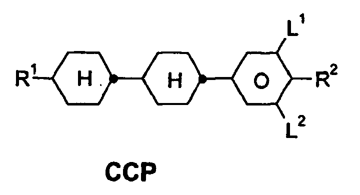 |
| 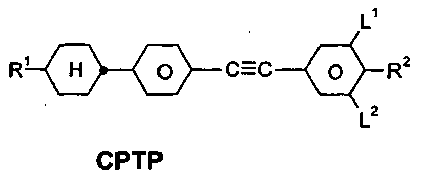 | |
| 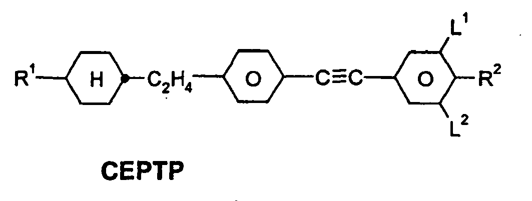 | |
| 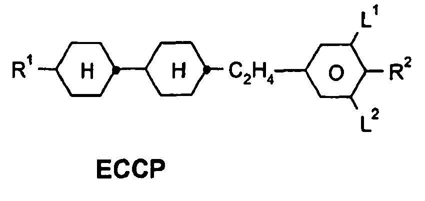 | 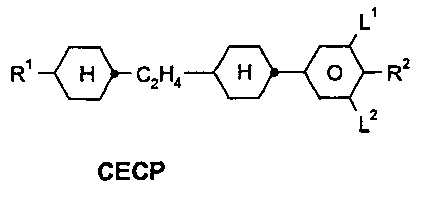 |
| 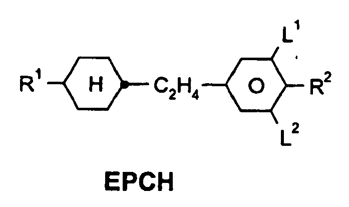 | 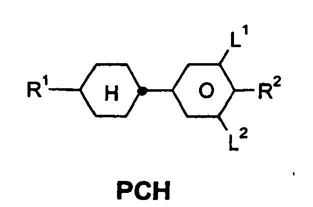 |
| 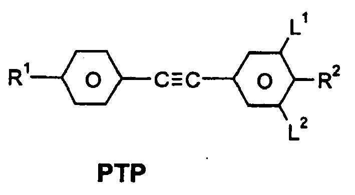 | 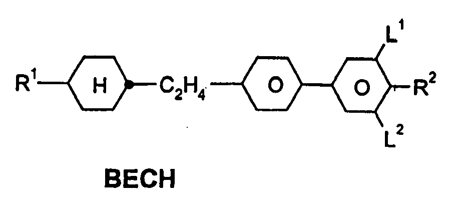 |
| 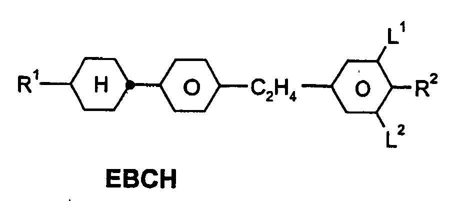 | 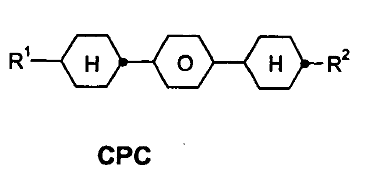 |
| 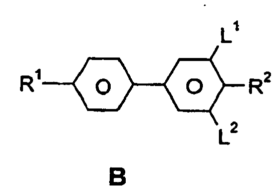 | 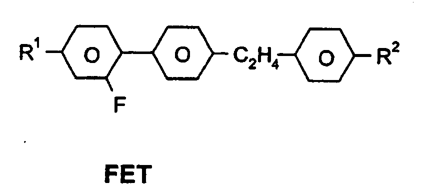 |
| 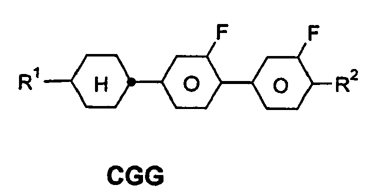 | 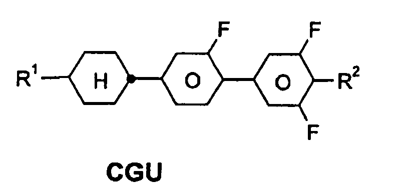 |
| 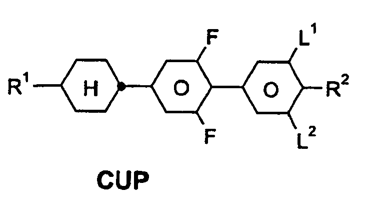 | 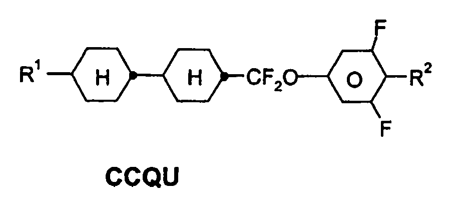 |
| 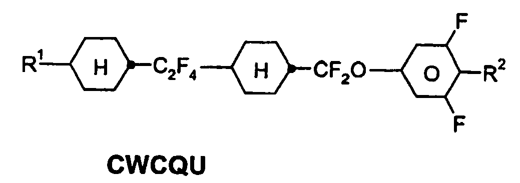 | |
| 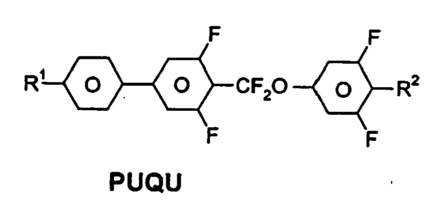 | |

**Tabelle B:**

| | |
|---|---|
| 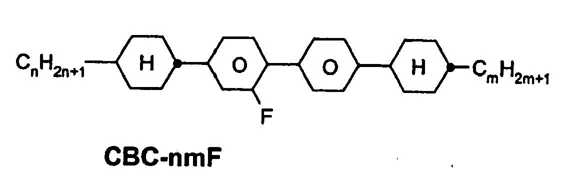 | |
| 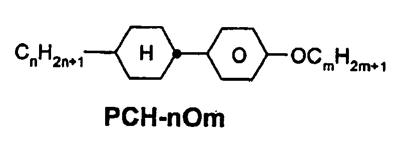 | |
| 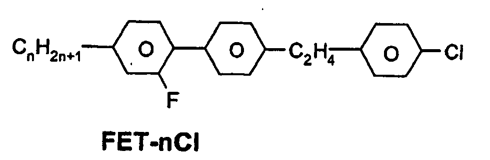 | |
| 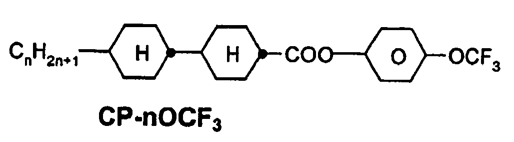 | |
| 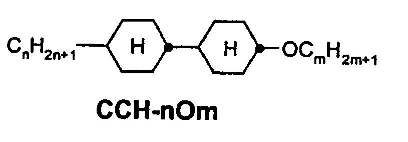 | |
| 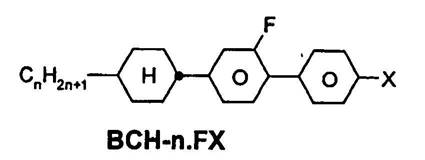 | |
| 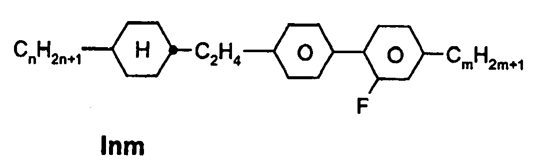 | |
| 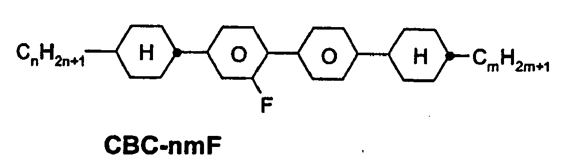 | |
| 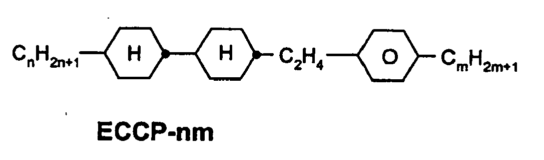 | |
| 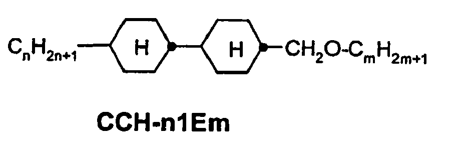 | |
| 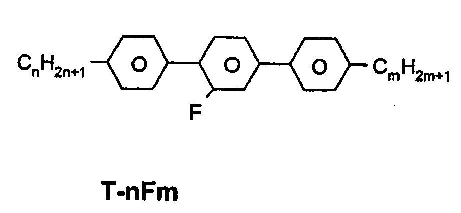 | 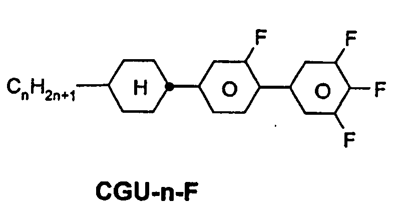 |
| 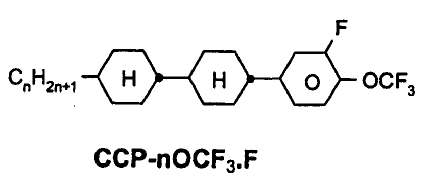 | 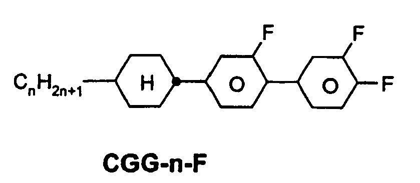 |
| 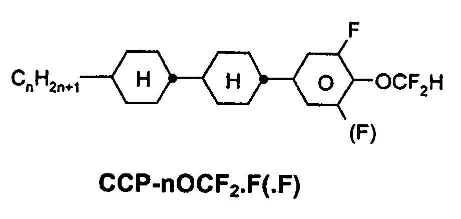 | 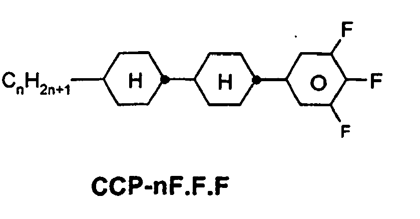 |
| 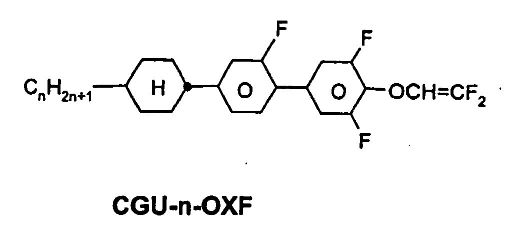 | 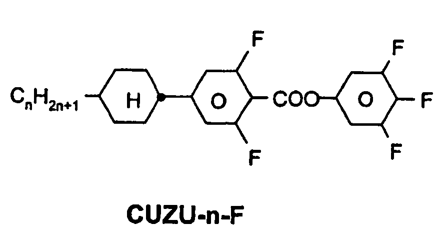 |
| 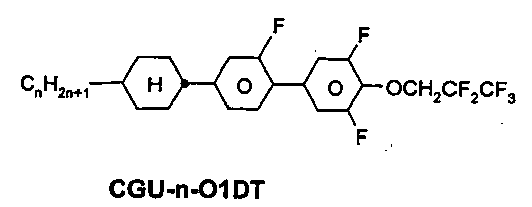 | |
| 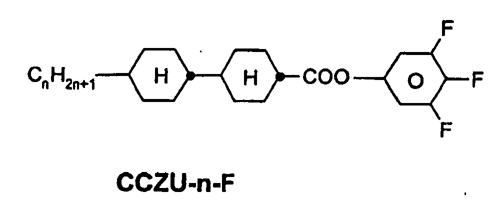 | |
| 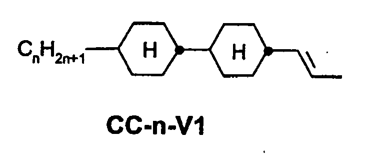 | 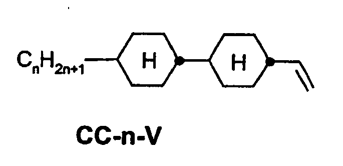 |
| 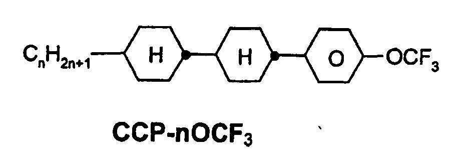 | |
| 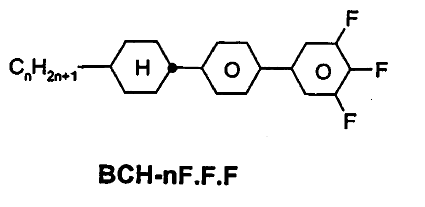 | |
| 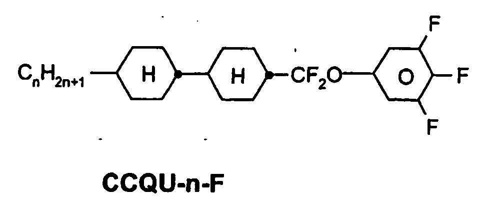 | |
| 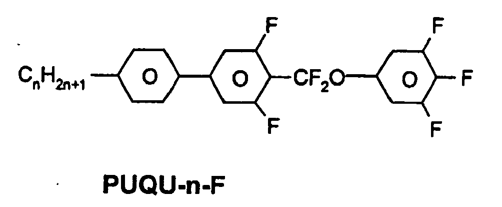 | |
| 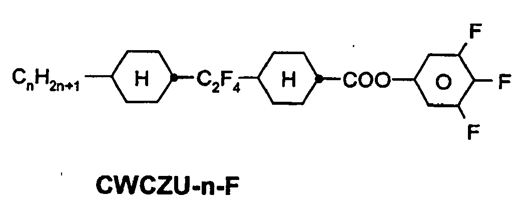 | |
| 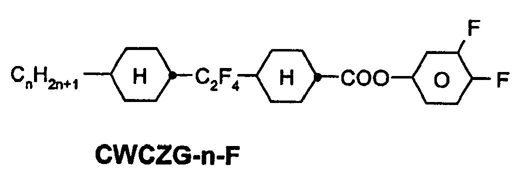 | |
| 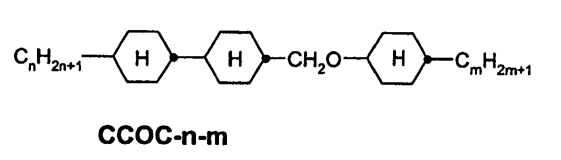 | |
| 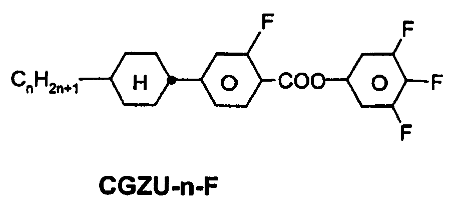 | |
| 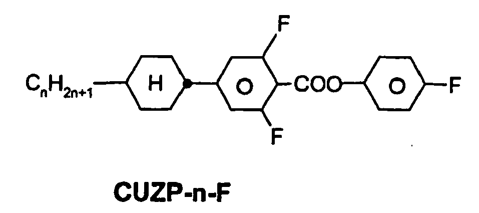 | |
| 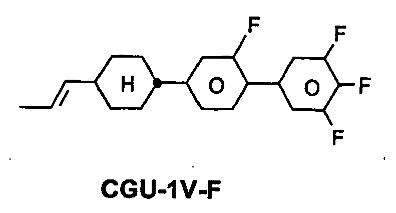 | 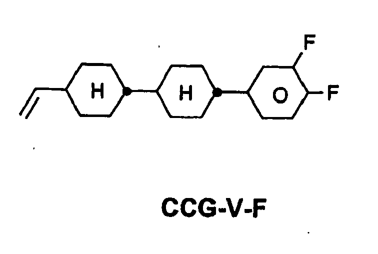 |
| 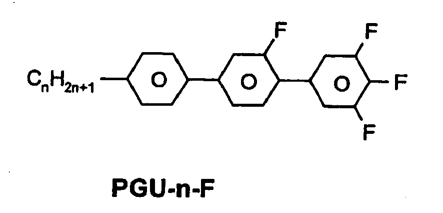 | |
| 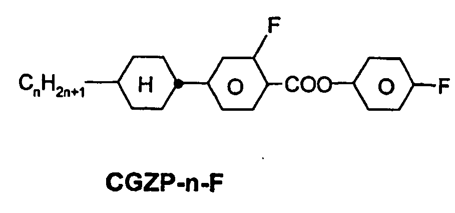 | 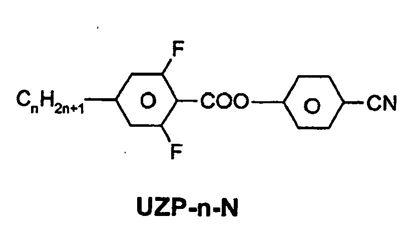 |
| 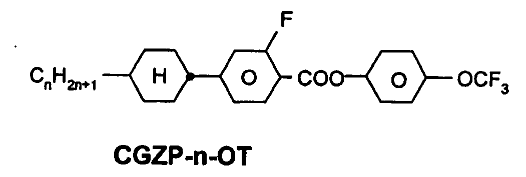 | |
| 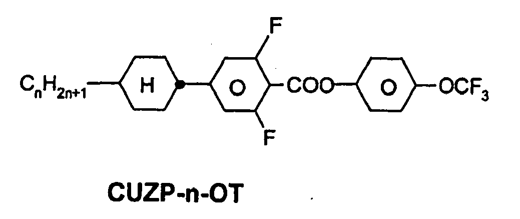 | |
| 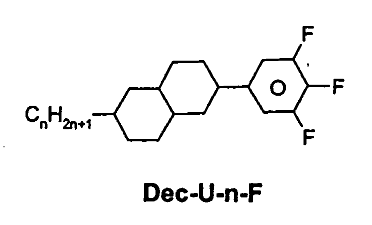 | |
| 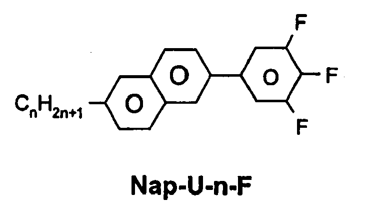 | 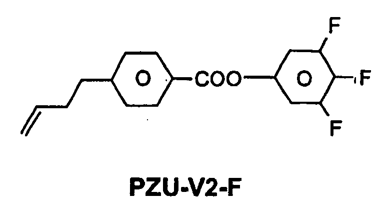 |
| 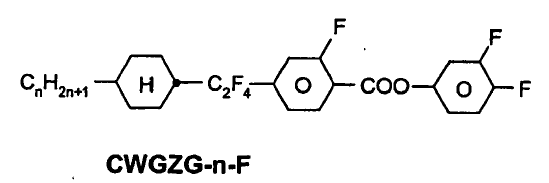 | |
| 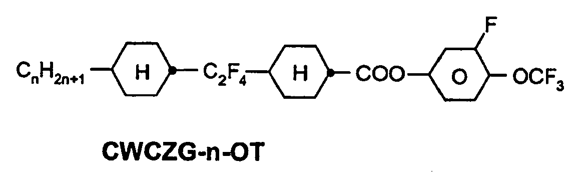 | |
| 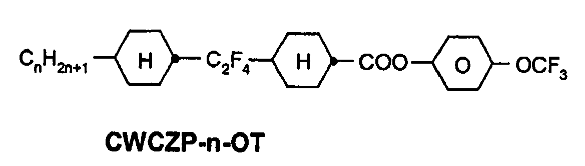 | |
| 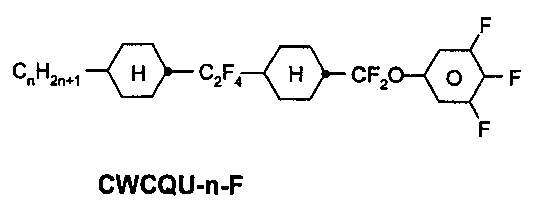 | |
| 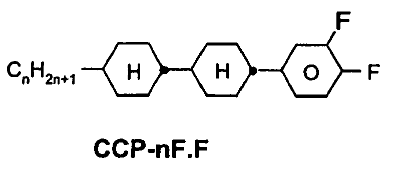 | |
| 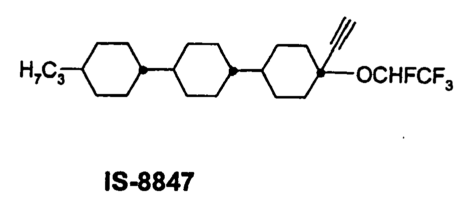 | 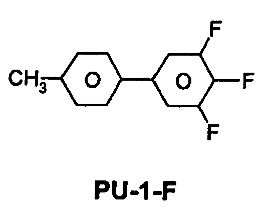 |
| 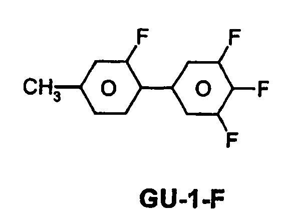 | 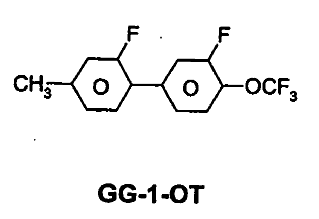 |
| 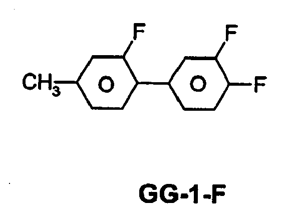 | 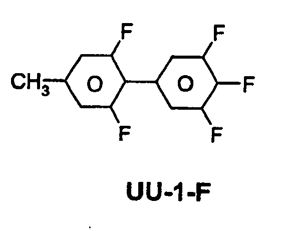 |
| 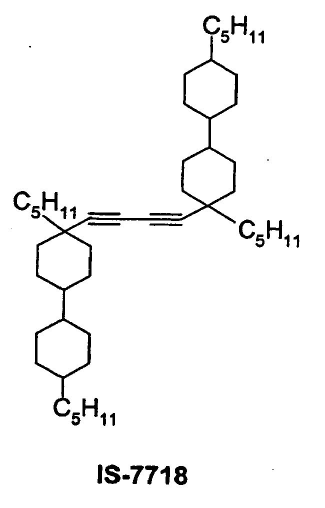 | |

**Tabelle C:**

| | |
|---|---|
| In der Tabelle C werden mögliche Dotierstoffe angegeben, die in der Regel den erfindungsgemäßen Mischungen zugesetzt werden. | |
| 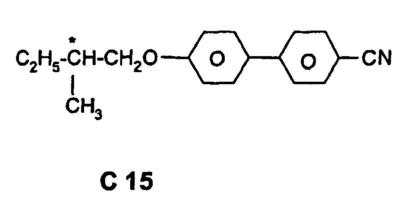 | 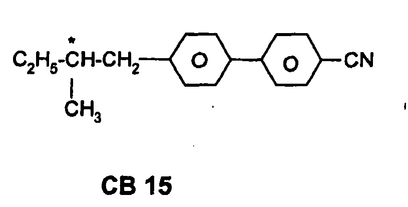 |
| 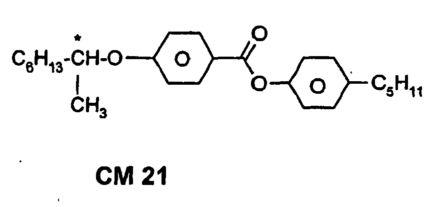 | |
| 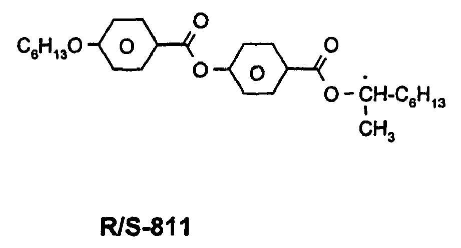 | |
| 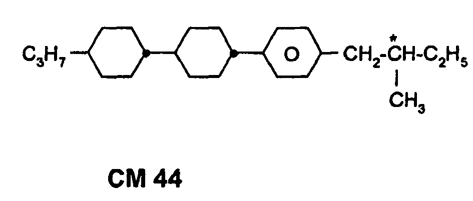 | |
| 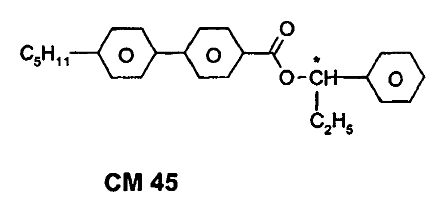 | 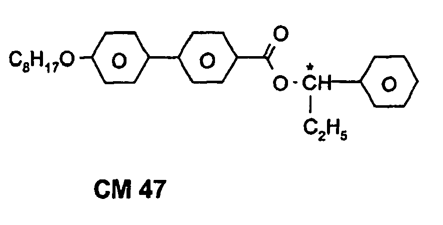 |
| 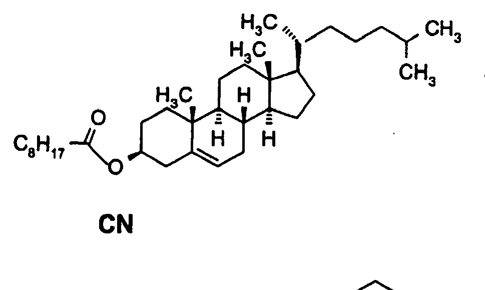 | |
| 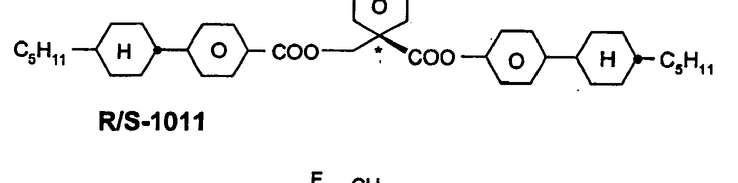 | |
| 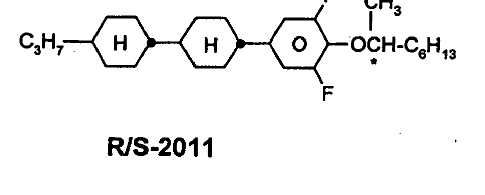 | |
| 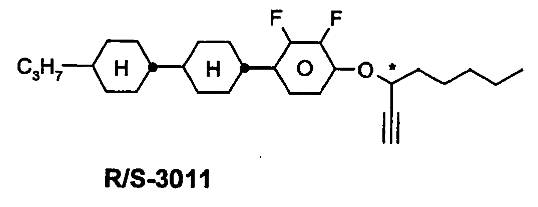 | |
| 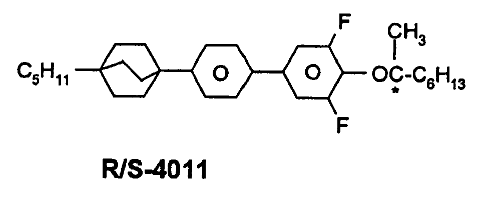 | |

**Tabelle D:**

| | |
|---|---|
| Stabilisatoren, die beispielsweise den erfindungsgemäßen Mischungen zugesetzt werden können, werden nachfolgend genannt. | |
| 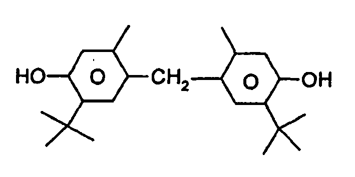 | 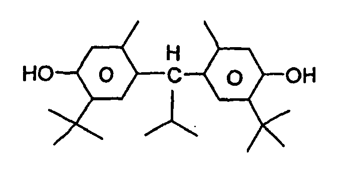 |
| 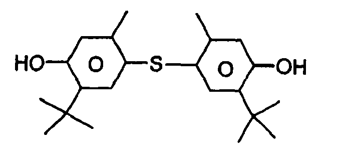 | 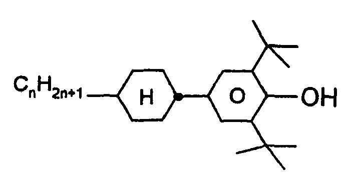 |
| 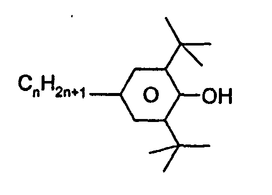 | 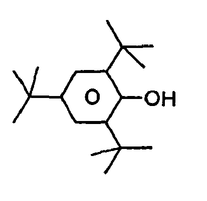 |
| 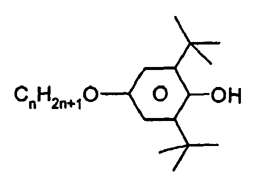 | 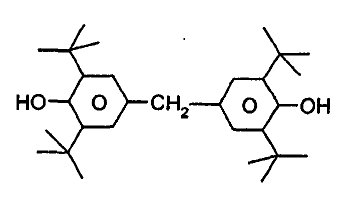 |
| 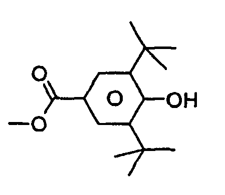 | 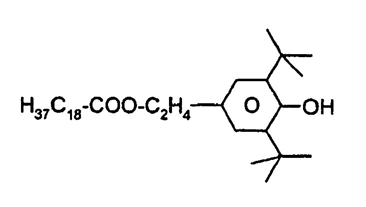 |
| 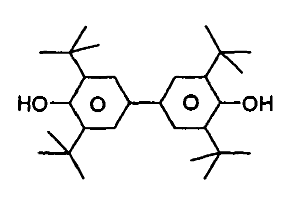 | 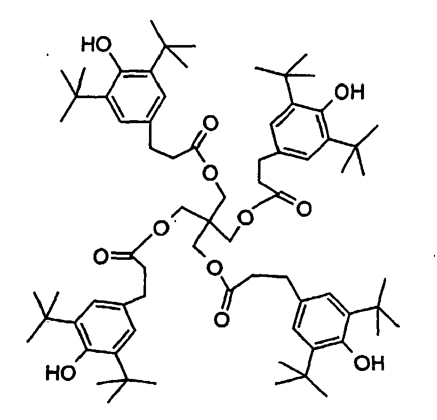 |
| 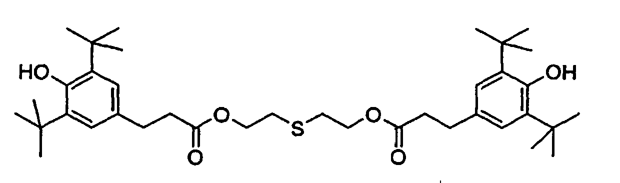 | |
| 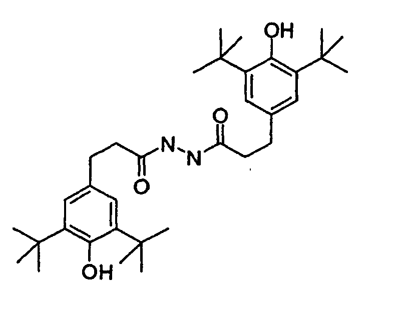 | 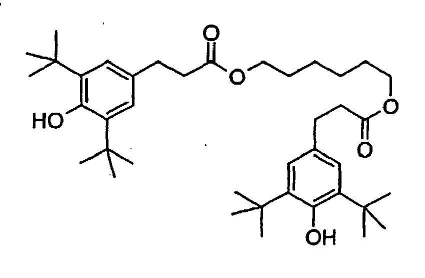 |
| 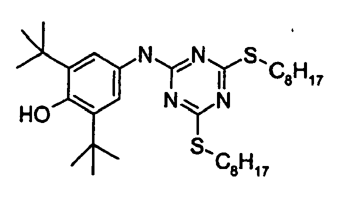 | 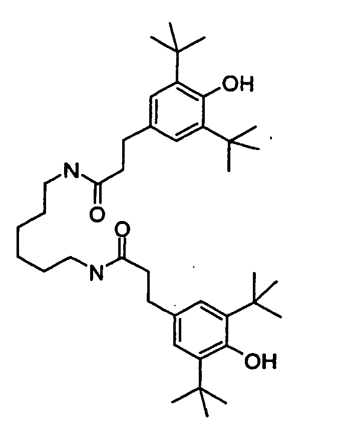 |
| 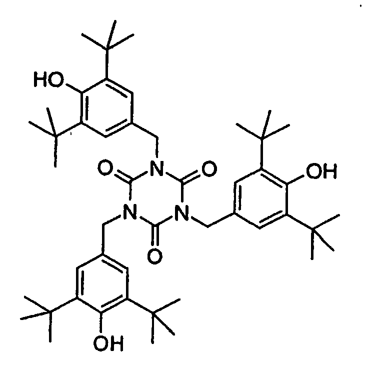 | 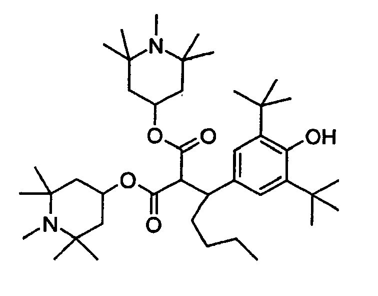 |
| 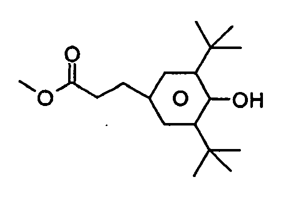 | 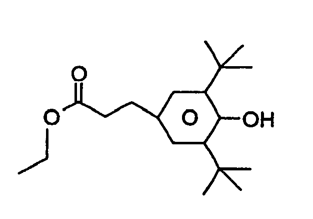 |
| 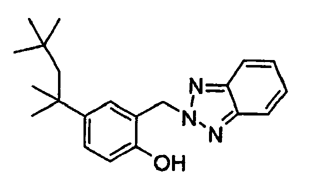 | 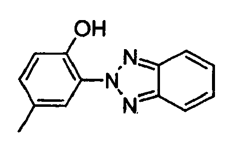 |
| 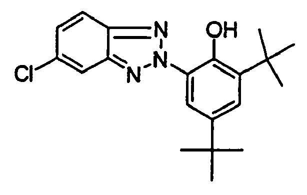 | 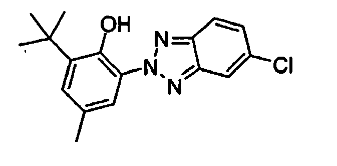 |
| 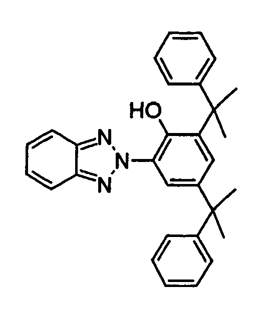 | 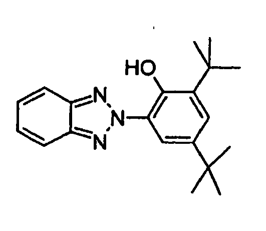 |
| 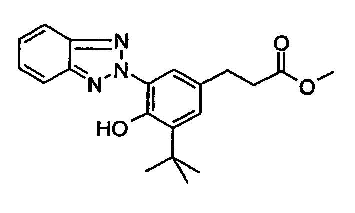 | 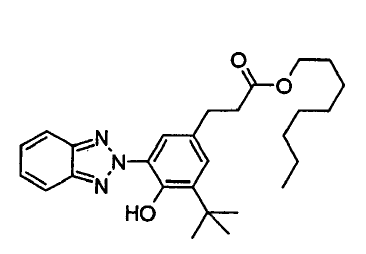 |
| 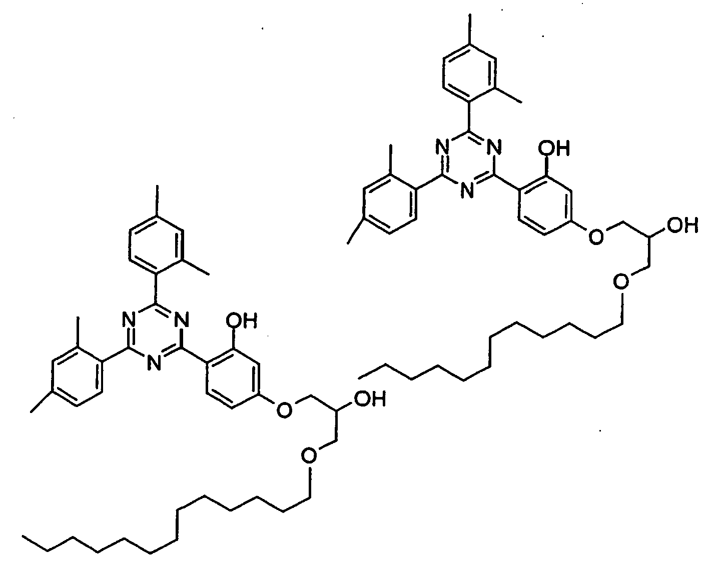 | |
| 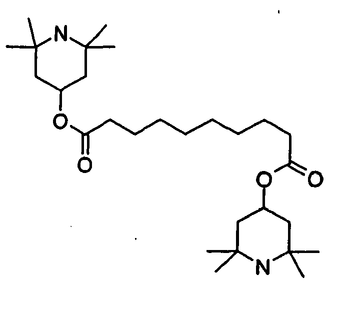 | 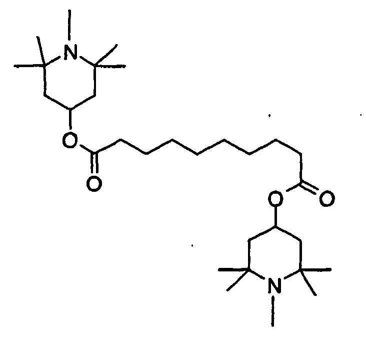 |

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C), Δε bedeutet dielektrische Anisotropie (1 kHz, 20 °C), die Fließviskosität ν₂₀ (mm²/sec) wurde bei 20 °C bestimmt. Die Rotationsviskosität γ1 (mPa·s) wurde ebenfalls bei 20 °C bestimmt.

"Übliche Aufarbeitung" bedeutet: man gibt gegebenenfalls Wasser hinzu, extrahiert mit Dichlormethan, Diethylether, Methyl-tert.Butylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- n-BuLi: 1,6 molare Lösung von n-Butyllithium in n-Hexan
- DMAP: 4-(Dimethylamino)-pyridin
- THF: Tetrahydrofuran
- DCC: N,N'-Dicyclohexylcarbodiimid

### Beispiel 1

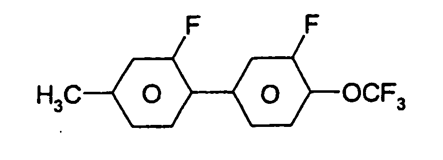

### Schritt 1.1

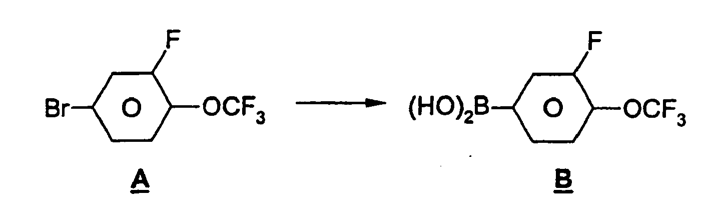

1,1 mol Magnesiumspäne in 200 ml abs. THF werden unter Rückfluss erhitzt und mit 1,0 ml A in 400 ml abs. THF versetzt. Man lässt 1 h unter Rückfluss kochen, kühlt auf 0-5 °C ab und tropft eine Lösung aus 1,0 mol Trimethylborat in 200 ml abs. THF hinzu. Bei 0-5 °C wird 15 Minuten nachgerührt. Nach Zugabe von 200 ml H₂O wird bei 15 °C mit konz. HCl angesäuert und mit 200 ml Methyl-tert. Butylether versetzt. Die organische Phase wird zuletzt wie üblich aufgearbeitet.

### Schritt 1.2

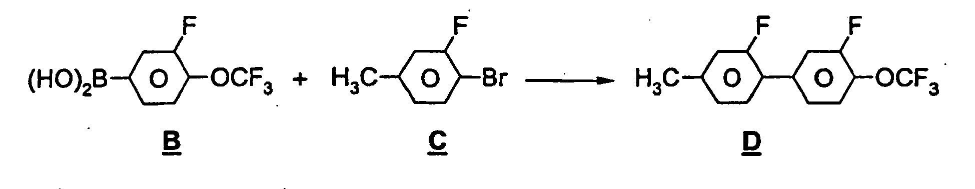

Zu 29,6 mmol Natriummetaborat-octahydrat in 39,5 ml H₂O werden 0,8 mmol Bis(triphenylphosphin)palladium, 0,82 mmol Hydraziniumhydroxid und 38,9 mmol C zugegeben und 5 Minuten gerührt. Nach Zugabe von 38,8 mmol B gelöst in 60 ml abs. THF wird über Nacht unter Rückfluss gekocht. Man lässt auf Raumtemperatur abkühlen, versetzt mit 30 ml Methyl-tert. Butylether und trennt die organische Phase ab. Zuletzt wird wie üblich aufgearbeitet.
K 16 I; Δn = 0,1310; Δε = 18,4; γ₁ = 26; ν₂₀ = 8

Analog werden die folgenden Verbindungen hergestellt:

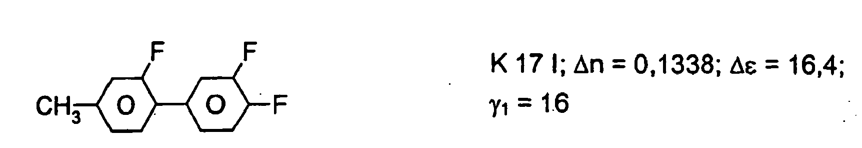

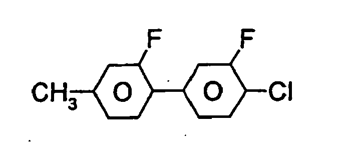

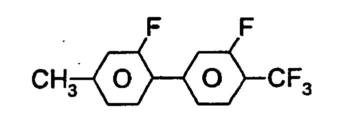

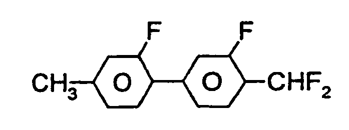

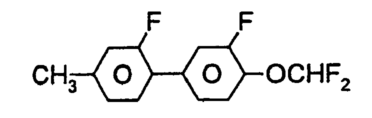

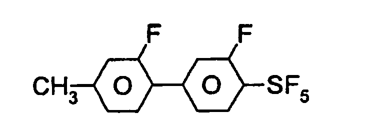

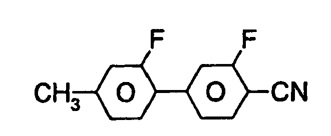

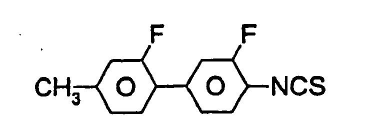

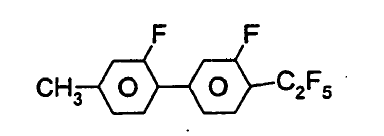

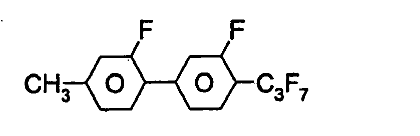

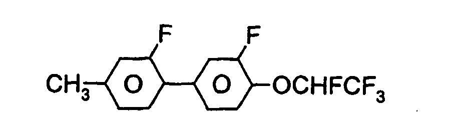

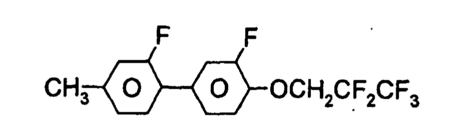

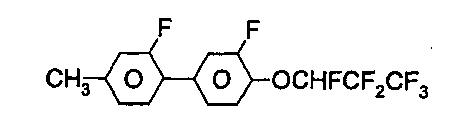

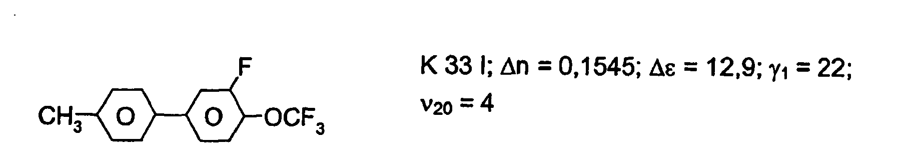

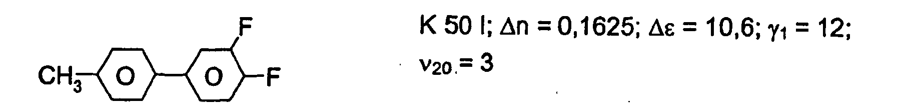

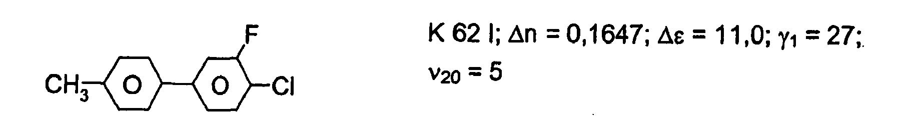

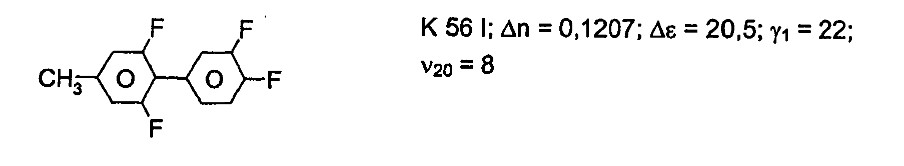

### Beispiel 2

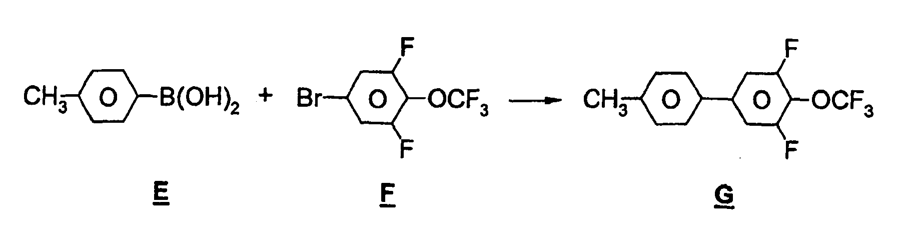

Zu 31,4 mmol Natriummetaborat-octahydrat in 42 ml H₂O werden 0,9 mmol Bis(triphenylphosphin)palladium, 0,8 mmol Hydraziniumhydroxid, 41,3 mmol F und 30 ml abs. THF zugegeben und 5 Minuten gerührt.

Nach Zugabe von E gelöst in 90 ml abs. THF wird 3 h unter Rückfluss erhitzt. Man lässt auf Raumtemperatur abkühlen und versetzt mit Wasser und Methyl-tert. Butylether. Die organische Phase wird abgetrennt und wie üblich aufgearbeitet.
K 37 I; Δn = 0,1413; Δε = 17,0; γ₁ = 29; ν₂₀ = 7

Analog werden die folgenden Verbindungen der Formel 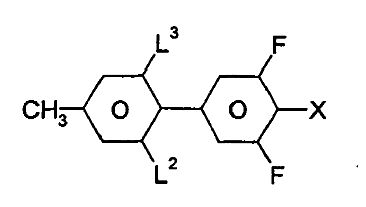 hergestellt:

| X | L² | L³ | |
|---|---|---|---|
| OCF₃ | H | F | K 53 I; Δn = 0,1223; Δε = 22,6; γ₁ = 37; ν₂₀ = 10 |
| F | H | H | K 27 I; Δn = 0,1388; Δε = 16,9; γ₁ = 13; V₂₀ = 3 |
| F | H | F | K 63 I; Δn = 0,1227; Δε = 21,1; γ₁ = 15; ν₂₀ =5 |
| CI | H | H | |
| CI | H | F | |
| CF₃ | H | H | K 63 I; Δn = 0,1530; Δε = 24,4; γ₁ = 31 |
| CF₃ | H | F | K 67 I; Δn = 0,1275; Δε = 28,2; γ₁ = 33 |
| CHF₂ | H | H | |
| CHF₂ | H | F | |
| OCHF₂ | H | H | |
| OCHF₂ | H | F | |
| SF₅ | H | H | |
| SF₅ | H | F | |
| CN | H | H | |
| CN | H | F | |
| F | F | F | K 73 I; Δn = 0,1013; Δε = 25,9; γ₁ = 22 |
| OCF₃ | F | F | K 77 I; Δn = 0,1088; Δε = 23,1 |
| NCS | H | H | |
| NCS | H | F | |
| C₂F₅ | H | H | |
| C₂F₅ | H | F | |
| C₃F₇ | H | H | K 84 I; Δn = 0,1387; Δε = 17,1 |
| C₃F₇ | H | F | K 32 I; Δn = 0,1215; Δε = 21,3 |
| OCHFCF₃ | H | H | K 55 I; Δn = 0,1417; Δε = 21,5; γ₁ = 56 |
| OCHFCF₃ | H | F | K 20 I; Δn = 0,1279; Δε = 25,3; γ₁ = 64 |
| OCH₂CF₂CF₃ | H | H | |
| OCH₂CF₂CF₃ | H | F | |
| OCHFCF₂CF₃ | H | H | |
| OCHFCF₂CF₃ | H | F | |

### Mischungsbeispiele

### Beispiel M1

| | | | |
|---|---|---|---|
| CCH-35 | 4,00 % | Klärpunkt [°C]: | 80,5 |
| CC-3-V1 | 9,00 % | Δn [589 nm, 20 °C]: | 0,1043 |
| CC-5-V | 8,50 % | γ₁ [mPa·s, 20 °C]: | 88 |
| CCP-20CF₃ | 7,00 % | V₁₀ [V]: | 1,37 |
| CCP-30CF₃ | 7,00 % | | |
| CCP-40CF₃ | 4,00 % | | |
| PG U-2-F | 8,50 % | | |
| CCZU-3-F | 14,00 % | | |
| CGZP-2-OT | 9,00 % | | |
| CGZP-3-OT | 7,00 % | | |
| GG-1-OT | 15,00 % | | |
| BCH-32 | 4,00 % | | |
| CBC-33 | 3,00 % | | |

### Beispiel M2

| | | | |
|---|---|---|---|
| CCP-2F.F.F | 10,00 % | S → N [°C]: | < -40 |
| CCP-20CF₃ | 7,00 % | Klärpunkt [°C]: | 78,0 |
| CCP-30CF₃ | 7,00 % | Δn [589 nm, 20 °C]: | 0,0901 |
| CCP-40CF₃ | 6,00 % | γ₁ [mPa·s, 20 °C]: | 130 |
| CCP-20CF₃.F | 12,00 % | | |
| CCZU-2-F | 5,00 % | | |
| CCZU-3-F | 15,00 % | | |
| CCZU-5-F | 4,00 % | | |
| CGZP-2-OT | 7,00 % | | |
| CGZP-3-OT | 7,00 % | | |
| CCGU-3-F | 4,00 % | | |
| CCH-3CF₃ | 1,00 % | | |
| GG-1-OT | 15,00 % | | |

### Beispiel M3

| | |
|---|---|
| CCP-1 F.F.F | 6,00 % |
| CCP-2F.F.F | 7,00 % |
| CCP-20CF₃.F | 12,00 % |
| CCZU-2-F | 5,00 % |
| CCZU-3-F | 15,00 % |
| CCZU-5-F | 5,00 % |
| CGZP-1-OT | 12,00 % |
| CGZP-2-OT | 8,00 % |
| CGZP-3-OT | 6,00 % |
| CCC-3-V | 9,00 % |
| GG-1-OT | 15,00 % |

### Beispiel M4

| | | | |
|---|---|---|---|
| CCP-1F.F.F | 5,00 % | S → N [°C]: | < -40 |
| CCP-2F.F.F | 8,00 % | Klärpunkt [°C]: | 82,0 |
| CCP-3F.F.F | 3,00 % | Δn [589 nm, 20 °C]: | 0,0887 |
| CCP-20CF₃.F | 5,00 % | γ₁ [mPa·s, 20 °C]: | 135 |
| CCP-20CF₃ | 6,00 % | | |
| CCP-30CF₃ | 4,00 % | | |
| CCZU-2-F | 4,00 % | | |
| CCZU-3-F | 15,00 % | | |
| CCZU-5-F | 5,00 % | | |
| CGZP-2-OT | 11,00 % | | |
| CGZP-3-OT | 9,00 % | | |
| CCOC-3-3 | 4,00 % | | |
| GG-1-OT | 11,00 % | | |
| CCGU-3-F | 4,00 % | | |
| CC-3-V | 6,00 % | | |

### Beispiel M5

| | | | |
|---|---|---|---|
| CC-5-V | 7,00 % | S → N [°C]: | < -20 |
| CC-3-V1 | 10,00 % | Klärpunkt [°C]: | 74,5 |
| CCH-35 | 5,00 % | Δn [589 nm, 20 °C]: | 0,0773 |
| CC-3-V | 18,00 % | γ₁ [mPa·s, 20 °C]: | 57 |
| GG-1-OT | 12,00 % | V₁₀ [V]: | 1,84 |
| CCP-V-1 | 7,00 % | | |
| CCZU-3-F | 15,00 % | | |
| CCP-30CF₃ | 8,00 % | | |
| CCP-40CF₃ | 8,00 % | | |
| CGZP-2-OT | 10,00 % | | |

### Beispiel M6

| | | | |
|---|---|---|---|
| CC-3-V | 6,00 % | S → N [°C]: | < -40 |
| CCP-2F.F.F | 7,00 % | Klärpunkt [°C]: | 71,0 |
| CCP-30CF₃ | 8,00 % | Δn [589 nm, 20 °C]: | 0,0887 |
| CCP-40CF₃ | 6,00 % | Δε [1 kHz, 20 °C]: | 12,3 |
| CCZU-2-F | 5,00 % | γ₁ [mPa·s, 20 °C]: | 108 |
| CCZU-3-F | 15,00 % | V₁₀[V]: | 1,08 |
| CCZU-5-F | 5,00 % | | |
| CGZP-2-OT | 11,00 % | | |
| CGZP-3-OT | 7,00 % | | |
| CCOC-3-3 | 3,00 % | | |
| CCOC-4-3 | 3,00 % | | |
| CCGU-3-F | 3,00 % | | |
| GG-1-OT | 21,00 % | | |

### Beispiel M7

| | | | |
|---|---|---|---|
| CC-5-V | 4,00 % | S → N [°C]: | < -30 |
| CC-3-V1 | 11,00 % | Klärpunkt [°C]: | 78,0 |
| CCH-35 | 5,00 % | Δn [589 nm, 20 °C]: | 0,0781 |
| CC-3-V | 18,00 % | γ1 [mPa·s, 20 °C]: | 61 |
| GG-1-OT | 12,00 % | V₁₀ [V]: | 1,78 |
| CCZU-2-F | 4,00 % | | |
| CCZU-3-F | 15,00 % | | |
| CCP-30CF₃ | 8,00 % | | |
| CCP-40CF₃ | 8,00 % | | |
| CGZP-2-OT | 7,00 % | | |
| CBC-33 | 1,00 % | | |
| CCP-V-1 | 7,00 % | | |

### Beispiel M8

| | |
|---|---|
| GG-1-OT | 10,00 % |
| CC-5-V | 5,00 % |
| CC-3-V1 | 5,00 % |
| CGU-2-F | 11,00 % |
| CGU-3-F | 11,00 % |
| CGU-5-F | 9,00 % |
| BCH-3F.F.F | 15,00 % |
| BCH-5F.F.F | 10,00 % |
| PGU-2-F | 9,00 % |
| PGU-3-F | 5,00 % |
| CCP-V-1 | 10,00 % |

### Beispiel M9

| | | | |
|---|---|---|---|
| CC-3-V | 9,50 % | Klärpunkt [°C]: | 73,0 |
| GG-1-OT | 12,00 % | Δn [589 nm, 20 °C]: | 0,1472 |
| PGU-2-F | 11,00 % | Δε [kHz, 20 °C]: | 12,0 |
| PGU-3-F | 11,00 % | γ₁ [mPa·s, 20 °C]: | 106 |
| PGU-5-F | 10,00 % | V₀ [V]: | 1,02 |
| CGZP-2-OT | 10,00 % | | |
| CGZP-3-OT | 4,00 % | | |
| BCH-2F.F | 6,00 % | | |
| PGIGI-3-F | 3,00 % | | |
| BCH-32 | 6,00 % | | |
| CCP-V-1 | 15,00 % | | |
| CBC-33 | 2,50 % | | |

### Beispiel M10

| | | | |
|---|---|---|---|
| CC-3-V | 2,00 % | Klärpunkt [°C]: | 69,5 |
| CCP-2F.F.F | 9,00 % | Δn [589 nm, 20 °C]: | 0,0889 |
| CCP-20CF₃.F | 7,00 % | γ₁ [mPa·s, 20 °C]: | 118 |
| CCP-30CF₃ | 6,00 % | V₁₀ [V]: | 1,03 |
| CCP-40CF₃ | 6,00 % | | |
| PGU-2-F | 1,00 % | | |
| CCZU-2-F | 5,00 % | | |
| CCZU-3-F | 15,00 % | | |
| CCZU-5-F | 5,00 % | | |
| CGZP-2-OT | 11,00 % | | |
| CGZP-3-OT | 9,00 % | | |
| CCOC-3-3 | 2,00 % | | |
| CCOC-4-3 | 3,00 % | | |
| GG-1-OT | 19,00 % | | |

### Beispiel M11

| | |
|---|---|
| CC-3-V | 15,00 % |
| CCGU-3-F | 3,00 % |
| CCP-30CF₃ | 8,00 % |
| CCP-40CF₃ | 6,00 % |
| CCZU-2-F | 5,00 % |
| CCZU-3-F | 15,00 % |
| CCZU-5-F | 5,00 % |
| CGZP-2-OT | 11,00 % |
| CGZP-3-OT | 9,00 % |
| CCOC-3-3 | 3,00 % |
| GG-1-OT | 20,00 % |

### Beispiel M12

| | |
|---|---|
| CC-5-V | 6,00 % |
| CC-3-V1 | 10,00 % |
| CCH-35 | 5,00 % |
| CC-3-V | 18,00 % |
| GG-1-OT | 12,00 % |
| CCP-V-1 | 4,00 % |
| CCZU-3-F | 14,00 % |
| CCP-30CF₃ | 8,00 % |
| CCP-40CF₃ | 8,00 % |
| CCP-50CF₃ | 4,00 % |
| CGZP-2-OT | 10,00% |
| CBC-33 | 1,00 % |

### Beispiel M13

| | | | |
|---|---|---|---|
| CC-5-V | 8,50 % | S → N [°C]: | < -20 |
| CC-3-V1 | 11,00 % | Klärpunkt [°C]: | 78,5 |
| CCH-35 | 5,00 % | Δn [589 nm, 20 °C]: | 0,0791 |
| CC-3-V | 18,00 % | γ₁ [mPa·s, 20 °C]: | 61 |
| GG-1-OT | 12,00 % | V₁₀ [V]: | 1,80 |
| CCZU-3-F | 15,50 % | | |
| CCP-30CF₃ | 8,00 % | | |
| CCP-40CF₃ | 8,00 % | | |
| CGZP-2-OT | 10,00 % | | |
| CBC-33 | 4,00 % | | |

### Beispiel M14

| | | | |
|---|---|---|---|
| CC-5-V | 3,50 % | S → N [°C]: | < -30 |
| CC-3-V1 | 11,00 % | Klärpunkt [°C]: | 78,5 |
| CCH-35 | 5,00 % | Δn [589 nm, 20 °C]: | 0,0791 |
| CC-3-V | 18,00 % | γ₁ [mPa·s, 20 °C]: | 61 |
| GG-1-OT | 12,00 % | V₁₀[V]: | 1,76 |
| CCZU-2-F | 4,00 % | | |
| CCZU-3-F | 15,00 % | | |
| CCP-30CF₃ | 8,00 % | | |
| CCP-40CF₃ | 8,00 % | | |
| CGZP-2-OT | 8,00 % | | |
| CBC-33 | 1,50% | | |
| CCP-V-1 | 6,00 % | | |

### Beispiel M15

| | | | |
|---|---|---|---|
| CC-3-V | 6,00 % | S → N [°C]: | < -12,0 |
| GG-1-OT | 10,00 % | Klärpunkt [°C]: | 75,0 |
| PGU-2-F | 9,00 % | △n [589 nm, 20 °C]: | 0,1532 |
| PGU-3-F | 10,00 % | | |
| PGU-5-F | 9,00 % | | |
| CGZP-2-OT | 9,00 % | | |
| CGZP-3-OT | 4,00 % | | |
| BCH-3F.F.F | 12,00 % | | |
| PGIGI-3-F | 9,00 % | | |
| BCH-32 | 5,00 % | | |
| CCP-V-1 | 15,00 % | | |
| CBC-33 | 2,00 % | | |

### Beispiel M 16

| | |
|---|---|
| CCP-2F.F.F | 13,00 % |
| CCP-3F.F.F | 11,00 % |
| CCP-5F.F.F | 6,00 % |
| CCZU-2-F | 4,00 % |
| CCZU-3-F | 15,00 % |
| CCZU-5-F | 4,00 % |
| CCP-20CF₃.F | 6,00 % |
| CCP-30CF₃.F | 5,00 % |
| CGU-2-F | 3,00 % |
| CGU-3-F | 2,00 % |
| CCOC-3-3 | 2,00 % |
| CCOC-4-3 | 2,00 % |
| CC-5-V | 4,00 % |
| CCH-3CF₃ | 7,00 % |
| GG-1-OT | 4,00 % |
| IS-8847 | 12,00 % |

### Beispiel M17

| | |
|---|---|
| CCH-35 | 5,00 % |
| CC-3-V1 | 9,00 % |
| CC-5-V | 9,00 % |
| CCP-20CF₃ | 7,00 % |
| CCP-30CF₃ | 7,00 % |
| CCP-40CF₃ | 4,00 % |
| PGU-2-F | 8,00 % |
| CCZU-3-F | 12,00 % |
| CGZP-2-OT | 10,00 % |
| CGZP-3-OT | 7,00 % |
| GG-1-OT | 14,00 % |
| BCH-32 | 5,00 % |
| CBC-33 | 3,00 % |

### Beispiel M18

| | |
|---|---|
| CCP-2F.F.F | 12,00 % |
| CCP-3F.F.F | 12,00 % |
| CCP-5F.F.F | 6,00 % |
| CCZU-2-F | 4,00 % |
| CCZU-3-F | 16,00 % |
| CCZU-5-F | 4,00 % |
| CCP-20CF₃.F | 6,00 % |
| CCP-50CF₃F | 6,00 % |
| CGU-2-F | 3,00 % |
| CGU-3-F | 7,00 % |
| CCOC-3-3 | 3,00% |
| CCOC-4-3 | 3,00 % |
| IS-8847 | 12,00 % |
| GG-1-OT | 6,00 % |

### Beispiel M19

| | |
|---|---|
| CCP-2F.F.F | 13,00 % |
| CCP-3F.F.F | 11,00 % |
| CCP-5F.F.F | 6,00 % |
| CCP-20CF₃.F | 11,00 % |
| CCP-50CF₃.F | 12,00 % |
| CGU-3-F | 5,00 % |
| CCOC-3-3 | 3,00 % |
| CCOC-4-3 | 4,00 % |
| CCOC-3-5 | 2,00 % |
| CCH-3CF₃ | 6,00 % |
| CCH-5CF₃ | 6,00 % |
| GG-1-OT | 6,00 % |
| IS-8847 | 15,00 % |

### Beispiel M20

| | |
|---|---|
| CCP-2F.F.F | 13,00 % |
| CCP-3F.F.F | 11,00 % |
| CCP-5F.F.F | 6,00 % |
| CCP-20CF₃.F | 12,00 % |
| CCP-30CF₃.F | 12,00 % |
| CCP-50CF₃.F | 12,00 % |
| CGU-2-F | 5,00 % |
| CCOC-3-3 | 3,00 % |
| CCOC-4-3 | 3,00 % |
| CCOC-3-5 | 2,00 % |
| CCH-5CF₃ | 5,00 % |
| GG-1-OT | 8,00 % |
| IS-7718 | 8,00 % |

### Beispiel M21

| | |
|---|---|
| CCP-2F.F.F | 11,00 % |
| CCP-3F.F.F | 13,00 % |
| CCP-5F.F.F | 6,00 % |
| CCZU-2-F | 5,00 % |
| CCZU-3-F | 16,00 % |
| CCZU-5-F | 4,00 % |
| CCP-30CF₃.F | 3,00 % |
| CCP-50CF₃.F | 12,00 % |
| CGU-2-F | 4,00 % |
| CGU-3-F | 12,00 % |
| CCOC-4-3 | 3,00 % |
| IS-7718 | 8,00 % |
| GG-1-OT | 3,00 % |

### Beispiel M22

| | |
|---|---|
| CCP-2F.F.F | 13,00 % |
| CCP-3F.F.F | 11,00 % |
| CCP-5F.F.F | 6,00 % |
| CCZU-2-F | 5,00 % |
| CCZU-3-F | 16,00 % |
| CCZU-5-F | 4,00 % |
| CCP-20CF₃.F | 6,00 % |
| CCP-50CF₃.F | 7,00 % |
| CCOC-3-3 | 3,00 % |
| CCOC-4-3 | 4,00 % |
| CCOC-3-5 | 2,00 % |
| GG-1-OT | 7,00 % |
| IS-7718 | 8,00 % |
| CCH-3CF₃ | 4,00 % |
| CCH-5CF₃ | 4,00 % |

### Beispiel M23

| | |
|---|---|
| CCP-2F.F.F | 13,00 % |
| CCP-3F.F.F | 11,00 % |
| CCP-5F.F.F | 6,00 % |
| CCZU-2-F | 5,00 % |
| CCZU-3-F | 16,00 % |
| CCZU-5-F | 4,00 % |
| CCP-20CF₃.F | 5,00 % |
| CCP-50CF₃.F | 4,00 % |
| CCOC-3-3 | 2,00 % |
| CCOC-4-3 | 2,00 % |
| GG-1-OT | 3,00 % |
| IS-8847 | 15,00 % |
| CCH-3CF₃ | 8,00 % |
| CCH-5CF₃ | 6,00 % |

### Beispiel M24

| | | | |
|---|---|---|---|
| PGU-2-F | 6,00 % | S → N [°C]: | < -40 |
| CGZP-2-OT | 9,00 % | Klärpunkt [°C]: | 73,5 |
| BCH-3F.F.F | 6,00 % | Δn [589 nm, 20 °C]: | 0,0910 |
| CCP-2F.F.F | 9,00 % | γ₁ [mPa·s, 20 °C]: | 77 |
| CCG-V-F | 4,00 % | V₀[V]: | 1,44 |
| CCZU-2-F | 3,00 % | | |
| CCZU-3-F | 13,00 % | | |
| CCP-V-1 | 6,00 % | | |
| CC-3-V1 | 11,00 % | | |
| CC-5-V | 16,00 % | | |
| CCH-35 | 4,00 % | | |
| GG-1-OT | 10,00 % | | |
| CBC-33 | 3,00 % | | |

### Beispiel M25

| | | | |
|---|---|---|---|
| CC-3-V | 10,00 % | S → N [°C]: | < -30 |
| GG-1-OT | 12,00 % | Klärpunkt [°C]: | 75,0 |
| PGU-2-F | 11,00 % | Δn [589 nm, 20 °C]: | 0,1467 |
| PGU-3-F | 11,00 % | γ₁ [mPa·s, 20 °C]: | 116 |
| PGU-5-F | 9,00 % | | |
| CGZP-2-OT | 7,50 % | | |
| CGZP-3-OT | 6,50 % | | |
| BCH-3F.F.F | 5,50 % | | |
| PGIGI-3-F | 4,00 % | | |
| BCH-32 | 4,00 % | | |
| CCP-V-1 | 15,00 % | | |
| CBC-33 | 2,00 % | | |
| CBC-33F | 2,50 % | | |

### Beispiel M26

| | |
|---|---|
| CCP-2F.F.F | 13,00 % |
| CCP-3F.F.F | 11,00 % |
| CCP-5F.F.F | 6,00 % |
| CCP-20CF₃.F | 11,00 % |
| CCP-50CF₃.F | 12,00 % |
| CGU-2-F | 7,00 % |
| CGU-3-F | 8,00 % |
| CCOC-3-3 | 3,00 % |
| CCOC-4-3 | 4,00 % |
| CCOC-3-5 | 2,00 % |
| GG-1-OT | 8,00 % |
| IS-8847 | 15,00 % |

### Beispiel M27

| | |
|---|---|
| CCP-2F.F.F | 13,00 % |
| CCP-3F.F.F | 11,00 % |
| CCP-5F.F.F | 6,00 % |
| CCP-20CF₃.F | 12,00 % |
| CCP-30CF₃.F | 12,00 % |
| CCP-50CF₃.F | 12,00 % |
| CGU-2-F | 4,00 % |
| CCH-5CF₃ | 2,00 % |
| GG-1-OT | 11,00 % |
| IS-7718 | 8,00 % |
| CCGU-3-F | 9,00 % |

### Beispiel M28

| | |
|---|---|
| CCP-1 F.F.F | 5,00 % |
| CCP-2F.F.F | 8,00 % |
| CCP-3F.F.F | 3,00 % |
| CCP-20CF₃.F | 5,00 % |
| CCP-20CF₃ | 6,00 % |
| CCP-30CF₃ | 4,00 % |
| CCZU-2-F | 4,00 % |
| CCZU-3-F | 15,00 % |
| CCZU-5-F | 5,00 % |
| CGZP-2-OT | 11,00 % |
| CGZP-3-OT | 9,00 % |
| CCOC-3-3 | 4,00 % |
| GG-1-OT | 11,00 % |
| CCGU-3-F | 4,00 % |
| CC-3-V | 6,00 % |

### Beispiel M29

| | | | |
|---|---|---|---|
| CCP-2F.F.F | 10,00 % | S → N [°C]: | < -40 |
| CCP-20CF₃ | 6,00 % | Klärpunkt [°C]: | 75,5 |
| CCP-30CF₃ | 6,00 % | Δn [589 nm, 20 °C]: | 0,0884 |
| CCP-40CF₃ | 4,00 % | γ₁ [mPa·s, 20 °C]: | 134 |
| CCP-20CF₃.F | 12,00 % | V₁₀ [V]: | 1,09 |
| CCP-30CF₃.F | 4,00 % | | |
| CCZU-2-F | 5,00 % | | |
| CCZU-3-F | 15,00 % | | |
| CCZU-5-F | 4,00 % | | |
| CGZP-2-OT | 8,00 % | | |
| CGZP-3-OT | 7,00 % | | |
| CCGU-3-F | 3,00 % | | |
| CCH-3CF₃ | 2,00 % | | |
| GG-1-F | 10,00 % | | |
| CGU-2-F | 4,00 % | | |

### Beispiel M30

| | | | |
|---|---|---|---|
| CC-5-V | 8,00 % | S → N [°C]: | < -30 |
| CC-3-V1 | 11,00 % | Klärpunkt [°C]: | 76,0 |
| CCH-35 | 5,00 % | Δn [589 nm, 20 °C]: | 0,0765 |
| CC-3-V | 18,00 % | γ₁ [mPa·s, 20 °C]: | 60 |
| GG-1-F | 9,00 % | V₁₀ [V]: | 1,79 |
| CCZU-2-F | 4,00 % | | |
| CCZU-3-F | 15,00 % | | |
| CCP-20CF₃ | 3,00 % | | |
| CCP-30CF₃ | 8,00 % | | |
| CCP-50CF₃ | 6,00 % | | |
| CGZP-2-OT | 11,00 % | | |
| CBC-33 | 2,00 % | | |

### Beispiel M31

| | | | |
|---|---|---|---|
| CC-3-V | 4,00 % | Klärpunkt [°C]: | 70,0 |
| CCP-2F.F.F | 10,00 % | Δn [589 nm, 20 °C]: | 0,0878 |
| CCP-30CF₃ | 8,00 % | V₁₀ [V]: | 1,04 |
| CCP-40CF₃ | 6,00 % | | |
| CCZU-2-F | 5,00 % | | |
| CCZU-3-F | 15,00 % | | |
| CCZU-5-F | 5,00 % | | |
| CGZP-2-OT | 11,00 % | | |
| CGZP-3-OT | 9,00 % | | |
| CCOC-3-3 | 3,00 % | | |
| CCOC-4-3 | 3,00 % | | |
| CCGU-3-F | 3,00 % | | |
| GG-1-F | 18,00 % | | |

### Beispiel M32

| | |
|---|---|
| CC-3-V1 | 10,00 % |
| CC-5-V | 3,50 % |
| CCH-35 | 5,00 % |
| CC-3-V | 18,00 % |
| GG-1-F | 8,00 % |
| PGU-2-F | 11,00% |
| CGZP-2-OT | 11,00 % |
| CGZP-3-OT | 4,50 % |
| CCZU-2-F | 4,00 % |
| CCZU-3-F | 14,00 % |
| CCP-30CF₃ | 8,00 % |
| CBC-33 | 3,00 % |

### Beispiel M33

| | |
|---|---|
| PGU-2-F | 8,00 % |
| CCP-20CF₃ | 8,00 % |
| CCP-30CF₃ | 8,00 % |
| CCP-40CF₃ | 6,00 % |
| CCP-50CF₃ | 7,00 % |
| CCP-2F.F.F | 10,00 % |
| CCP-30CF₃.F | 11,00 % |
| CC-3-V1 | 10,00 % |
| CCH-35 | 5,00 % |
| CC-5-V | 10,00 % |
| GG-1-F | 12,00 % |
| CCGU-3-F | 4,50 % |
| CBC-33 | 0,50 % |

### Beispiel M34

| | |
|---|---|
| CCH-35 | 5,00 % |
| CC-3-V1 | 9,00 % |
| CC-5-V | 6,00 % |
| CCP-20CF₃ | 5,00 % |
| CCP-30CF₃ | 8,00 % |
| CCP-40CF₃ | 6,00 % |
| PGU-2-F | 8,00 % |
| CCZU-3-F | 14,00 % |
| CGZP-2-OT | 9,00 % |
| CGZP-3-OT | 8,00 % |
| GG-1-F | 14,00 % |
| CBC-33 | 3,00 % |
| BCH-32 | 5,00 % |

### Beispiel M35

| | | | |
|---|---|---|---|
| CC-5-V | 4,00 % | S → N [°C]: | < -30 |
| CC-3-V1 | 11,00 % | Klärpunkt [°C]: | 77,0 |
| CCH-35 | 5,00 % | Δn [589 nm, 20 °C]: | 0,0787 |
| CC-3-V | 17,00 % | γ₁ [mPa·s, 20 °C]: | 58 |
| PU-1-F | 10,00 % | V₁₀ [V]: | 1,78 |
| CCZU-2-F | 4,00 % | | |
| CCZU-3-F | 15,00 % | | |
| CCP-20CF₃ | 3,00 % | | |
| CCP-30CF₃ | 8,00 % | | |
| CCP-40CF₃ | 6,00 % | | |
| CGZP-2-OT | 7,00 % | | |
| CGZP-3-OT | 2,00 % | | |
| CCP-V-1 | 8,00 % | | |

### Beispiel M36

| | | | |
|---|---|---|---|
| CCP-2F.F.F | 10,00 % | Klärpunkt [°C]: | 72,5 |
| CCP-20CF₃ | 8,00 % | Δn [589 nm, 20 °C]: | 0,0873 |
| CCP-30CF₃ | 7,00 % | γ₁ [mPa·s, 20 °C]: | 118 |
| CCP-40CF₃ | 6,00 % | V₁₀ [V]: | 1,08 |
| CCP-20CF₃.F | 11,00 % | | |
| CCH-3CF₃ | 1,00% | | |
| CCZU-2-F | 5,00 % | | |
| CCZU-3-F | 15,00 % | | |
| CCZU-5-F | 5,00 % | | |
| CGZP-2-OT | 11,00 % | | |
| CGZP-3-OT | 9,00 % | | |
| PU-1-F | 12,00 % | | |

### Beispiel M37

| | | | |
|---|---|---|---|
| CCH-35 | 4,00 % | Klärpunkt [°C]: | 78,0 |
| CC-3-V1 | 9,00 % | Δn [589 nm, 20 °C]: | 0,1060 |
| CC-5-V | 3,50 % | γ₁ [mPa·s, 20 °C]: | 87 |
| CCP-20CF₃ | 7,00 % | V₁₀ [V]: | 1,35 |
| CCP-30CF₃ | 7,00 % | | |
| CCP-40CF₃ | 5,00 % | | |
| PGU-2-F | 8,00 % | | |
| PGU-3-F | 3,00 % | | |
| CCZU-2-F | 3,50 % | | |
| CCZU-3-F | 14,00 % | | |
| CGZP-2-OT | 9,00 % | | |
| CGZP-3-OT | 7,00 % | | |
| PU-1-F | 12,00 % | | |
| BCH-32 | 4,00 % | | |
| CCP-V-1 | 4,00 % | | |

### Beispiel M38

| | | | |
|---|---|---|---|
| CC-5-V | 4,00 % | Klärpunkt [°C]: | 79,0 |
| CC-3-V1 | 11,00 % | Δn [589 nm, 20 °C]: | 0,0777 |
| CCH-35 | 5,00 % | V₁₀ [V]: | 1,79 |
| CC-3-V | 18,00 % | | |
| GU-1-F | 10,00 % | | |
| CCZU-2-F | 4,00 % | | |
| CCZU-3-F | 15,00 % | | |
| CCP-30CF₃ | 8,00 % | | |
| CCP-40CF₃ | 8,00 % | | |
| CGZP-2-OT | 7,50 % | | |
| CBC-33 | 1,50 % | | |
| CCP-V-1 | 8,00 % | | |

### Beispiel M39

| | | | |
|---|---|---|---|
| CCP-2F.F.F | 10,00 % | Klärpunkt [°C]: | 75,5 |
| CCP-20CF₃ | 8,00 % | Δn [589 nm, 20 °C]: | 0,0888 |
| CCP-30CF₃ | 8,00 % | γ₁ [mPa·s, 20 °C]: | 122 |
| CCP-40CF₃ | 6,00 % | V₁₀ [V]: | 1,03 |
| CCP-20CF₃.F | 6,00 % | | |
| CCGU-3-F | 1,00 % | | |
| CCZU-2-F | 4,00 % | | |
| CCZU-3-F | 15,00 % | | |
| CCZU-5-F | 5,00 % | | |
| CGZP-2-OT | 11,00 % | | |
| CGZP-3-OT | 9,00 % | | |
| GU-1-F | 14,00 % | | |
| CCOC-3-3 | 1,00 % | | |
| CCP-V-1 | 2,00 % | | |

### Beispiel M40

| | | | |
|---|---|---|---|
| CC-5-V | 4,00 % | S → N [°C]: | < -20,0 |
| Cc-3-V1 | 11,00 % | Klärpunkt [°C]: | +77,0 |
| CCH-35 | 4,00 % | Δn [589 nm, 20 °C]: | +0,0782 |
| CC-3-V | 19,00 % | γ₁ [mPa·s, 20 °C]: | 59 |
| GG-1-F | 7,00 % | V_{10,0,20} M: | 1,79 |
| GU-1-F | 3,00 % | | |
| CCZU-2-F | 4,00 % | | |
| CCZU-3-F | 15,00 % | | |
| CCP-30CF₃ | 8,00 % | | |
| CCP-40CF₃ | 4,00 % | | |
| CGZP-2-OT | 6,00 % | | |
| CGZP-3-OT | 4,00 % | | |
| CCP-V-1 | 11,00% | | |

### Beispiel M41

| | | | |
|---|---|---|---|
| CC-5-V | 5,00 % | S → N [°C]: | < -20,0 |
| CC-3-V1 | 10,00 % | Klärpunkt [°C]: | +77,0 |
| CCH-35 | 5,00 % | Δn [589 nm, 20 °C]: | +0,0779 |
| CC-3-V | 17,00 % | γ₁ [mPa·s, 20 °C]: | 60 |
| GG-1-F | 8,00 % | V_{10,0,20} [V]: | 1,76 |
| GU-1-F | 2,00 % | | |
| CCZU-2-F | 4,00 % | | |
| CCZU-3-F | 15,00 % | | |
| CCP-20CF₃ | 4,00 % | | |
| CCP-30CF₃ | 8,00 % | | |
| CCP-40CF₃ | 5,00% | | |
| CGZP-2-OT | 6,50 % | | |
| CGZP-3-OT | 2,50 % | | |
| CCP-V-1 | 8,00 % | | |

### Beispiel M42

| | | | |
|---|---|---|---|
| CC-3-V | 10,00 % | S → N [°C]: | < -30,0 |
| GG-1-F | 10,00 % | Klärpunkt [°C]: | +74,0 |
| PGU-2-F | 12,00 % | Δn [589 nm, 20 °C]: | +0,1511 |
| GPU-3-F | 12,00 % | Δε (1 kHz, 20 °C]: | +12,4 |
| PGU-5-F | 10,00 % | γ1 [mPa·s, 20 °C]: | 119 |
| CGZP-2-OT | 8,00 % | | |
| CGZP-3-OT | 8,00 % | | |
| BCH-3F.F.F | 4,00 % | | |
| PGIGI-3-F | 5,00 % | | |
| BCH-32 | 6,00 % | | |
| CP-V-1 | 11,00 % | | |
| CBC-33 | 2,00 % | | |
| CBC-53 | 2,00 % | | |

### Beispiel M43

| | | | |
|---|---|---|---|
| CC-5-V | 2,00 % | S → N [°C]: | < -20,0 |
| CC-3-V1 | 12,00 % | Klärpunkt [°C]: | +76,0 |
| CCH-35 | 5,00 % | Δn [589 nm, 20 °C]: | +0,0796 |
| CC-3-V | 19,00 % | V_{10,0,20} [V]: | 1,79 |
| GG-1-F | 8,00 % | | |
| GU-1-F | 3,00 % | | |
| CCZU-2-F | 2,00 % | | |
| CCZU-3-F | 15,00 % | | |
| CCP-30CF₃ | 8,00 % | | |
| CCP-40CF₃ | 4,00 % | | |
| CGZP-2-OT | 6,00 % | | |
| CGZP-3-OT | 4,00 % | | |
| CCP-V-1 | 12,00 % | | |

### Beispiel M44

| | | | |
|---|---|---|---|
| CC-3-V | 10,00 % | Klärpunkt [°C]: | +74,0 |
| GU-1-F | 11,00 % | Δn [589 nm, 20 °C]: | +0,1501 |
| PGU-2-F | 12,00 % | | |
| PGU-3-F | 12,00 % | | |
| PGU-5-F | 10,00 % | | |
| CGZP-2-OT | 8,00 % | | |
| CGZP-3-OT | 7,00 % | | |
| PGIGI-3-F | 6,00 % | | |
| BCH-32 | 6,00 % | | |
| CCP-V-1 | 13,00 % | | |
| CBC-33 | 3,00 % | | |
| CBC-53 | 2,00 % | | |

### Beispiel M45

| | | | |
|---|---|---|---|
| CC-3-V | 11,00 % | Klärpunkt [°C]: | +75,0 |
| GU-1-F | 8,00 % | Δn [589 nm, 20 °C]: | +0,1508 |
| PGU-2-F | 12,00 % | | |
| PGU-3-F | 12,00 % | | |
| PGU-5-F | 10,00 % | | |
| CGZP-2-OT | 8,00 % | | |
| CGZP-3-OT | 8,00 % | | |
| BCH-3F.F.F | 4,00 % | | |
| PGIGI-3-F | 6,00 % | | |
| BCH-32 | 6,00 % | | |
| CCP-V-1 | 12,00 % | | |
| CBC-33 | 3,00 % | | |

### Beispiel M46

| | | | |
|---|---|---|---|
| CC-3-V | 12,00 % | Klärpunkt [°C]: | +74,0 |
| GU-1-F | 6,00% | Δn [589 nm, 20°C]: | +0,1494 |
| PGU-2-F | 12,00 % | | |
| PGU-3-F | 12,00 % | | |
| PGU-5-F | 10,00 % | | |
| CGZP-2-OT | 8,00 % | | |
| CGZP-3-OT | 8,00 % | | |
| BCH-3F.F.F | 7,00 % | | |
| PGIGI-3-F | 6,00 % | | |
| BCH-32 | 5,00 % | | |
| CCP-V-1 | 12,00 % | | |
| CBC-33 | 2,00 % | | |

### Beispiel M47

| | | | |
|---|---|---|---|
| CC-5-V | 4,00 % | S → N [°C]: | < -40,0 |
| CC-3-V1 | 11,00 % | Klärpunkt [°C]: | +77,0 |
| CCH-3-V | 5,00 % | Δn [589 nm, 20 °C]: | +0,0788 |
| PU-1-F | 17,00 % | γ₁ [mPa·s, 20 °C]: | 59 |
| GU-1-F | 7,00 % | V₁₀ [V]: | 1,67 |
| CCZU-2-F | 3,00 % | | |
| CCZU-2-F | 4,00 % | | |
| CCZU-3-F | 15,00 % | | |
| CCP-20CF₃ | 3,00 % | | |
| CCP-30CF₃ | 8,00 % | | |
| CCP-40CF₃ | 6,00 % | | |
| CGZP-2-OT | 6,50 % | | |
| CGZP-3-OT | 2,50 % | | |
| CCP-V-1 | 8,00 % | | |

### Beispiel M48

| | | | |
|---|---|---|---|
| CCP-1 F.F.F | 6,00 % | S → N [°C]: | < -40,0 |
| CCP-2F.F.F | 9,00 % | Klärpunkt [°C]: | +79,5 |
| CCP-20CF₃ | 7,00 % | Δn [589 nm, 20 °C]: | +0,0878 |
| CCP-30CF₃ | 7,00 % | γ₁ [mPa·s, 20 °C]: | 148 |
| CCP-40CF₃ | 4,00 % | V₁₀ [V]: | 1,00 |
| CCP-20CF₃.F | 11,00 % | | |
| CCGU-3-F | 5,00 % | | |
| CCZU-2-F | 4,00 % | | |
| CCZU-3-F | 15,00 % | | |
| CCZU-5-F | 4,00 % | | |
| CGZP-2-OT | 10,00 % | | |
| CGZP-3-OT | 8,00 % | | |
| GU-1-F | 4,00 % | | |
| GU-2-F | 2,00 % | | |
| UU-1-F | 4,00 % | | |

### Beispiel M49

| | |
|---|---|
| CCP-20CF₃ | 8,00 % |
| CCP-30CF₃ | 8,00 % |
| CCZU-2-F | 4,00 % |
| CCZU-3-F | 15,00 % |
| CCZU-5-F | 4,00 % |
| CCP-V-1 | 14,00 % |
| CDU-2-F | 9,00 % |
| PGU-2-F | 1 ,00 % |
| GU-1-F | 7,00 % |
| PUQU-2-F | 4,00 % |
| PUQU-3-F | 4,50 % |
| CC-3-V1 | 11,00 % |
| CC-5-V | 5,50 % |
| CCH-35 | 5,00 % |

### Beispiel M50

| | |
|---|---|
| CCP-20CF₃ | 8,00 % |
| CCP-30CF₃ | 8,00 % |
| CCZU-2-F | 4,00 % |
| CCZU-3-F | 15,00 % |
| CCZU-5-F | 4,00 % |
| CDU-2-F | 9,00 % |
| CDU-3-F | 2,50 % |
| GU-1-F | 7,00 % |
| PUQU-2-F | 4,00 % |
| PUQU-3-F | 4,00 % |
| CCP-V-1 | 14,00 % |
| CC-3-V1 | 10,50 % |
| CC-3-V | 10,00 % |

### Beispiel M51

| | | | |
|---|---|---|---|
| CCP-20CF₃ | 8,00 % | Klärpunkt [°C]: | +79,0 |
| CCP-30CF₃ | 8,00 % | Δn [589 nm, 20 °C]: | +0,0851 |
| CCZU-2-F | 4,00 % | Δε [kHz, 20°C]: | 10,3 |
| CCZU-3-F | 15,00 % | γ₁ [mPa·s, 20 °C]: | 85 |
| CCZU-5-F | 4,00 % | | |
| CDU-2-F | 9,00 % | | |
| CDU-3-F | 3,50 % | | |
| PGU-2-F | 1,00 % | | |
| GU-1-F | 6,00 % | | |
| PUQU-2-F | 4,00 % | | |
| PUQU-3-F | 5,00 % | | |
| CCP-V-1 | 14,00 % | | |
| CC-3-V1 | 11,00 % | | |
| CC-3-V | 7,50 % | | |

### Beispiel M52

| | |
|---|---|
| CCP-2F.F.F | 9,50 % |
| CCP-20CF₃ | 7,00 % |
| CGZP-2-OT | 10,00 % |
| CGZP-3-OT | 6,00 % |
| CCZU-2-F | 4,00 % |
| CCZU-3-F | 10,50 % |
| PUQU-2-F | 4,00 % |
| PUQU-3-F | 5,00 % |
| CDU-2-F | 7,00 % |
| PGU-2-F | 5,00 % |
| GU-1-F | 5,00 % |
| CCP-V-1 | 9,50 % |
| CC-3-V1 | 10,50 % |
| CC-5-V | 2,00 % |
| CCH-35 | 5,00 % |

### Beispiel M53

| | |
|---|---|
| CCP-2F.F.F | 9,50 % |
| CCP-30CF₃ | 8,00 % |
| CGZP-2-OT | 9,00 % |
| CGZP-3-OT | 7,00 % |
| CCZU-2-F | 4,00 % |
| CCZU-3-F | 10,50 % |
| PUQU-2-F | 4,00 % |
| PUQU-3-F | 5,00 % |
| CDU-2-F | 6,00 % |
| PGU-2-F | 6,00 % |
| GU-1-F | 5,00 % |
| CCP-V-1 | 8,00 % |
| CC-3-V1 | 9,00 % |
| CC-3-V | 9,00 % |

### Beispiel M54

| | |
|---|---|
| CCP-20CF₃ | 5,00 % |
| CCP-30CF₃ | 8,00 % |
| CDU-2-F | 7,00 % |
| PGU-2-F | 6,50 % |
| CGZP-2-OT | 10,00 % |
| CGZP-3-OT | 5,00 % |
| CCZU-2-F | 4,00 % |
| CCZU-3-F | 13,00 % |
| PUQU-2-F | 4,00 % |
| PUZU-3-F | 5,00 % |
| CCP-V-1 | 4,50% |
| CC-3-V1 | 10,00 % |
| CC-5-V | 8,00 % |
| CCH-35 | 5,00 % |
| GU-1-F | 5,00 % |

### Beispiel M55

| | |
|---|---|
| CCP-30CF₃ | 7,50 % |
| CGZP-2-OT | 10,00 % |
| CGZP-3-OT | 4,00 % |
| CCZU-2-F | 4,00 % |
| CCZU-3-F | 12,50 % |
| PUQU-2-F | 4,00 % |
| PUQU-3-F | 4,00 % |
| PZU-V2-F | 5,00 % |
| GU-1-F | 5,00 % |
| CDU-2-F | 8,50 % |
| CDU-3-F | 3,50 % |
| PUGU-2-F | 2,50 % |
| CCP-V-1 | 15,50 % |
| CC-3-V1 | 11,00 % |
| CCH-35 | 3,00 % |

## Patentansprüche

1. Flüssigkristallines Medium auf der Basis eines Gemisches von polaren Verbindungen mit positiver oder negativer dielektrischer Anisotropie, **dadurch gekennzeichnet, dass** es eine oder mehrere Verbindungen der allgemeinen Formel I, 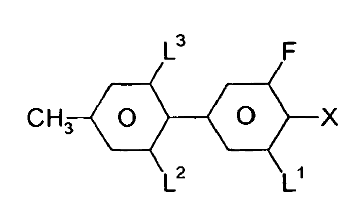 worin
X F, Cl, CN, SF₅, NCS, einen halogenierten Alkylrest mit bis zu 8 C-Atomen, wobei eine oder mehrere CH₂-Gruppen durch -O- oder -CH=CH- so ersetzt sein können, dass O-Atome nicht direkt miteinander verknüpft sind,und
L¹, L², L³ jeweils unabhängig voneinander H oder F
bedeutet
und
eine oder mehrere Verbindungen ausgewählt aus der Gruppe bestehend aus den allgemeinen Formeln II, III, IV, V, VI, VII, VIII, IX und X enthält: 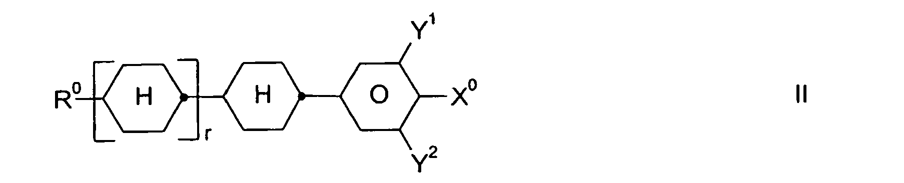 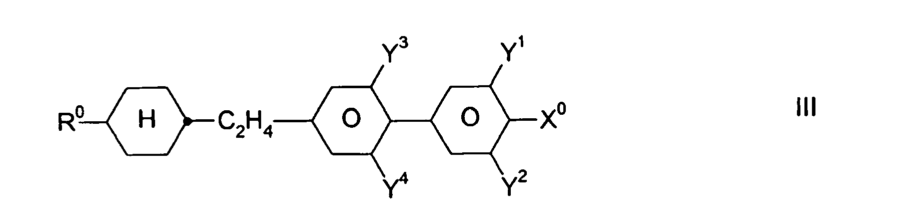 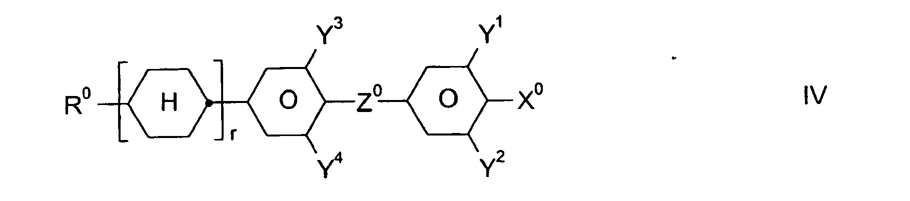 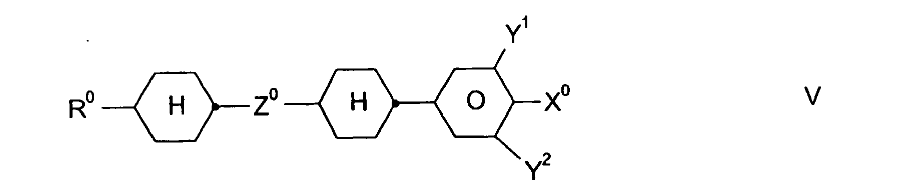 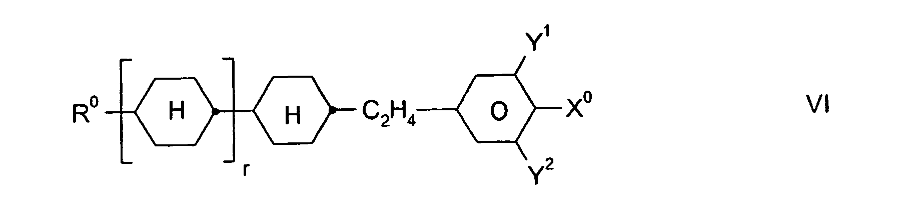 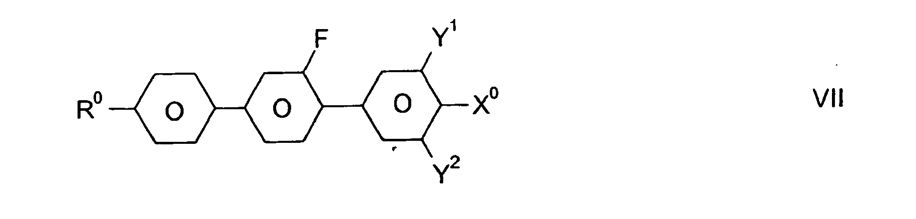 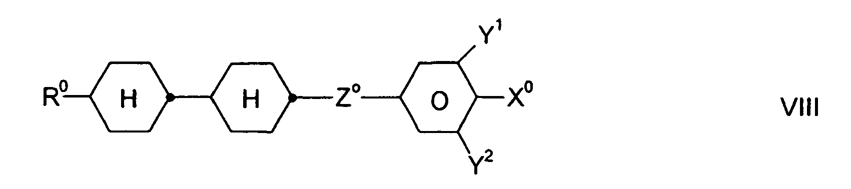 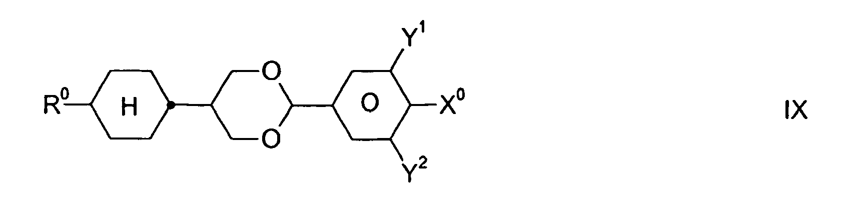 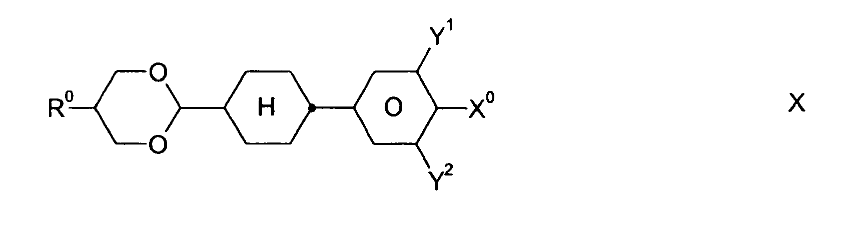 worin die einzelnen Reste die folgenden Bedeutungen haben:
R⁰ n-Alkyl, Oxaalkyl, Fluoralkyl, Alkenyloxy oder Alkenyl mit jeweils 2 bis 12 C-Atomen
X⁰ F, Cl, halogeniertes Alkyl, halogeniertes Alkenyl, halogeniertes Alkenyloxy oder halogeniertes Alkoxy mit jeweils bis zu 8 C-Atomen,
Z⁰ -CH=CH-, -CH₂O, -OCH₂-, -(CH₂)₄-, -C₂H₄-, -C₂F₄-, -CF=CF-, -CF₂O-, -OCF₂- oder -COO-,
Y¹,Y², Y³ und Y⁴ jeweils unabhängig voneinander H oder F, und
r 0 oder 1.

2. Flüssigkristallines Medium nach Anspruch 1, **dadurch gekennzeichnet, dass** X in Formel I
F, Cl, CN, NCS, CF₃, SF₅, CF₂H, CF₂CF₃, CF₂CF₂CF₃, CH₂CH₂CF₃, CF₂CH₂CF₃, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃, OCF₂CHFCF₃, OCCIFCF₂CF₃
bedeutet.

3. Flüssigkristallines Medium nach Anspruch 1 oder 2, **dadurch gekennzeichnet, dass** es mindestens eine Verbindung aus der Gruppe der Verbindungen der Formeln 11 bis 15, 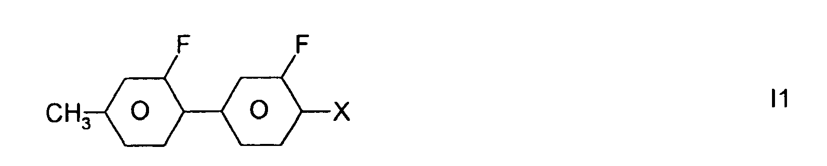 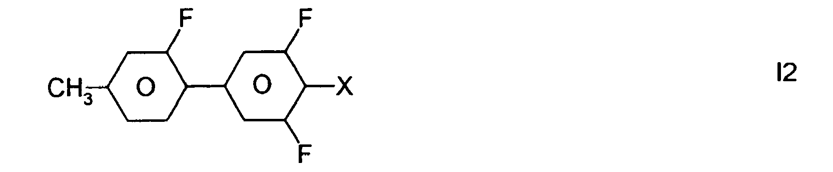 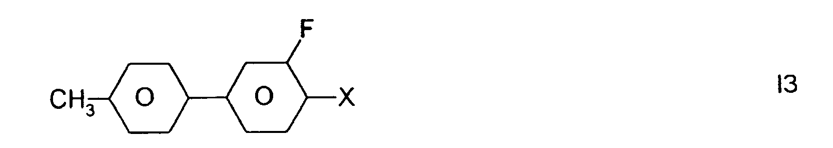 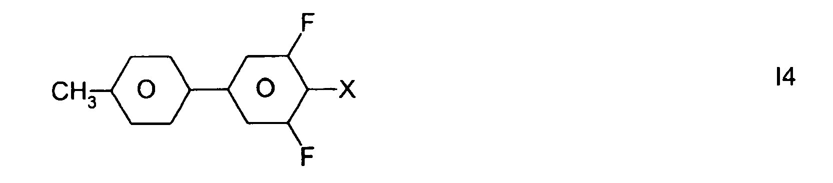 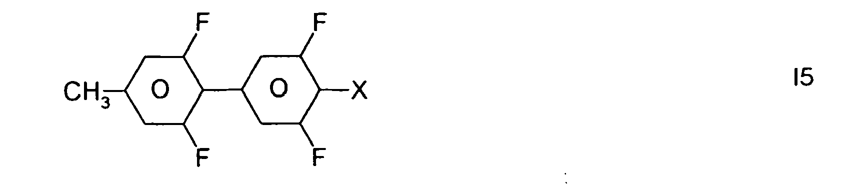 enthält.

4. Medium nach Anspruch 3, **dadurch gekennzeichnet, dass** der Anteil an Verbindungen der Formeln I bis X im Gesamtgemisch mindestens 50 Gew.-% beträgt.

5. Medium nach einem der Ansprüche 1 bis 4, **dadurch gekennzeichnet, dass** es zusätzlich eine oder mehrere Verbindungen der Formeln RI bis RXV, 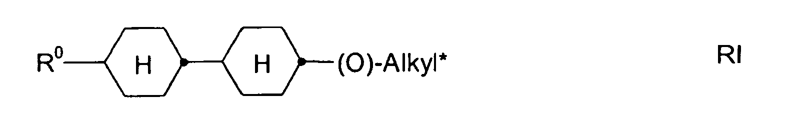 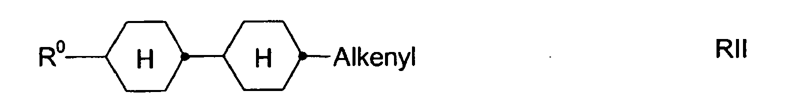 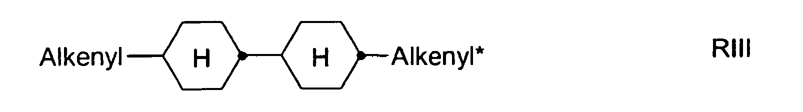 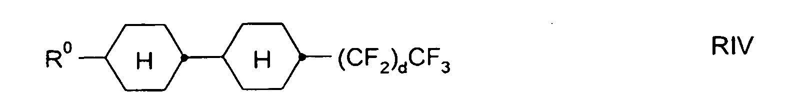 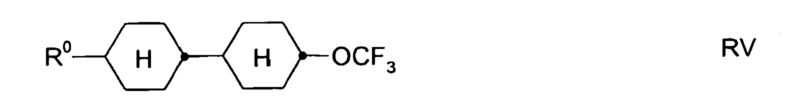 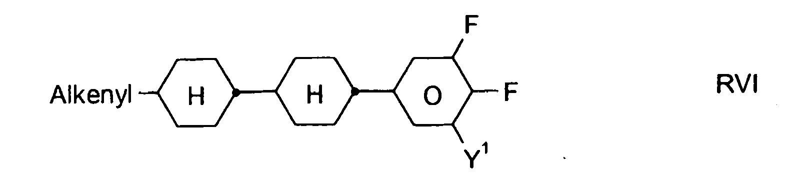 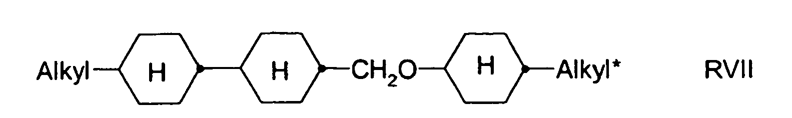 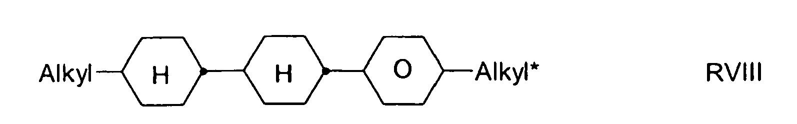 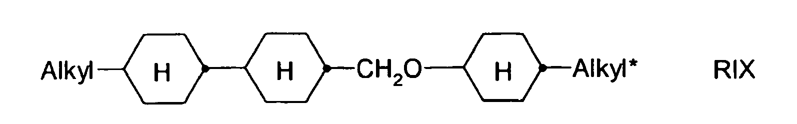 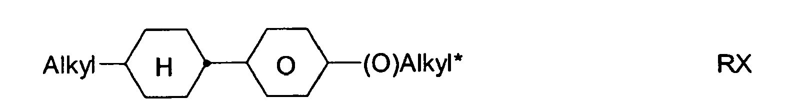 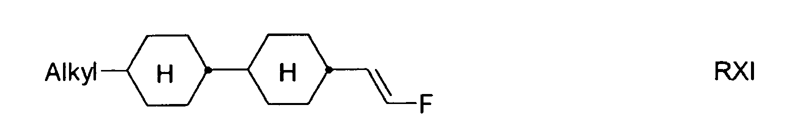 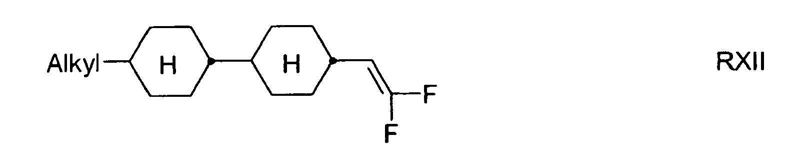 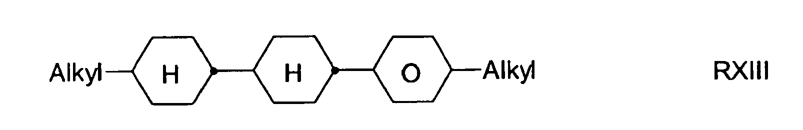 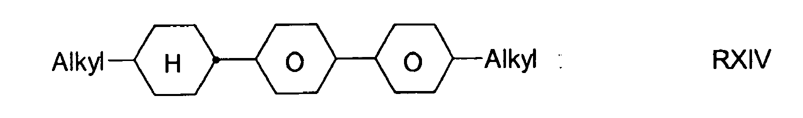 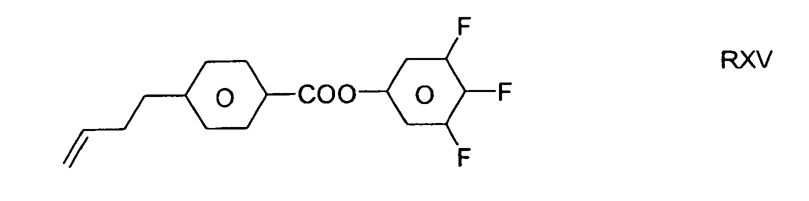 worin
R⁰ n-Alkyl, Oxaalkyl, Fluoralkyl, Alkenyloxy oder Alkenyl mit jeweils 2 bis 12 C-Atomen,
d 0, 1 oder 2,
Y¹ H oder F,
Alkyl oder Alkyl* jeweils unabhängig voneinander ein geradkettiger oder verzweigter Alkylrest mit 2 bis 8 C-Atomen,
Alkenyl oder Alkenyl* jeweils unabhängig voneinander einen geradkettigen oder verzweigten Alkenylrest mit 2 bis 8 C-Atomen
bedeuten,
enthält.

6. Medium nach Anspruch 1, **dadurch gekennzeichnet, dass** X⁰ F, OCHF₂ oder OCF₃ und Y² H oder F bedeuten.

7. Verwendung des flüssigkristallinen Mediums nach Anspruch 1 für elektrooptische Zwecke.

8. Elektrooptische Flüssigkristallanzeige enthaltend ein flüssigkristallines Medium nach Anspruch 1.

## Claims

1. Liquid-crystalline medium based on a mixture of polar compounds of positive or negative dielectric anisotropy, **characterised in that** it comprises one or more compounds of the general formula I 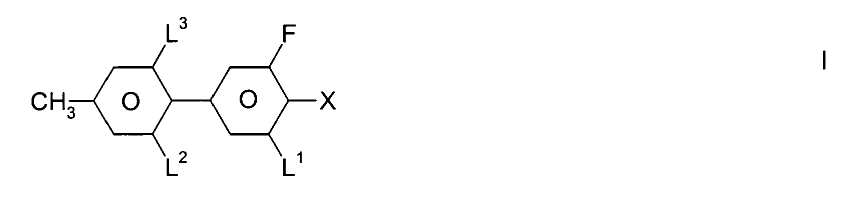 in which
X denotes F, Cl, CN, SF₅, NCS, a halogenated alkyl radical having up to 8 C atoms, in which one or more CH₂ groups may be replaced by -O- or -CH=CH- in such a way that O atoms are not linked directly to one another, and
L¹, L², L³ each, independently of one another, denote H or F,
and
one or more compounds selected from the group consisting of the general formulae II, III, IV, V, VI, VII, VIII, IX and X: 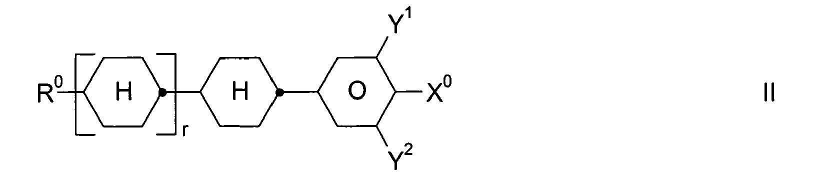 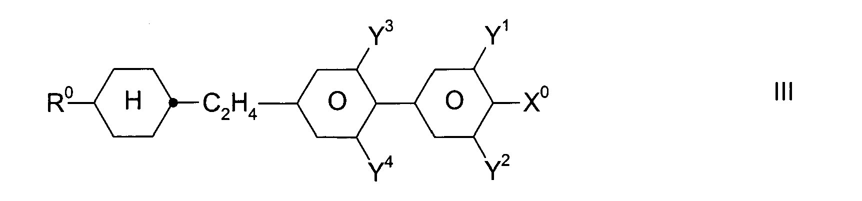 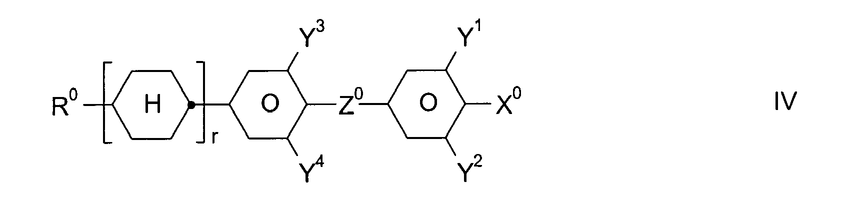 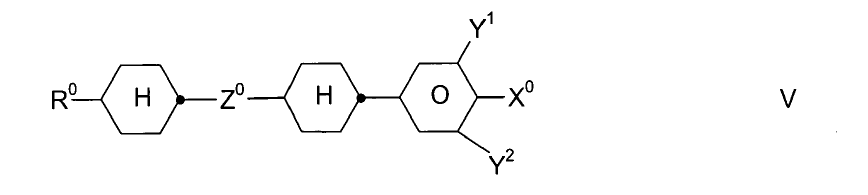 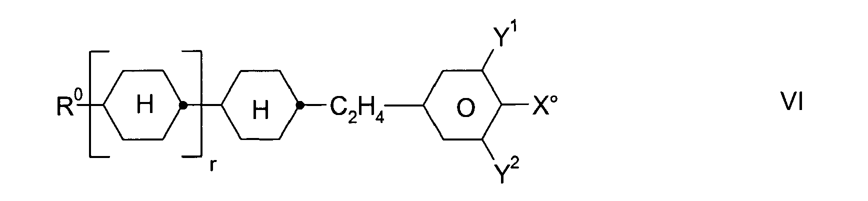 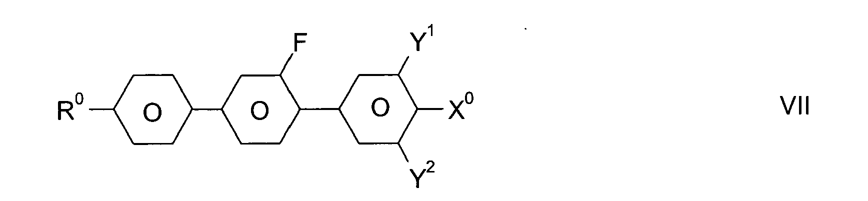 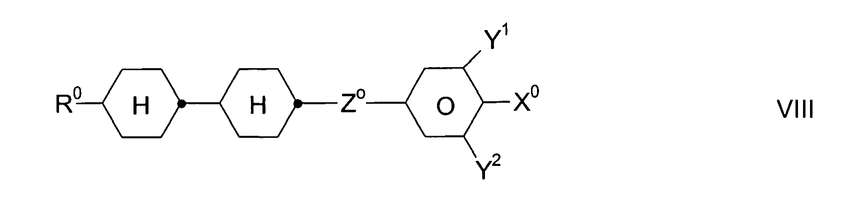 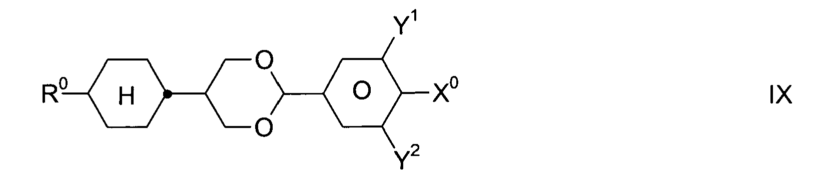 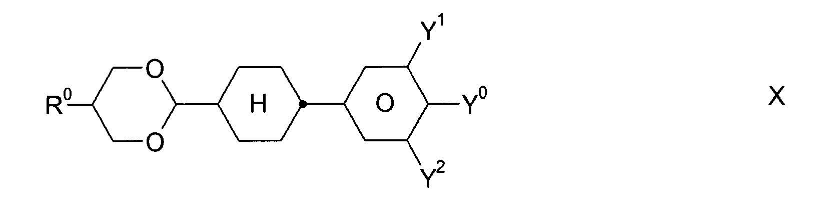 in which the individual radicals have the following meanings:
R⁰ n-alkyl, oxaalkyl, fluoroalkyl, alkenyloxy or alkenyl, each having 2 to 12 C atoms,
X⁰ F, Cl, halogenated alkyl, halogenated alkenyl, halogenated alkenyloxy or halogenated alkoxy, each having up to 8 C atoms,
Z⁰ -CH=CH-, -CH₂O-, -OCH₂-, -(CH₂)₄-, -C₂H₄-, -C₂F₄-, -CF=CF-, -CF₂O-, -OCF₂- or -COO-,
Y¹,Y², Y³ and Y⁴ each, independently of one another, H or F, and
r 0 or 1.

2. Liquid-crystalline medium according to Claim 1, **characterised in that** X in formula I denotes
F, Cl, CN, NCS, CF₃, SF₅, CF₂H, CF₂CF₃, CF₂CF₂CF₃, CH₂CH₂CF₃, CF₂CH₂CF₃, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃, OCF₂CHFCF₃, OCCIFCF₂CF₃.

3. Liquid-crystalline medium according to Claim 1 or 2, **characterised in that** it comprises at least one compound from the group of the compounds of the formulae I1 to I5

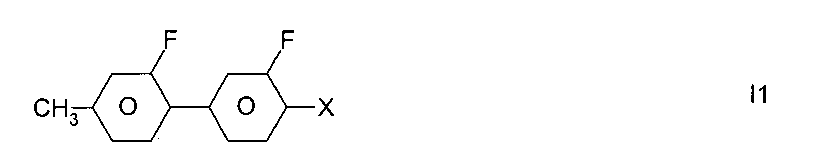

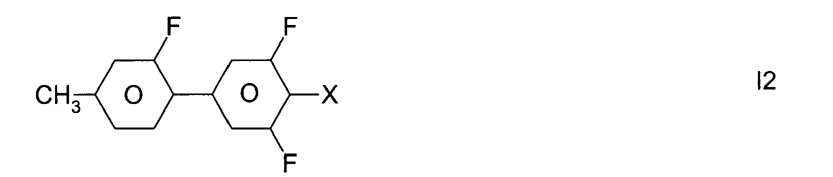

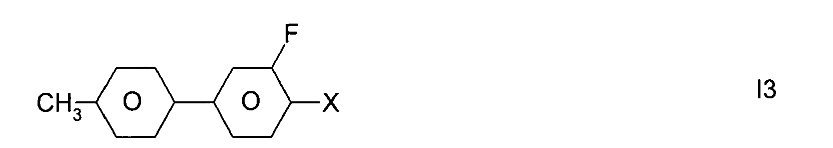

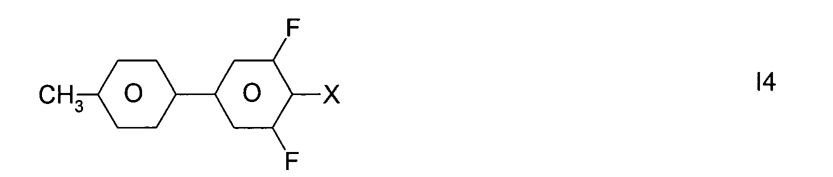

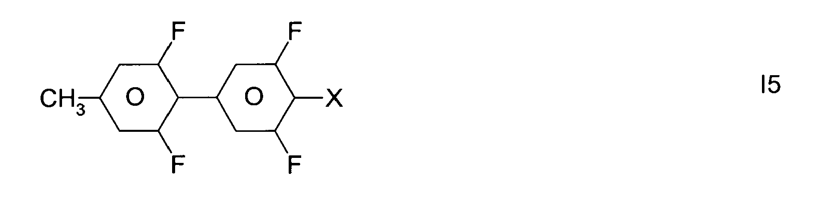

4. Medium according to Claim 3, **characterised in that** the proportion of compounds of the formulae I to X in the mixture as a whole is at least 50% by weight.

5. Medium according to one of Claims 1 to 4, **characterised in that** it additionally comprises one or more compounds of the formulae RI to RXV 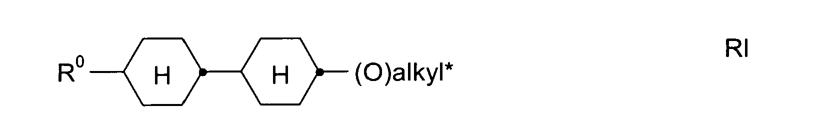 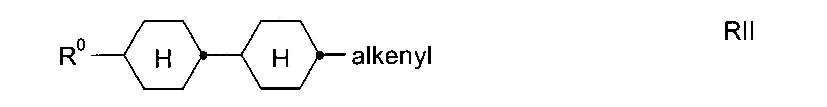 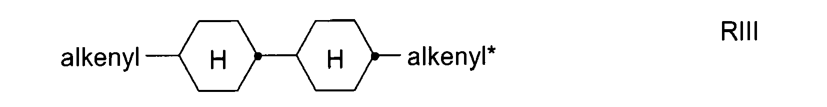 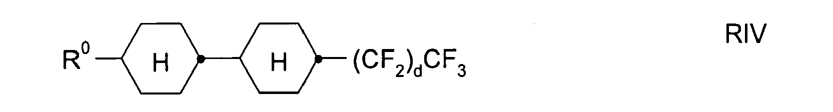 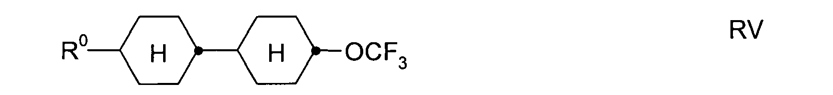 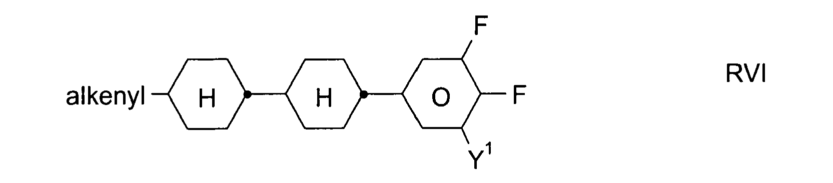 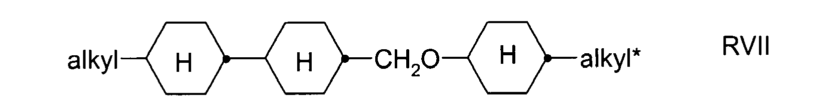 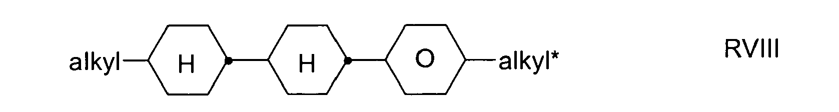 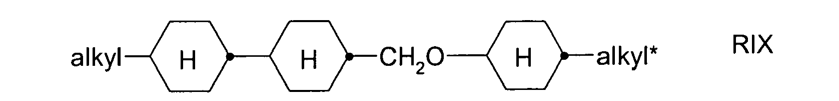 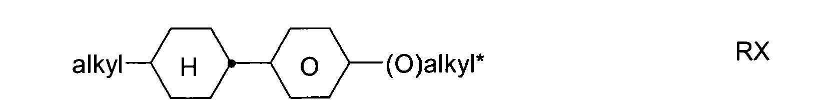 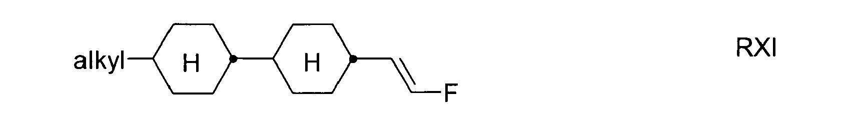 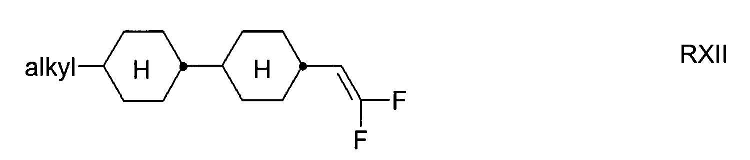 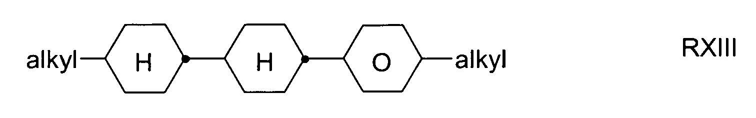 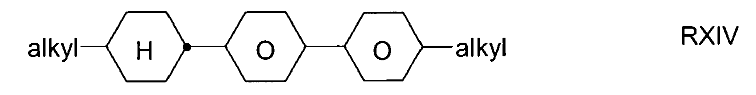 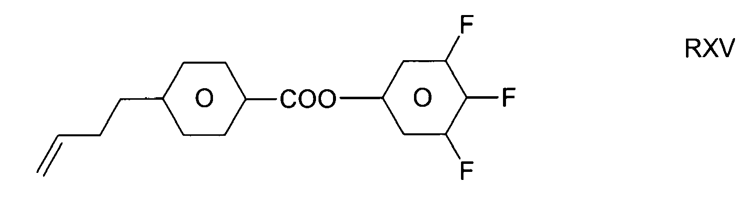 in which
R⁰ denotes n-alkyl, oxaalkyl, fluoroalkyl, alkenyloxy or alkenyl, each having 2 to 12 C atoms,
d denotes 0, 1 or 2,
Y¹ denotes H or F,
alkyl or alkyl* each, independently of one another, denote a straight-chain or branched alkyl radical having 2 to 8 C atoms,
alkenyl or alkenyl* each, independently of one another, denote a straight-chain or branched alkenyl radical having 2 to 8 C atoms.

6. Medium according to Claim 1, **characterised in that** X⁰ denotes F, OCHF₂ or OCF₃ and Y² denotes H or F.

7. Use of the liquid-crystalline medium according to Claim 1 for electro-optical purposes.

8. Electro-optical liquid-crystal display containing a liquid-crystalline medium according to Claim 1.

## Revendications

1. Milieu de cristal liquide basé sur un mélange de composés polaires d'une anisotropie diélectrique positive ou négative, **caractérisé en ce qu'**il comprend un ou plusieurs composés de la formule générale I 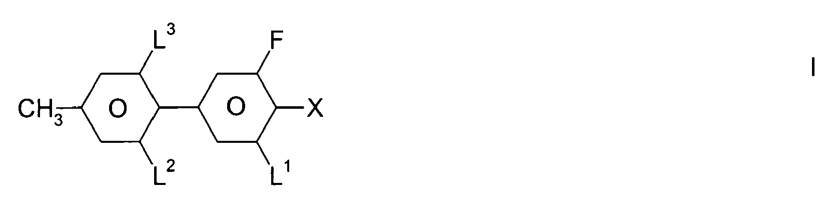 dans laquelle
X représente F, Cl, CN, SF₅, NCS, un radical alkyle halogéné comportant jusqu'à 8 atomes de C où un ou plusieurs groupes CH₂ peuvent être remplacés par -O- ou -CH=CH- de telle sorte que des atomes de O ne soient pas liés directement les uns aux autres ; et
L¹, L², L³ chacun indépendamment des autres, représentent H ou F,
et
un ou plusieurs composés choisis parmi le groupe comprenant les formules générales II, III, IV, V, VI, VII, VIII, IX et X : 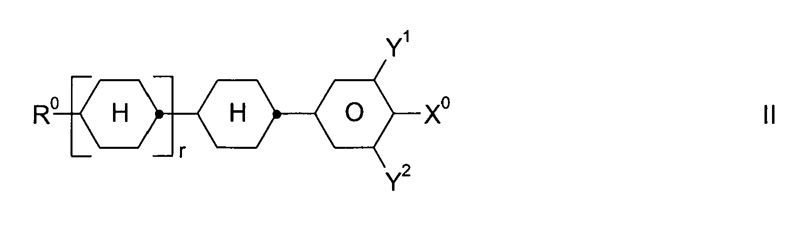 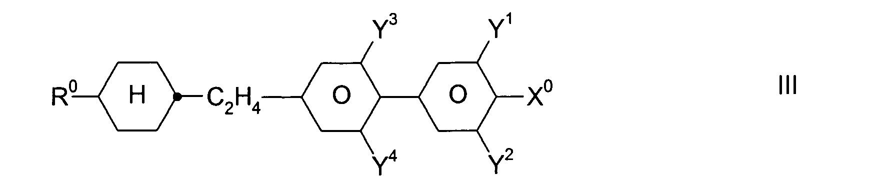 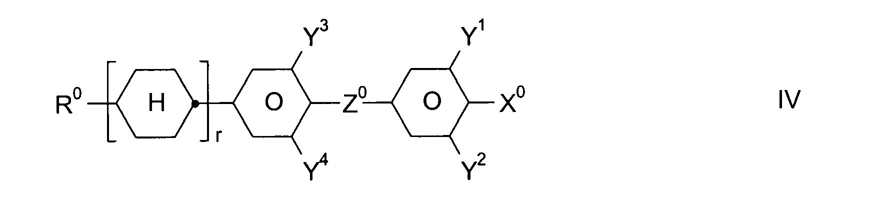 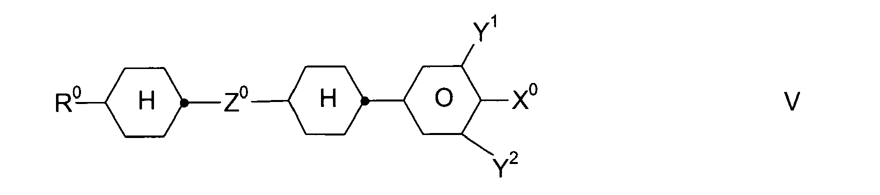 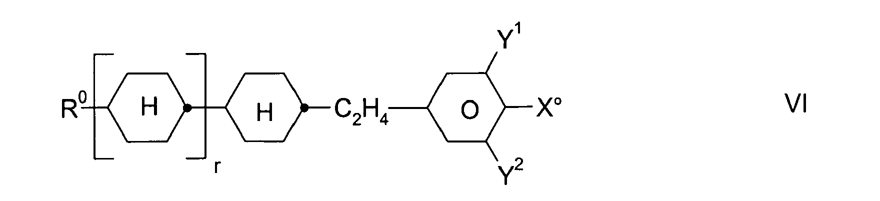 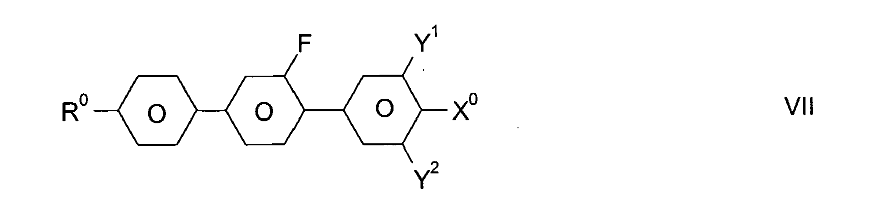 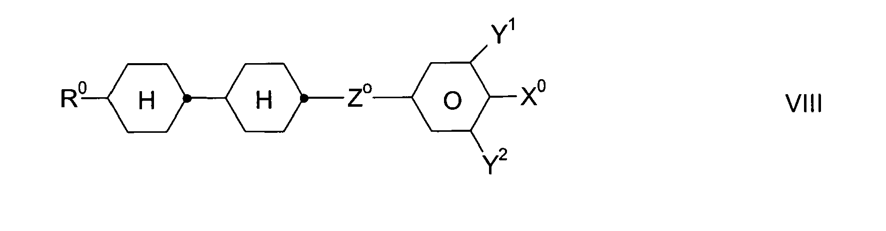 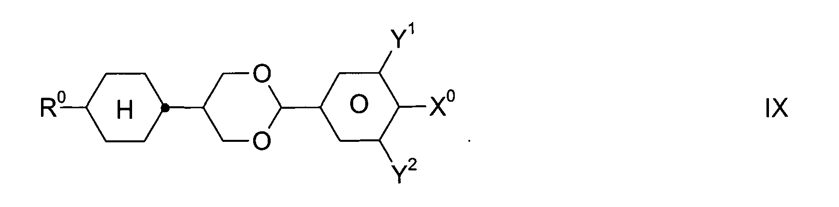 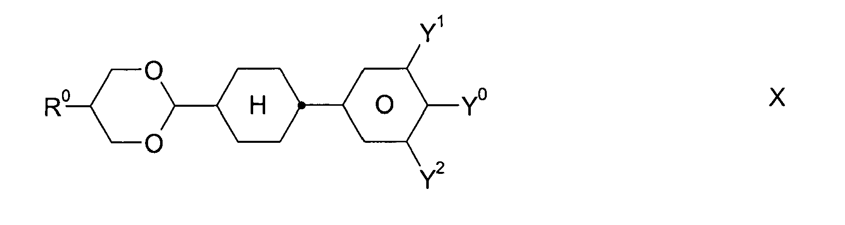 dans lesquelles les radicaux individuels présentent les significations qui suivent :
R⁰ n-alkyle, oxaalkyle, fluoroalkyle, alkényloxy ou alkényle, chacun comportant de 2 à 12 atomes de C,
X⁰ F, Cl, alkyle halogéné, alkényle halogéné, alkényloxy halogéné ou alkoxy halogéné, chacun comportant jusqu'à 8 atomes de C,
Z⁰ -CH=CH-, -CH₂O-, -OCH₂-, -(CH₂)₄-, -C₂H₄-, -C₂F₄-, -CF=CF-, -CF₂O-, -OCF₂- ou -COO-,
Y¹, Y², Y³ et Y⁴ chacun indépendamment des autres, H ou F, et
r 0 ou 1.

2. Milieu de cristal liquide selon la revendication 1, **caractérisé en ce que** X dans la formule I représente
F, CI, CN, NCS, CF₃, SF₅, CF₂H, CF₂CF₃, CF₂CF₂CF₃, CH₂CH₂CF₃, CF₂CH₂CF₃, OCF₃, OCF₂H, OCFHCF₃, OCFHCFH₂, OCFHCF₂H, OCF₂CH₃, OCF₂CFH₂, OCF₂CF₂H, OCF₂CF₂CF₂H, OCF₂CF₂CFH₂, OCFHCF₂CF₃, OCFHCF₂CF₂H, OCF₂CF₂CF₃, OCF₂CHFCF₃, OCClFCF₂CF₃.

3. Milieu de cristal liquide selon la revendication 1 ou 2, **caractérisé en ce qu'**il comprend au moins un composé pris parmi le groupe des composés des formules I1 à I5

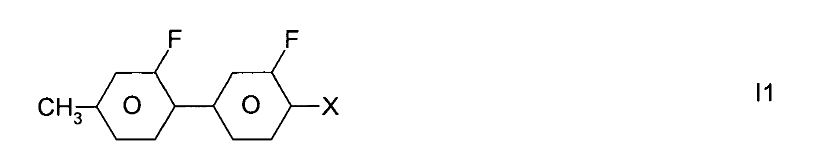

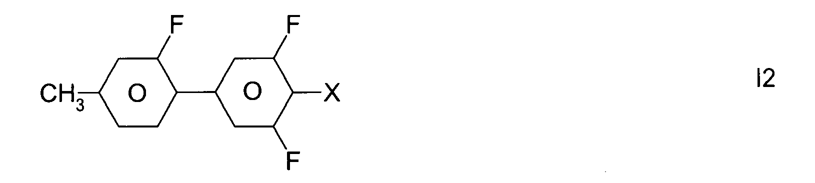

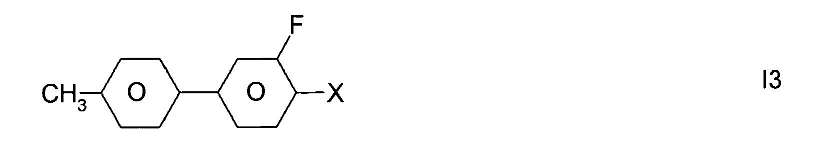

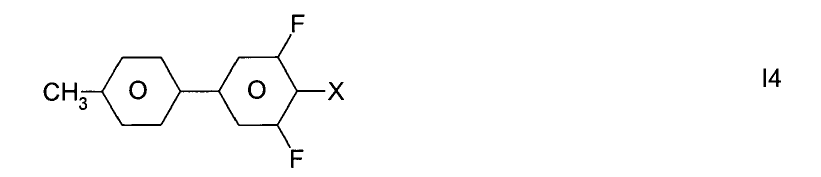

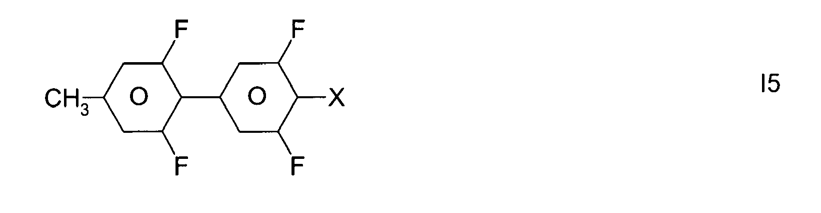

4. Milieu selon la revendication 3, **caractérisé en ce que** la proportion de composés des formules I à X dans le mélange pris dans sa globalité est d'au moins 50% en poids.

5. Milieu selon l'une quelconque des revendications 1 à 4, **caractérisé en ce qu'**il comprend de façon additionnelle un ou plusieurs composés des formules RI à RXV 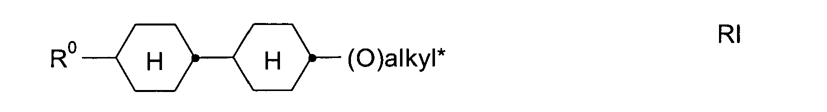 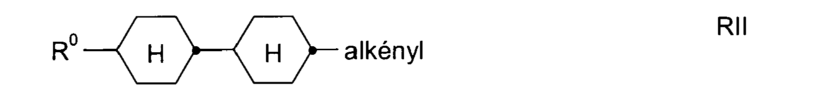 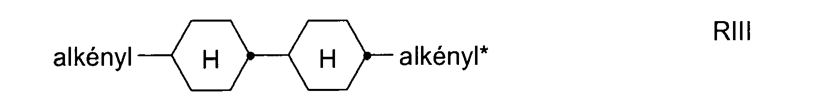 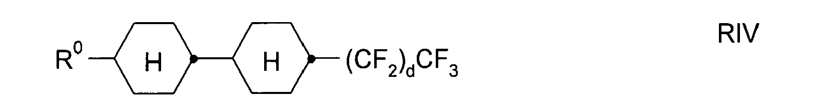 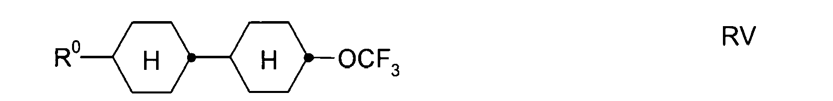 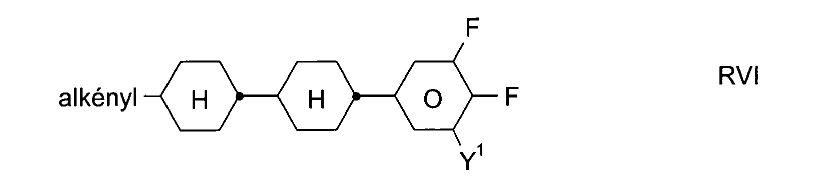 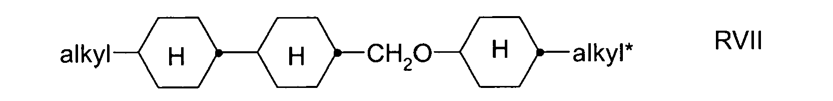 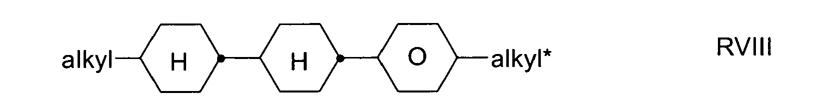 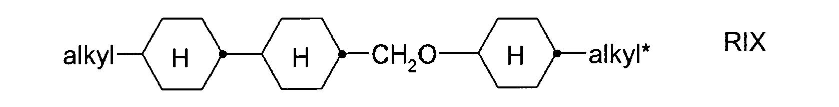 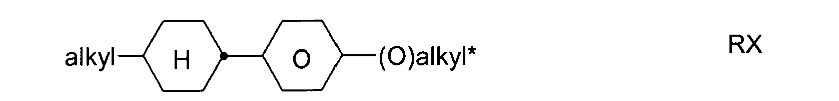 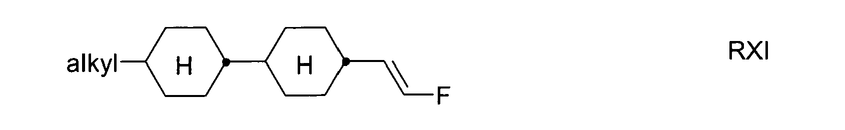 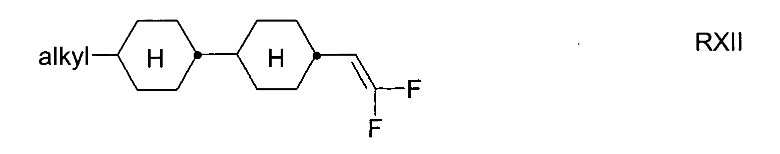 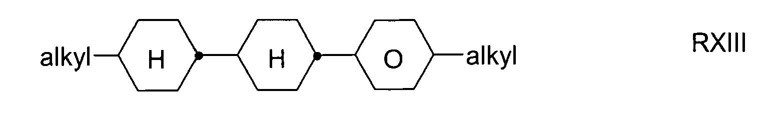 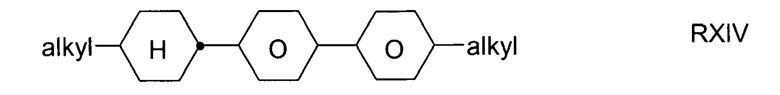 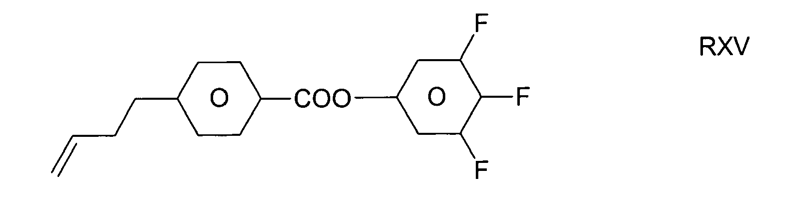 dans lesquelles
R⁰ représente n-alkyle, oxaalkyle, fluoroalkyle, alkényloxy ou alkényle dont chacun comporte de 2 à 12 atomes de C,
d représente 0, 1 ou 2,
Y¹ représente H ou F,
alkyl ou alkyl* chacun indépendamment de l'autre, représentent un radical alkyle en chaîne droite ou ramifié comportant de 2 à 8 atomes de C,
alkényl ou alkényl* chacun indépendamment de l'autre, représentent un radical alkyle en chaîne droite ou ramifié comportant de 2 à 8 atomes de C.

6. Milieu selon la revendication 1, **caractérisé en ce que** X⁰ représente F, OCHF₂ ou OCF₃ et Y² représente H ou F.

7. Utilisation du milieu de cristal liquide selon la revendication 1 à des fins électro-optiques.

8. Affichage à cristal liquide électro-optique contenant un milieu de cristal liquide selon la revendication 1.
